(19) Europäisches Patentamt
European Patent Office
Office européen des brevets

(11) EP 2 865 665 B1

(12) **EUROPEAN PATENT SPECIFICATION**

(45) Date of publication and mention of the grant of the patent:
**30.05.2018 Bulletin 2018/22**

(21) Application number: **13807006.5**

(22) Date of filing: **05.06.2013**

(51) Int Cl.:
***C07C 317/44*** *(2006.01)* ***A01N 41/10*** *(2006.01)*
***A01N 41/12*** *(2006.01)* ***A01N 43/40*** *(2006.01)*
***A01N 43/54*** *(2006.01)* ***A01P 7/02*** *(2006.01)*
***A01P 7/04*** *(2006.01)* ***A01P 17/00*** *(2006.01)*
***C07C 323/62*** *(2006.01)* ***C07D 213/56*** *(2006.01)*
***C07D 239/26*** *(2006.01)*

(86) International application number:
**PCT/JP2013/066127**

(87) International publication number:
**WO 2013/191041 (27.12.2013 Gazette 2013/52)**

(54) **AMIDE COMPOUND AND USE THEREOF FOR PEST CONTROL**

AMIDVERBINDUNG UND VERWENDUNG DAVON ZUR SCHÄDLINGSBEKÄMPFUNG

COMPOSÉ AMIDE ET SON UTILISATION POUR LA LUTTE CONTRE LES ANIMAUX NUISIBLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2012 JP 2012140587**

(43) Date of publication of application:
**29.04.2015 Bulletin 2015/18**

(73) Proprietor: **Sumitomo Chemical Co., Ltd**
**Chuo-ku, Tokyo 104-8260 (JP)**

(72) Inventors:
- **MAEHATA, Ryota**
  **Takarazuka-shi**
  **Hyogo 665-8555 (JP)**
- **MIZUNO, Hajime**
  **Takarazuka-shi**
  **Hyogo 665-8555 (JP)**
- **SHIMIZU, Chie**
  **Takarazuka-shi**
  **Hyogo 665-8555 (JP)**
- **NOKURA, Yoshihiko**
  **Takarazuka-shi**
  **Hyogo 665-8555 (JP)**

(74) Representative: **Vossius & Partner**
**Patentanwälte Rechtsanwälte mbB**
**Siebertstrasse 3**
**81675 München (DE)**

(56) References cited:
**WO-A1-2008/074427** **WO-A1-2010/127928**
**WO-A1-2010/133312** **WO-A1-2012/086848**
**JP-A- H07 157 486** **JP-A- H11 240 857**
**JP-A- 2003 034 671** **US-A1- 2002 156 281**

Remarks:
The file contains technical information submitted after the application was filed and not included in this specification

EP 2 865 665 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to an amide compound and a use thereof for pest control.

### BACKGROUND ART

WO2003-34671 describes a benzamide derivative used for an agricultural and horticultural agent.

### SUMMARY OF THE INVENTION

The present invention provides a compound having an excellent control effect on pests and a method for controlling pests using the compound.
According to the present invention, an amide compound represented by the following formula (1) has an excellent control effect on pests.
More specifically, the present invention is as described below.

[1] An amide compound represented by formula (1):

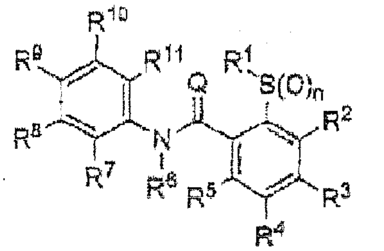

(1)

[wherein

$R^1$ represents a C1 to C6 chain hydrocarbon group optionally having one or more atoms or groups selected from group X or a C3 to C6 cycloalkyl group optionally having one or more atoms or groups selected from group Y,
$R^2$, $R^3$, $R^4$ and $R^5$ are the same or different and represent a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom,
$R^6$ represents a methyl group, an ethyl group, a propyl group, an isopropyl group, a butyl group, an isobutyl group, or a hexyl group,
$R^7$ and $R^{11}$ are the same or different and represent a C1 to C6 chain hydrocarbon group optionally having one or mere atoms or groups selected from group X, a C3 to C6 cycloalkyl group optionally having one or more atoms or groups selected from group Y, $-OR^{16}$, $-S(O)_mR^{16}$, a fluorine atom or a hydrogen atom,
$R^8$, $R^9$ and $R^{10}$ are the same or different and represent a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 5-membered heterocyclic group optionally having one or more atoms or groups selected from group Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{14}$, $-C(O)NR^{15}R^{16}$, $-SF_5$, a cyano group, a nitro group, a halogen atom, or a hydrogen atom,
$R^{12}$ and $R^{13}$ are the same or different and represent a C1 to C6 alkyl group optionally having one or more halogen atoms, a phenyl group optionally having one or more atoms or groups selected from group Z, a 5- or 6-membered heterocyclic group optionally having one or more atoms or groups selected from group Z or a hydrogen atom,
$R^{14}$ represents a C1 to C6 alkyl group optionally having one or more halogen atoms, a phenyl group optionally having one or more atoms or groups selected from group Z or a 5- or 6-membered heterocyclic group optionally having one or more atoms or groups selected from group Z,
$R^{15}$ represents a C1 to C6 alkyl group optionally having one or more halogen atoms or a hydrogen atom,

$R^{16}$ represents a C1 to C6 alkyl group optionally having one or more halogen atoms,
Q represents an oxygen atom or a sulfur atom,
m represents 0, 1 or 2, and
n represents 0, 1 or 2;
Group X: a group consisting of C3 to C6 cycloalkyl groups optionally having one or more atoms or groups selected from the group consisting of halogen atoms and C1 to C3 alkyl groups, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, and halogen atoms;
Group Y: a group consisting of C1 to C6 alkyl groups optionally having one or more halogen atoms, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, and halogen atoms;
Group Z: a group consisting of C1 to C6 alkyl groups optionally having one or more halogen atoms, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C1 to C6 alkylamino groups optionally having one or more halogen atoms, C2 to C8 dialkylamino groups optionally having one or more halogen atoms, C2 to C6 alkylcarbonyl groups optionally having one or more halogen atoms, C2 to C6 alkoxycarbonyl groups optionally having one or more halogen atoms, hydroxy groups, thiol groups, amino groups, cyano groups, nitro groups, and halogen atoms;
Group W: a group consisting of C3 to C6 cycloalkyl groups optionally having one or more atoms or groups selected from the group consisting of halogen atoms and C1 to C3 alkyl groups, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkoxycarbonyl groups optionally having one or more halogen atoms, hydroxy groups, cyano groups, and halogen atoms;
wherein $R^8$, $R^9$ and $R^{10}$ do not simultaneously represent a hydrogen atom, when $R^7$, $R^8$, $R^9$ and $R^{11}$ represent a hydrogen atom, $R^{10}$ does not represent a methyl group, and when $R^7$, $R^8$, $R^{10}$ and $R^{11}$ represent a hydrogen atom, $R^9$ does not represent a pyrrolidin-1-yl group, and
when m is 1 or 2 in $-S(O)_mR^{12}$, $R^{12}$ does not represent a hydrogen atom.]
or an N-oxide thereof (hereinafter, referred to as the compound of the present invention).

[2] The compound according to [1],

wherein $R^1$ is a C3 to C6 cycloalkyl group optionally having one or more atoms or groups selected from the group consisting of halogen atoms and C1 to C3 alkyl groups,
a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups),
a C2 to C6 alkenyl group optionally having one or more halogen atoms, or
a C2 to C6 alkynyl group optionally having one or more halogen atoms,
$R^2$, $R^4$ and $R^5$ are the same or different and are a C1 to C6 alkyl group optionally having one or more halogen atoms, $-OR^{12}$, $-S(O)_mR^{12}$, a halogen atom, or a hydrogen atom,
$R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom,
$R^6$ represents a methyl group, an ethyl group, a propyl group, an isopropyl group, a butyl group, an isobutyl group, or a hexyl group,
$R^7$ and $R^{11}$ are the same or different and are a C1 to C6 alkyl group optionally having one or more halogen atoms, $-OR^{16}$, $-S(O)_mR^{16}$, a fluorine atom or a hydrogen atom,
$R^8$, $R^9$ and $R^{10}$ are the same or different and are a C1 to C6 chain hydrocarbon group optionally having one or more atoms or groups selected from group X, $-OR^{12}$, $-S(O)_mR^{12}$, $-SF_5$, a halogen atom or a hydrogen atom,
$R^{12}$ and $R^{13}$ are the same or different and are a C1 to C6 alkyl group optionally having one or more halogen atoms, a phenyl group (wherein the phenyl group optionally has a C1 to C3 alkyl group optionally having one

or more halogen atoms or one or more halogen atoms), a 5- or 6-membered heterocyclic group (wherein the 5- or 6-membered heterocyclic group optionally has a C1 to C3 alkyl group optionally having one or more halogen atoms or one or more halogen atoms) or a hydrogen atom,

$R^{14}$ is a C1 to C6 alkyl group optionally having one or more halogen atoms, a phenyl group (wherein the phenyl group optionally has a C1 to C3 alkyl group optionally having one or more halogen atoms or one or more halogen atoms) or a 5- or 6-membered heterocyclic group (wherein the 5- or 6-membered heterocyclic group optionally has a C1 to C3 alkyl group optionally having one or more halogen atoms or one or more halogen atoms), and

Q is an oxygen atom.

[3] The compound according to [1],

wherein $R^1$ is a C3 to C6 cycloalkyl group optionally having one or more atoms or groups selected from the group consisting of halogen atoms and C1 to C3 alkyl groups, a C2 to C6 alkyl group optionally having one or more halogen atoms or a (C3 to C6 cycloalkyl) C1 to C3 alkyl group optionally having one or more halogen atoms,

$R^2$, $R^4$ and $R^5$ are the same or different and are a halogen atom or a hydrogen atom,

$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- cr 6-membered heterocyclic group (wherein the 5- or 6-membered heterocyclic group optionally has a C1 to C3 alkyl group optionally having one or more halogen atoms or one or more halogen atoms), $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, a halogen atom, or a hydrogen atom,

$R^6$ represents a methyl group, an ethyl group, a propyl group, an isopropyl group, a butyl group, an isobutyl group, or a hexyl group,

$R^7$ and $R^{11}$ are the same or different and are a fluorine atom or a hydrogen atom,

$R^8$, $R^9$ and $R^{10}$ are the same or different and are a C1 to C6 alkyl group optionally having one or more halogen atoms, a C2 to C6 alkenyl group optionally having one or more halogen atoms, a C2 to C6 alkynyl group optionally having one or more halogen atoms, $-OR^{12}$, $-S(O)_mR^{12}$, a halogen atom or a hydrogen atom,

$R^{12}$ and $R^{13}$ are the same or different and are a C1 to C6 alkyl group optionally having one or more halogen atoms or a hydrogen atom,

$R^{14}$ is a C1 to C6 alkyl group optionally having one or more halogen atoms, and

Q is an oxygen atom.

[4] The compound as defined in any of [1] to [3], wherein $R^2$, $R^4$ and $R^5$ are a hydrogen atom.

[5] The compound as defined in any of [1] to [4], wherein $R^7$ and $R^{11}$ are a hydrogen atom.

[6] The compound according to any of [1] to [5], wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a C1 to C6 alkylamino group optionally having one or more halogen atoms, a C2 to C8 dialkylamino group optionally having one or more halogen atoms, a C2 to C6 alkylcarbonylamino group optionally having one or more halogen atoms, a C2 to C6 alkoxycarbonylamino group optionally having one or more halogen atoms, a C1 to C3 alkylsulfonylamino group optionally having one or more halogen atoms, an amino group, a C2 to C6 alkylcarbonyl group optionally having one or more halogen atoms, a C2 to C6 alkoxycarbonyl group optionally having one or more halogen atoms, a C2 to C6 alkylaminocarbonyl group optionally having one or more halogen atoms, a C3 to C8 dialkylaminocarbonyl group optionally having one or more halogen atoms, an aminocarbonyl group, a pyridyl group (wherein the pyridyl group optionally has one or more atoms or substituents selected from the group consisting of C1 to C3 alkyl groups optionally having a halogen atom, C1 to C3 alkoxy groups optionally having a halogen atom, and halogen atoms), a pyrimidinyl group (wherein the pyrimidinyl group optionally has one or more atoms or substituents selected from the group consisting of C1 to C3 alkyl groups optionally having a halogen atom, C1 to C3 alkoxy groups optionally having a halogen atom, and halogen atoms), a halogen atom, or a hydrogen atom.

[7] The compound according to any of [1] to [6], wherein

$R^6$ is a methyl group or ethyl group.

[8] The compound according to any of [1] to [7], wherein $R^1$ is an ethyl group, a cyclopropyl group, or a cyclopropylmethyl group.

[9] The compound according to any of [1] to [8], wherein $R^8$, $R^9$ and $R^{10}$ are the same or different and are a C1 to C6 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

[10] The compound according to any cf [1] to [8], wherein $R^6$, $R^9$ and $R^{10}$ are the same or different and are a C1 to

C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

[11] The compound according to any of [1] to [8], wherein $R^7$, $R^6$, $R^{10}$ and $R^{11}$ are a hydrogen atom, $R^9$ is a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, or a halogen atom.

[12] The compound according to any of [1] to [8], wherein $R^7$, $R^9$ and $R^{11}$ are a hydrogen atom, $R^8$ and $R^{10}$ are the same or different and are a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

[13] A pest control composition comprising the compound as defined in any of [1] to [12], and an inert carrier.

[14] A method for controlling pests comprising applying an effective amount of the compound as defined in any of [1] to [12] to a pest or a pest-infested area.

MODE FOR CARRYING OUT THE INVENTION

In the compound of the present invention, an N-oxide is a compound in which the nitrogen atom constituting the ring on the heterocyclic group is oxidized. Examples of the heterocyclic group that may form the N-oxide include a pyridine ring.

The groups used in the description of the present specification will be described below with examples.

The notation of Ca to Cb chain hydrocarbon group in the present specification represents a straight-chain or branched-chain saturated or unsaturated hydrocarbon group having the number of carbon atoms of a to b.

Examples of the "C1 to C6 chain hydrocarbon group" include C1 to C6 alkyl groups such as a methyl group, an ethyl group, a propyl group, an isopropyl group, a butyl group, an isobutyl group, a sec-butyl group, a tert-butyl group, a pentyl group, a neopentyl group and a hexyl group; C2 to C6 alkenyl groups such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group and a 1-hexenyl group; and C2 to C6 alkynyl groups such as an ethynyl group, a propargyl group, a 2-butynyl group, a 3-butynyl group, a 1-pentynyl group and a 1-hexynyl group.

The notation of Ca to Cb alkyl group in the present specification represents a straight-chain or branohed-chain hydrocarbon group having the number of carbon atoms of a to b.

Examples of the "C1 to C6 alkyl group" include a methyl group, an ethyl group, a propyl group, an isopropyl group, a butyl group, an isobutyl group, a sec-butyl group, a tert-butyl group, a pentyl group, a neopentyl group, and a hexyl group.

The notation of Ca to Cb alkenyl group in the present specification represents a straight-chain or branched-chain unsaturated hydrocarbon group having the number of carbon atoms of a to b, and having one or two or more double bonds in the molecule.

Examples of the "C2 to C6 alkenyl group" include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, and a 1-hexenyl group.

The notation of Ca to Cb alkynyl group in the present specification represents a straight-chain or branched-chain unsaturated hydrocarbon group having the number of carbon atoms of a to b, and having one or two or more triple bonds in the molecule.

Examples of the "C2 to C6 alkynyl group" include an ethynyl group, a propargyl group, a 2-butynyl group, a 3-butynyl group, a 1-pentynyl group, and a 1-hexynyl group.

The notation of Ca to Cb cycloalkyl group in the present specification represents a cyclic alkyl group having the number of carbon atoms of a to b.

The "C3 to C6 cycloalkyl group" includes a cyclopropyl group, a cyclobutyl group, a cyclopentyl group, and a cyclohexyl group.

The notation of "Ca to Cb alkoxy group" in the present specification represents a straight-chain or branched-chain alkyl -0- group having the number of carbon atoms of a to b.

Examples of the "C1 to C6 alkoxy group" include a methoxy group, an ethoxy group, a propoxy group, an isopropoxy group, a butoxy group, an isobutoxy group, a sec-butoxy group, a tert-butoxy group, a pentyloxy group, a neopentyloxy group, and a hexyloxy group.

The notation of Ca to Cb alkylsulfanyl group in the present specification represents a straight-chain or branched-chain alkyl -S- group having the number of carbon atoms of a to b.

Examples of the "C1 to C6 alkylsulfanyl group" include a methylsulfanyl group, an ethylsulfanyl group, a propylsulfanyl group, an isopropylsulfanyl group, a butylsulfanyl group, a pentylsulfanyl group, and a hexylsulfanyl group.

The notation of Ca to Cb alkylsulfinyl group in the present specification represents a straight-chain or branched-chain alkyl -S(O)- group having the number of carbon atoms of a to b.

Examples of the "C1 to C6 alkylsulfinyl group" include a methylsulfinyl group, an ethylsulfinyl group, a propylsulfinyl group, an isopropylsulfinyl group, a butylsulfinyl group, a pentylsulfinyl group, and a hexylsulfinyl group.

The notation of Ca to Cb alkylsulfonyl group in the present specification represents a straight-chain or branched-chain alkyl $-S(O)_2-$ group having the number of carbon atoms of a to b.

Examples of the "C1 to C6 alkylsulfonyl group" include a methylsulfonyl group, an ethylsulfonyl group, a propylsulfonyl group, an isopropylsulfonyl group, a butylsulfonyl group, a pentylsulfonyl group, and a hexylsulfonyl group.

The notation of Ca to Cb alkylamino group in the present specification represents a straight-chain or branched-chain alkyl -NH- group having the number of carbon atoms of a to b.

Examples of the "C1 to C6 alkylamino group" include a methylamino group, an ethylamino group, a propylamino group, an isopropylamino group, and a butylamino group.

The notation of Ca to Cb dialkylamino group in the present specification represents a straight-chain or branched-chain dialkylamino group having a total number of carbon atoms of each alkyl group of a to b, in which the number of carbon atoms of each alkyl group may be the same or different.

Examples of the "C2 to C8 dialkylamino group" include a dimethylamino group, a diethylamino group, and a dipropylamino group.

The notation of Ca to Cb alkylcarbonyl group in the present specification represents a straight-chain or branched-chain alkyl -C(O)- group having the number of carbon atoms of a to b.

Examples of the "C2 to C6 alkylcarbonyl group" include an acetyl group, a propionyl group, a butyryl group, a pentanoyl group, and a hexanoyl group.

The notation of Ca to Cb alkoxycarbonyl group in the present specification represents a straight-chain or branched-chain alkyl -O-C(O) - group having the number of carbon atoms of a to b.

Examples of the "C2 to C6 alkoxycarbonyl group" include a methoxycarbonyl group, an ethoxycarbonyl group, a propoxycarbonyl group, a butoxycarbonyl group, a pentyloxycarbonyl group, and a tert-butoxyoarbonyl group.

The notation of (Ca to Cb alkoxy)Cc to Cd alkyl group in the present specification represents a Cc to Cd alkyl group in which a hydrogen atom bound to the carbon atom of the Cc to Cd alkyl group is substituted by a Ca to Cb alkoxy group. The numbers of carbon atoms of the alkoxy group and the alkyl group are the same or different, and the notation represents a straight-ohain or branched-chain alkyl -O- alkyl group.

The" (C1 to C6 alkoxy) C1 to C6 alkyl group" in the compound of the present invention is a C1 to C6 alkyl group combining with a C1 to C6 alkoxy group, and the number of carbon atoms of the (C1 to C6 alkoxy) C1 to C6 alkyl group is 2 to 12.

Examples of the "(C1 to C6 alkoxy) C1 to C6 alkyl group" include a methoxymethyl group, an ethoxymethyl group, a 1-(methoxy)ethyl group, a 2-(methoxy)ethyl group, a 1-(ethoxy)ethyl group, and a 2-(ethoxy)ethyl group.

The notation of (Ca to Cb cycloalkyl) Cc to Cd alkyl group in the present specification represents a Cc to Cd alkyl group in which a hydrogen atom bound to the carbon atom of the Cc to Cd alkyl group is substituted by a Ca to Cb cycloalkyl group. The numbers of carbon atoms of the cycloalkyl group and the alkyl group may be the same or different, and the notation represents a cyclic alkyl-alkyl group.

The "(C3 to C6 cycloalkyl) C1 to C3 alkyl group" in the compound of the present invention is a C1 to C3 alkyl group combining with a C3 to C6 cycloalkyl group, and the number of carbon atoms of the (C3 to C6 cycloalkyl) C1 to C3 alkyl group is 4 to 9.

Examples of the "(C3 to C6 cycloalkyl) C1 to C3 alkyl group" include a cyclopropylmethyl group, a 2-cyclopropylethyl group, and a 1-cyclopropylethyl group.

The notation of Ca to Cb haloalkyl group in the present specification represents a straight-chain or branched-ohain hydrocarbon group having the number of carbon atoms of a to b, in which one or more hydrogen atoms bound to the carbon atom are substituted by a halogen atom, and at that time, when having two or more halogen atoms, those halogen atoms may be the same or different from each other.

Examples of the "C1 to C6 haloalkyl group" include a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

The notation of Ca to Cb haloalkenyl group in the present specification represents a straight-chain or branched-chain unsaturated hydrocarbon group having the number of carbon atoms of a to b, in which one or more hydrogen atoms bound to the carbon atom are substituted by a halogen atom, and having one or two or more double bonds in the molecule and at that time, when having two or more halogen atoms, those halogen atoms may be the same or different from each other.

Examples of the "C2 to C6 haloalkenyl group" include a 3,3-dichloro-2-propenyl group, a 3,3-dibromo-2-propenyl group, and the like.

The notation of Ca to Cb haloalkoxy in the present specification represents a straight-chain or branched-chain

alkyl -O- group having the number of carbon atoms of a to b, in which one or more hydrogen atoms bound to the carbon atom are substituted by a halogen atom, and at that time, when having two or more halogen atoms, those halogen atoms may be the same or different from each other.

Examples of the "C1 to C6 haloalkoxy group" include a trifluoromethoxy group, a 2,2,2-trifluoroethoxy group, a 2,2,2-trichoroethoxy group, a 2,2,3,3-tetrafluoropropoxy group, and a 2,2,3,4,4,4-hexafluorobutoxy group.

The notation of Ca to Cb haloalkylsulfanyl in the present specification represents a straight-chain or branched-chain alkyl -S- group having the number of carbon atoms of a to b, in which one or more hydrogen atoms bound to the carbon atom are substituted by a halogen atom, and at that time, when having two or more halogen atoms, those halogen atoms may be the same or different from each other.

Examples of the "C1 to C6 haloalkylsulfanyl group" include a trifluoromethylsulfany group, a 2,2,2-trifluoroethylsulfanyl group, a 2,2,2-trichoroethylsulfanyl group, a 2,2,3,3-tetrafluoropropylsulfanyl group, and a 2,2,3,4,4,4-hexafluorobutylsulfanyl group.

The notation of Ca to Cb haloalkylsulfinyl in the present specification represents a straight-chain or branched-chain alkyl -S(O)- group having the number of carbon atoms of a to b, in which one or more hydrogen atoms bound to the carbon atom are substituted by a halogen atom, and at that time, when having two or more halogen atoms, those halogen atoms may be the same or different from each other.

Examples of the "C1 to C6 haloalkylsulfinyl group" include a trifluoromethylsulfiny group, a 2,2,2-trifluoroethylsulfinyl group, a 2,2,2-trichoroethylsulfinyl group, a 2,2,3,3-tetrafluoropropylsulfinyl group, and a 2,2,3,4,4,4-hexafluorobutylsulfinyl group.

The notation of Ca to Cb haloalkylsulfonyl in the present specification represents a straight-chain or branched-chain alkyl $-S(O)_2-$ group having the number of carbon atoms of a to b, in which one or more hydrogen atoms bound to the carbon atom are substituted by a halogen atom, and at that time, when having two or more halogen atoms, those halogen atoms may be the same or different from each other.

Examples of the "C1 to C6 haloalkylsulfonyl group" include a trifluoromethylsulfony group, a 2,2,2-trifluoroethylsulfonyl group, a 2,2,2-trichoroethylsulfonyl group, a 2,2,3,3-tetrafluoropropylsulfonyl group, and a 2,2,3,4,4,4-hexafluorobutylsulfonyl group.

The notation of Ca to Cb perfluoroalkyl in the present specification represents a straight or branched alkyl group having the number of carbon atoms of a to b, in which all hydrogen atoms bound to the carbon atom are substituted by a fluorine atom.

Examples of the "C1 to C3 perfluoroalkyl group" include a trifluoromethyl group, a pentafluoroethyl group, a heptafluoropropyl group, and a heptafluoroisopropyl group.

The notation of Ca to Cb perfluoroalkoxy in the present specification represents a straight-chain or branched-chain alkyl -O- group having the number of carbon atoms of a to b, in which all hydrogen atoms bound to the carbon atom are substituted by a fluorine atom.

Examples of the "C1 to C3 perfluoroalkoxy group" include a trifluoromethoxy group, a pentafluoroethoxy group, a heptafluoropropoxy group, and a heptafluoroisopropoxy group.

The notation of Ca to Cb perfluoroalkylsulfanyl in the present specification represents a straight-chain or branched-chain alkyl -S- group having the number of carbon atoms of a to b, in which all hydrogen atoms bound to the carbon atom are substituted by a fluorine atom.

Examples of the "C1 to C3 perfluoroalkylsulfanyl group" include a trifluoromethylsulfanyl group, a pentafluoroethylsulfanyl group, a heptafluoropropylsulfanyl group, and a heptafluoroisopropylsulfanyl group.

The notation of Ca to Cb perfluoroalkylsulfinyl in the present specification represents a straight-chain or branched-chain alkyl -S(O)- group having the number of carbon atoms of a to b, in which all hydrogen atoms bound to the carbon atom are substituted by a fluorine atom.

Examples of the "C1 to C3 perfluoroalkylsulfinyl group" include a trifluoromethylsulfinyl group, a pentafluoroethylsulfinyl group, a heptafluoropropylsulfinyl group, and a heptafluoroisopropylsulfinyl group.

The notation of Ca to Cb perfluoroalkylsulfonyl in the present specification represents a straight-chain or branched-chain alkyl $-S(O)_2-$ group having the number of carbon atoms of a to b, in which all hydrogen atoms bound to the carbon atom are substituted by a fluorine atom.

Examples of the "C1 to C3 perfluoroalkylsulfonyl group" include a trifluoromethylsulfonyl group, a pentafluoroethylsulfonyl group, a heptafluoropropylsulfonyl group, and a heptafluoroisopropylsulfonyl group.

In the notation of "optionally having one or more atoms or groups selected from group X" in the present specification, when having two or more atoms or groups selected from group X, the atoms or groups selected from the group X may be the same or different from each other.

In the notation of "optionally having one or more atoms or groups selected from group Y" in the present specification, when having two or more atoms or groups selected from group Y, the atoms or groups selected from the group Y may be the same or different from each other.

In the notation of "optionally having one or more atoms or groups selected from group Z" in the present specification, when having two or more atoms or groups selected from group Z, the atoms or groups selected from the group Z may be the same or different from each other.

In the notation of "optionally having one or more atoms or groups selected from group W" in the present specification, when having two or more atoms or groups selected from group W, the atoms or groups selected from the group W may be the same or different from each other.

In the notation of "optionally having one or more halogen atoms" in the present specification, when having two or more halogen atoms, those halogen atoms may be the same or different from each other.

In the notation of "optionally having one or more atoms or groups selected from the group consisting of halogen atoms and C1 to C3 alkyl groups" in the present specification, when having two or more atoms or groups, the atoms or groups may be the same or different from each other.

The "halogen atom" in the compound of the present invention refers to a fluorine atom, a chlorine atom, a bromine atom, and an iodine atom.

The notation of the "C1 to C6 chain hydrocarbon group optionally having one or more atoms or groups selected from group X" in the compound of the present invention represents a straight-chain or branched-chain hydrocarbon group comprising a carbon atom number of 1 to 6, in which a hydrogen atom bound to the carbon atom is optionally substituted by an atom or group selected from group X, and at that time, when having two or more atoms or groups selected from group X, the atoms or groups selected from group X may be the same or different from each other.

Examples of the "C1 to C6 chain hydrocarbon group optionally having one or more atoms or groups selected from group X" include C1 to C6 alkyl groups optionally having one or more atoms or groups selected from group X such as a methyl group, an ethyl group, a propyl group, an isopropyl group, a butyl croup, an isobutyl group, a sec-butyl group, a tert-butyl group, a pentyl group, a neopentyl group, a hexyl group, a methoxymethyl group, an ethoxymethyl group, a propoxymethyl group, an isopropoxymethyl group, a butoxymethyl group, a sec-butoxymethyl group, a tert-butoxymethyl group, a 2-methoxyethyl group, a 2-ethoxyethyl group, a 2-propoxyethyl group, a 2-isopropoxyethyl group, a 2-butoxyethyl group, a 2-sec-butoxyethyl group, a 2-tert-butoxyethyl group, a trifluoromethyl group, a trichloromethyl group, a 2-fluoroethyl group, a 2,2-difluoroethyl group, a 2,2,2-trifluoroethyl group, a pentafluoroethyl group, a methylsulfanylethyl group, an ethylsulfanylethyl group, a methylsulfinylethyl group, a methylsulfonylethyl group, a cyclopropylmethyl group, a 1-methylcyclopropylmethyl group, and a 2,2-difluorocyclopropylmethyl group; C2 to C6 alkenyl groups optionally having one or more atoms or groups selected from group X such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group and a pentafluoroallyl group; and C2 to C6 alkynyl groups optionally having one or more atoms or groups selected from group X such as an ethynyl group, a propargyl group, a 2-butynyl group, a 3-butynyl group, a 1-pentynyl group, a 1-hexynyl group, a 4,4,4-trifluoro-2-butynyl group, and the C1 to C6 chain hydrocarbon group optionally having one or more atoms or groups selected from group X is selected in the range of each specified number of carbon atoms.

The notation of the "C3 to C6 cycloalkyl group optionally having one or more atoms or groups selected from group Y" in the compound of the present invention represents a cyclic alkyl group comprising a carbon atom number of 3 to 6, in which a hydrogen atom bound to the carbon atom is optionally substituted by an atom or group selected from group Y, and at that time, when having two or more atoms or groups selected from group Y, the atoms or groups selected from group Y may be the same or different from each other.

Examples of the "C3 to C6 cycloalkyl group optionally having one or more atoms or groups selected from group Y" include a cyclopropyl group, a cyclobutyl group, a cyclopentyl group, a cyclohexyl group, a 1-methylcyclohexyl group, a 2-methylcyclohexyl group, a 3-methylcyclohexyl group, a 4-methylcyclohexyl group, a 2-methoxylcyclohexyl group, a 3-methoxylcyclohexyl group, a 4-methoxylcyclohexyl group, a 1-fluorocyclohexyl group, a 2-fluorocyclohexyl group, a 3-fluorocyclohexyl group, and a 4-fluorocyclohexyl group.

Examples of the "C1 to C6 chain hydrocarbon group optionally having one or more atoms or groups selected from group W" in the compound of the present invention include C1 to C6 alkyl groups optionally having one or more atoms or groups selected from group W such as a methyl group, an ethyl group, a propyl group, an isopropyl group, a butyl group, an isobutyl group, a sec-butyl group, a tert-butyl group, a pentyl group, a neopentyl group, a hexyl group, a trifluoromethyl group, a trichloromethyl group, a 2-fluoroethyl group, a 2,2-difluoroethyl group, a 2,2,2-trifluoroethyl group, a pentafluoroethyl group, a methoxymethyl group, an ethoxymethyl group, a propoxymethyl group, an isopropoxymethyl group, a butoxymethyl group, a sec-butoxymethyl group, an isobutoxymethyl group, a tert-butoxymethyl group, a methoxyethyl group, an ethoxyethyl group, a propoxyethyl group, an isopropoxyethyl group, a butoxyethyl group, a sec-butoxyethyl group, an isobutoxyethyl group, a tert-butoxyethyl group, a methylsulfanylethyl group, an ethylsulfanylethyl group, a methylsulfinylethyl group, a methylsulfonylethyl group, a methoxycarbonylmethyl group, a methoxycarbonylethyl group, a 2-cyanoethyl group, a propyl-2-one group, a cyclopropylmethyl group, and a cyclopropylmethyl group; C2 to C6 alkenyl groups optionally having one or more atoms or groups selected from group W such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group and a

pentafluoroallyl group; and C2 to C6 alkynyl groups optionally having one or more atoms or groups selected from group W such as an ethynyl group, a propargyl group, a 2-butynyl group, a 3-butynyl group, a 1-pentynyl group, a 1-hexynyl group, and a 4,4,4-trifluoro-2-butynyl group. At that time, when having two or more atoms or groups selected from group W, the atoms or groups selected from group W may be the same or different from each other.

Examples of the "C1 to C6 alkyl group optionally having one or more halogen atoms" in the compound of the present invention include a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

Examples of the C2 to C6 alkyl groups optionally having one or more halogen atoms" in the compound of the present invention include an ethyl group, a propyl group, an isopropyl group, a butyl group, an isobutyl group, a sec-butyl group, a tert-butyl group, a pentyl group, a neopentyl group, a hexyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

Examples of the "C1 to C3 alkyl groups optionally having one or more halogen atoms" in the compound of the present invention include a methyl group, an ethyl group, a propyl group, an isopropyl group, a trifluoromethyl group, a trichloromethyl group, a 2,2-difluoroethyl group, and a 2,2,2-trifluoroethyl group.

Examples of the "C2 to C6 alkenyl group optionally having one or more halogen atoms" in the compound of the present invention include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group, and a pentafluoroallyl group.

Examples of the "C2 to C6 alkynyl group optionally having one or more halogen atoms" in the compound of the present invention include an ethynyl group, a propargyl group, a 2-butynyl group, a 3-butynyl group, a 1-pentynyl group, a 1-hexynyl group, and a 4,4,4-trifluoro-2-butynyl group.

The notation of the "a (C1 to C6 alkoxy) C1 to C6 alkyl group optionally having one or more halogen atoms" in the compound of the present invention represents a C1 to C6 alkyl group having one C1 to C6 alkoxy group optionally having one or more halogen atoms, and when having two or more halogen atoms, those halogen atoms maybe the same or different from each other. Examples include a methoxymethyl group, an ethoxymethyl group, a 1-(methoxy)ethyl group, a 2-(methoxy)ethyl group, a 1-(ethoxy)ethyl group, a 2-(ethoxy)ethyl group, and a (2,2,2-trifluoroethoxy)methyl group.

Examples of the "C3 to C6 cycloalkyl groups optionally having one or more atoms or groups selected from the group consisting of halogen atoms and C1 to C3 alkyl groups" in the compound of the present invention include a cyclopropyl group, a 1-methylcycloprcpyl group, a 2-methylcyclopropyl group, a 1-fluorocyclopropyl group, a 2,2-difluorocyclopropyl group, a 2,2-dichlorocyclopropyl group, a 2,2-dibromocyclopropyl group, a cyclobutyl group, a cyclopentyl group, and a cyclohexyl group.

Examples of the "C1 to C6 alkoxy groups optionally having one or more halogen atoms" in the compound of the present invention include a methoxy group, a trifluoromethoxy group, an ethoxy group, a 2,2,2-trifluoroethoxy group, a propoxy group, an isopropoxy group, a butoxy group, an isobutoxy group, a sec-butoxy group, a tert-butoxy group, a pentyloxy group, and a hexyloxy group.

Examples of the "C1 to C3 alkoxy groups optionally having one or more halogen atoms" in the compound of the present invention include a methoxy group, a trifluoromethoxy group, an ethoxy group, a 2,2,2-trifluoroethoxy group, a propoxy group, and an isopropoxy group.

Examples of the "C1 to C6 alkylsulfanyl groups optionally having one or more halogen atoms" in the compound of the present invention include a methylsulfanyl group, an ethylsulfanyl group, a propylsulfanyl group, an isopropylsulfanyl group, a butylsulfanyl group, a pentylsulfanyl group, a hexylsulfanyl group, a trifluoromethylsulfanyl group, a 2,2,2-trifluoroethylsulfanyl group, and a pentafluoroethylsulfanyl group.

Examples of the "C1 to C3 alkylsulfanyl groups optionally having one or more halogen atoms" in the compound of the present invention include a methylsulfanyl group, an ethylsulfanyl group, a propylsulfanyl group, an isopropylsulfanyl group, a trifluoromethylsulfanyl group, and a 2,2,2-trifluoroethylsulfanyl group.

Examples of the "C1 to C6 alkylsulfinyl groups optionally having one or more halogen atoms" in the compound of the present invention include a methylsulfinyl group, an ethylsulfinyl group, a propylsulfinyl group, an isopropylsulfinyl group, a butylsulfinyl group, a pentylsulfinyl group, a hexylsulfinyl group, a trifluoromethylsulfinyl group, a 2,2,2-trifluoroethylsulfinyl group, and a pentafluoroethylsulfinyl group.

Examples of the "C1 to C3 alkylsulfinyl groups optionally having one or more halogen atoms" in the compound of the present invention include a methylsulfinyl group, an ethylsulfinyl group, a propylsulfinyl group, an isopropylsulfinyl group, a trifluoromethylsulfinyl group, and a 2,2,2-trifluoroethylsulfinyl group.

Examples of the "C1 to C6 alkylsulfonyl groups optionally having one or more halogen atoms" in the compound of the present invention include a methylsulfonyl group, an ethylsulfonyl group, a propylsulfonyl group, an isopropylsulfonyl group, a butylsulfonyl group, a pentylsulfonyl group, a hexylsulfonyl group, a trifluoromethylsulfonyl group, a 2,2,2-trifluoroethylsulfonyl group, and a pentafluoroethylsulfonyl group.

Examples of the "C1 to C3 alkylsulfonyl groups optionally having one or more halogen atoms" in the compound of the present invention include a methylsulfonyl group, an ethylsulfonyl group, a propylsulfonyl group, an isopropylsulfonyl group, a trifluoromethylsulfinyl group, and a 2,2,2-trifluoroethylsulfonyl group.

Examples of the "C1 to C6 alkylamino groups optionally having one or more halogen atoms" in the compound of the present invention include a methylamino group, an ethylamino group, a 2,2,2-trifluoroethylamino group, a propylamino group, an isopropylamino group, and a butylamino group.

Examples of the "C2 to C8 dialkylamino groups optionally having one or more halogen atoms" in the compound of the present invention include a dimethylamino group, a diethylamino group, a bis(2,2,2-trifluoroethyl)amino group, and a dipropylamino group.

Examples of the "C2 to C6 alkylcarbonylamino groups optionally having one or more halogen atoms" in the compound of the present invention include an acetylamino group, a propionylamino group, a butyrylamino group, a pentanoylamino group, a hexanoylamino group, and a trifluoroacetylamino group.

Examples of the "C2 to C6 alkoxycarbonylamino groups optionally having one or more halogen atoms" in the compound of the present invention include a methoxycarbonylamino group, an ethoxycarbonylamino group, a propoxycarbonylamino group, a butoxycarbonylamino group, a pentyloxycarbonylamino group, a tert-butoxycarbonylamino group, and a 2,2,2-trifluoroethoxyearbonylamino group.

Examples of the "C1 to C3 alkylsulfonylamino groups optionally having one or more halogen atoms" in the compound of the present invention include a methylsulfonylamino group, an ethylsulfonylamino group, a propylsulfonylamino group, an isopropylsulfonylamino group, a trifluoromethylsulfinylamino group, and a 2,2,2-trifluoroethylaulfonylamino group.

Examples of the "C2 to C6 alkylcarbonyl groups optionally having one or more halogen atoms" in the compound of the present invention include an acetyl group, a propionyl group, a butyryl group, a pentanoyl group, a hexanoyl group, and a trifluoroacetyl group.

Examples of the "C2 to C6 alkoxycarbonyl groups optionally having one or more halogen atoms" in the compound of the present invention include a methoxycarbonyl group, an ethoxycarbonyl group, a propoxycarbonyl group, a butoxycarbonyl group, a pentyloxycarbonyl group, a tert-butoxycarbonyl group, and a 2,2,2-trifluoroethoxycarbonyl group.

Examples of the "C1 to C6 alkylaminocarbonyl groups optionally having one or more halogen atoms" in the compound of the present invention include a methylaminocarbonyl group, an ethylaminocarbonyl group, a 2,2,2-trifluoroethylaminocarbonyl group, a propylaminocarbonyl group, an isopropylaminocarbonyl group, and a butylaminocarbonyl group.

Examples of the "C3 to C8 dialkylaminocarbonyl groups optionally having one or more halogen atoms" in the compound of the present invention include a dimethylaminocarbonyl group, a diethylaminocarbonyl group, a bis(2,2,2-trifluoroethyl)aminocarbonyl group, and a dipropylaminocarbonyl group.

Examples of the "C1 to C6 alkyl group optionally having one or more atoms or groups selected from the group consisting of halogen atoms and a cyclolpropyl group (wherein the cyclolpropyl group may have one or more halogen atoms or one or more C1 to C3 alkyl groups)" in the compound of the present invention include a methyl group, an ethyl group, a propyl group, an isopropyl group, a butyl group, an isobutyl group, a sec-butyl group, a tert-butyl group, a pentyl group, a neopentyl group, a hexyl group, a trifluoromethyl group, a trichloromethyl group, a 2-fluoroethyl group, a 2,2-difluoroethyl group, a 2,2,2,-trifluoroethyl group, a pentafluoroethyl group, a heptafluoroisopropyl group, a cyclopropylmethyl group, a 2-cyclopropylethyl group, a 1-cyclopropylethyl group, a 1-methyloyclopropylmethyl group, a 2,2-dimethylcyclopropylmethyl group, and a 2,2-difluorocyclopropylmethyl group.

Examples of the "C2 to C6 alkyl group optionally having one or more atoms or groups selected from the group consisting of halogen atoms and a cyclolpropyl group (wherein the cyclolpropyl group may have one or more halogen atoms or one or more C1 to C3 alkyl groups)" in the compound of the present invention include an ethyl group, a propyl group, an isopropyl group, a butyl group, an isobutyl group, a sec-butyl group, a tert-butyl group, a pentyl group, a neopentyl group, a hexyl group, a 2-fluoroethyl group, a 2,2-difluoroethyl group, a 2,2,2,-trifluoroethyl group, a pentafluoroethyl group, a heptafluoroisopropyl group, a 2-cyclopropylethyl group, a 1-cyclopropylethyl group, and a 2-(2,2-difluorocyclopropyl)ethyl group.

Examples of the " (C3 to C6 cycloalkylalkyl) C1 to C3 alkyl groups optionally having one or more halogen atoms" in the compound of the present invention include a cyclopropylmethyl group, a 2-cyclopropylethyl group, a 1-cyclopropylethyl group, and a 2,2-difluorocyclopropylmethyl group.

Examples of the "C3 to C6 cycloalkyl group optionally having one or more atoms or groups selected from the group consisting of halogen atoms and C1 to C3 alkyl groups" in the compound of the present invention include a cyclopropyl group, a cyclobutyl group, a cyclopentyl group, a cyclohexyl group, a 1-methylcyclohexyl group, a 2-methylcyclohexyl group, a 3-methylcyclohexyl group, a 4-methylcyclohexyl group, a 1-fluorocyclohexyl group, a 2-fluorocyclohexyl group, a 3-fluorocyclohexyl group, and a 4-fluorocyclohexyl group.

The notation of the "phenyl group optionally having one or more atoms or groups selected from group Z" in the compound of the present invention represents a phenyl group in which a hydrogen atom bound to the carbon atom is optionally substituted by an atom or group selected from group Z, and at that time, when having two or more atoms or groups selected from group Z, the atoms or groups selected from group Z may be the same or different from each other.

Examples of the "phenyl group optionally having one or more atoms or groups selected from group Z" include a phenyl group, a 2-fluorophenyl group, a 3-fluorophenyl group, a 4-fluorophenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2,3,4,5,6-pentafluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a 2-trifluoromethylphenyl group, a 3-trifluoromethylphenyl group, a 4-trifluoromethylphenyl group, a 2-trifluoromethoxyphenyl group, a 3-trifluoromethoxyphenyl group, a 4-trifluoromethoxyphenyl group, a 2-trifluoromethylsulfanylphenyl group, a 3-trifluoromethylsulfanylphenyl group, a 4-trifluoromethylsulfanylphenyl group, a 4-methoxycarbonylphenyl group, a 4-nitrophenyl group, a 4-cyanophenyl group, a 4-methylaminophenyl group, a 4-dimethylaminophenyl group, a 4-methyleulfinylphenyl group, a 4-methylaulfonylphenyl group, a 4-acetylphenyl group, and a 4-methoxycarbonylphenyl group.

The "heterocyclic group" in the compound of the present invention represents a heterocyclic compound residue containing one or more nitrogen atoms, oxygen atoms or sulfur atoms, other than carbon atoms, as ring-constituting atoms, in the ring structure. In addition, in the compound of the present invention, a 5-membered heterocyclic group means a 5-membered aromatic heterocyclic group or a 5-membered nonaromatic heterocyclic group, and a 6-membered heterocyclic group means a 6-membered aromatic heterocyclic group or a 6-membered nonaromatic heterocyclic group.

The "heterocyclic group" in the "5- or 6-membered heterocyclic group optionally having one or more atoms or groups selected from group Z" in the compound of the present invention represents a heterocyclic compound residue containing one or more nitrogen atoms, oxygen atoms or sulfur atoms, other than carbon atoms, in the ring structure, and at that time, when having two or more atoms or groups selected from group Z, the atoms or groups selected from group Z may be the same or different from each other.

In addition, in the compound of the present invention, a 5- or 6-membered heterocyclic group means a 5- or 6-membered aromatic heterocyclic group or a 5- or 6-membered nonaromatic heterocyclic group.

Examples of the "5- or 6-membered heterocyclic group optionally having one or more atoms or groups selected from group Z" include 5- or 6-membered nonaromatic heterocyclic groups optionally having one or more atoms or groups selected from group Z such as a pyrrolidin-1-yl group, a 3,3,4,4-tetrafluoropyrrolidin-1-yl group, a tetrahydrofuran-2-yl group, a piperidyl group, a morpholinyl group and a thiomorpholinyl group; and 5- or 6-membered aromatic heterocyclic groups optionally having one or more atoms or groups selected from group Z such as a 2-pyrrolyl group, a 2-furyl group, a 3-furyl group, a 5-pyrazolyl group, a 4-pyrazolyl group, a 1-pyrrolyl group, a 1-methyl-2-pyrrolyl group, a 2-methylsulfanyl-1-pyrrolyl group, a 2-methylsulfinyl-1-pyrrolyl group, a 2-methylsulfonyl-1-pyrrolyl group, a 2-methylamino-1-pyrrolyl group, a 2-dimethylamino-1-pyrrolyl group, a 5-bromo-2-furyl group, a 5-nitro-2-furyl group, a 5-cyano-2-furyl group, a 5-methoxy-2-furyl group, a 5-acetyl-2-furyl group, a 5-methoxycarbonyl-2-furyl group, a 2-methyl-3-furyl group, a 2,5-dimethyl-3-furyl group, a 2,4-dimethyl-3-furyl group, a 5-methyl-2-thienyl group, a 3-methyl-2-thienyl group, a 1-methyl-3-trifluoromethyl-5-pyrazolyl group, a 5-chloro-1,3-dimethyl-4-pyrazolyl group, a pyrazol-1-yl group, a 3-chloro-pyrazol-1-yl group, a 3-bromopyrazol-1-yl group, a 4-chloropyrazol-1-yl group, a 4-bromopyrazol-1-yl group, an imidazole-1-yl group, a 1,2,4-triazole-1-yl group, a 3-chloro-1,2,4-triazole-1-yl group, a 1,2,3,4-tetrazol-1-yl group, a 1,2,3,5-tetrazol-1-yl group, a 2-thienyl group, a 3-thienyl group, a 3-trifluoromethyl-1,2,4-triazol-1-yl group, a 4-trifluoromethylpyrazol-1-yl group, a pyrazinyl group, a 4-pyrimidinyl group, a 5-pyrimidinyl group, a 2-pyridyl group, a 3-pyridyl group, a 4-pyridyl group, a 3-fluoro-2-pyridyl group, a 4-fluoro-2-pyridyl group, a 5-fluoro-2-pyridyl group, a 6-fluoro-2-pyridyl group, a 2-pyrimidinyl group, a 3-chloro-5-trifluoromethylpyridin-2-yl group and a 5-trifluoromethylpyridin-2-yl group.

Examples of the "phenyl group (wherein the phenyl group optionally has a C1 to C3 alkyl group optionally having one or more halogen atoms or one or more halogen atoms) " in the compound of the present invention include a phenyl group, a 2-fluorophenyl group, a 3-fluorophenyl group, a 4-fluorophenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2,3,4,5,6-pentafluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a 2-trifluoromethylphenyl group, a 3-trifluoromethylphenyl group, and a 4-trifluoromethylphenyl group.

Examples of the "5- or 6-membered heterocyclic group (wherein the 5- or 6-membered heterocyclic group optionally has one or more halogen atoms or a C1 to C3 alkyl group optionally having one or more halogen atoms)" in the compound of the present invention include 5- or 6-membered nonaromatic heterocyclic groups such as a pyrrolidin-1-yl group, a 3,3,4,4-tetrafluoropyrrolidin-1-yl group, a tetrahydrofuran-2-yl group, a piperidyl group, a morpholinyl group

and a thiomorpholinyl group; and 5- or 6-membered aromatic heterocyclic groups such as a 2-pyrrolyl group, a 2-furyl group, a 3-furyl group, a 5-pyrazolyl group, a 4-pyrazolyl group, a 1-pyrrolyl group, a 1-methyl-2-pyrrolyl group, a 5-bromo-2-furyl group, a 2-methyl-3-furyl group, a 2,5-dimethyl-3-furyl group, a 2,4-dimethyl-3-furyl group, a 5-methyl-2-thienyl group, a 3-methyl-2-thienyl group, a 1-methyl-3-trifluoromethyl-5-pyrazolyl group, a 5-chloro-1,3-dimethyl-4-pyrazolyl group, a pyrazol-1-yl group, a 3-chloro-pyrazol-1-yl group, a 3-bromopyrazol-1-yl group, a 4-chloropyrazol-1-yl group, a 4-bromopyrazol-1-yl group, an imidazol-1-yl group, a 1,2,4-triazol-1-yl group, a 3-chloro-1,2,4-triazol-1-yl group, a 1,2,3,4-tetrazol-1-yl group, a 1,2,3,5-tetrazol-1-yl group, a 2-thienyl group, a 3-thienyl group, a 3-trifluoromethyl-1,2,4-triazol-1-yl group, a 4-trifluoromethylpyrazol-1-yl group, a pyrazinyl group, a 4-pyrimidinyl group, a 5-pyrimidinyl group, a 2-pyridyl group, a 3-pyridyl group, a 4-pyridyl group, a 3-fluoro-2-pyridyl group, a 4-fluoro-2-pyridyl group, a 5-fluoro-2-pyridyl group, a 6-fluoro-2-pyridyl group, a 2-pyrimidinyl group, a 3-chloro-5-trifluoromethylpyridin-2-yl group and a 5-trifluoromethylpyridin-2-yl group.

Examples of the "C1 to C6 alkyl group having one phenyl group (wherein the phenyl group may have one or more atoms or groups selected from group Z)" in the compound of the present invention include a phenylmethyl group, a 4-chlorophenylmethyl group, a 4-trifluoromethylphenylmethyl group, and the like. At that time, when having two or more atoms or groups selected from group Z, the atoms or groups selected from group Z may be the same or different from each other.

Examples of the "C1 to C6 alkyl group having one 5- or 6-membered heterocyclic group (wherein the 5- or 6-membered heterocyclic group may have one or more atoms or groups selected from group Z) " in the compound of the present invention include C1 to C6 alkyl groups having a 5- or 6-membered nonaromatic heterocycle such as a tetrahydrofuran-2-ylmethyl group, a tetrahydropyran-2-ylmethyl group and a tetrahydropyran-3-ylmethyl group; and C1 to C6 alkyl groups having a 5- or 6-membered aromatic heterocycle such as a thiazol-5-ylmethyl group, a 2-chlorothiazol-5-ylmethyl group, a pyridin-3-ylmethyl group, a 6-chloropyridin-3-ylmethyl group and a 6-trifluoromethylpyridin-3-ylmethyl group. At that time, when having two or more atoms or groups selected from group Z, the atoms or groups selected from group Z may be the same or different from each other.

Examples of the "C1 to C6 alkyl group having one thiazolyl group (wherein the thiazolyl group optionally has one or more atoms or substituents selected from the group consisting of C1 to C3 alkyl groups optionally having a halogen atom, C1 to C3 alkoxy groups optionally having a halogen atom, and halogen atoms.)" in the compound of the present invention include a (thiazol-5-yl)methyl group, a (2-chlorothiazol-5-yl)methyl group, and a 1-(2-chlorothiazol-5-yl)ethyl group.

Examples of the "C1 to C6 alkyl group having one pyridyl group (wherein the pyridyl group optionally has one or more atoms or substituents selected from the group consisting of C1 to C3 alkyl groups optionally having a halogen atom, C1 to C3 alkoxy groups optionally having a halogen atom, and halogen atoms.)" in the compound of the present invention include a (pyridin-5-yl)methyl group, a (2-chloropyridin-5-yl)methyl group, a 1-(2-chloropyridin-5-yl)ethyl group, and a (2-trifluoromethylpyridin-5-yl)methyl group.

Examples of the "pyridyl group (wherein the pyridyl group optionally has one or more atoms or substituents selected from the group consisting of C1 to C3 alkyl groups optionally having a halogen atom, C1 to C3 alkoxy groups optionally having a halogen atom, and halogen atoms.)" in the compound of the present invention include a 2-pyridyl group, a 3-pyridyl group, a 4-pyridyl group, a 5-trifluoromethyl-2-pyridyl group, and a 3-chloro-5-trifluoromethyl-2-pyridyl group.

Examples of the "pyrimidinyl group (wherein the pyrimidinyl group optionally has one or more atoms or substituents selected from the group consisting of C1 to C3 alkyl groups optionally having a halogen atom, C1 to C3 alkoxy groups optionally having a halogen atom, and halogen atoms.)" in the compound of the present invention include a 2-pyrimidinyl group, a 4-pyrimidinyl group, a 5-pyrimidinyl group, and a 2-chloro-4-pyrimidinyl group.

Examples of the compound of the present invention include the following compounds.

In the formula (1), compounds wherein $R^1$ is a C1 to C6 chain hydrocarbon group optionally having one or more atoms or groups selected from group X.

In the formula (1), compounds wherein $R^1$ is a C3 to C6 cycloalkyl group optionally having one or more atoms or groups selected from group Y.

In the formula (1), compounds wherein $R^1$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, or C3 to C6 cycloalkyl group optionally having one or more atoms or groups selected from the group consisting of halogen atoms and C1 to C3 alkyl groups.

In the formula (1), compounds wherein $R^1$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, or a C2 to C6 alkynyl group optionally having one or more halogen atoms.

In the formula (1), compounds wherein $R^1$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups) .

In the formula (1), compounds wherein $R^1$ is a C3 to C6 cycloalkyl group optionally having one or more atoms or groups selected from the group consisting of halogen atoms and C1 to C3 alkyl groups.

In the formula (1), compounds wherein $R^1$ is a C2 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups) or a C3 to C6 cycloalkyl group optionally having one or more atoms or groups selected from the group consisting of halogen atoms and C1 to C3 alkyl groups.

In the formula (1), compounds wherein $R^1$ is a C3 to C6 cycloalkyl group optionally having one or more atoms or groups selected from the group consisting of halogen atoms and C1 to C3 alkyl groups, a C2 to C6 alkyl group optionally having one or more halogen atoms, or a (C3 to C6 cycloalkylalkyl)C1 to C3 alkyl groups optionally having one or more halogen atoms.

In the formula (1), compounds wherein $R^1$ is a C2 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups) .

In the formula (1), compounds wherein $R^1$ is a C2 to C6 alkyl group, a C2 to C6 alkenyl group, or a C2 to C6 alkynyl group.

In the formula (1), compounds wherein $R^1$ is a C2 to C6 alkyl group, a cyclopropyl group, or a cyclopropylmethyl group.

In the formula (1), compounds wherein $R^1$ is a methyl group, an ethyl group, a propyl group, an isopropyl group, a butyl group, a propargyl group, a trifluoromethyl group, a 2,2,2-trifluoroethyl group, a cyclopropyl group, a cyclobutyl group, a cyclopropylmethyl group, or a cyclobutylmethyl group.

In the formula (1), compounds wherein $R^1$ is an ethyl group, a cyclopropyl group, or a cyclopropylmethyl group.

In the formula (1), compounds wherein $R^1$ is an ethyl group.

In the formula (1), compounds wherein $R^1$ is a cyclopropyl group.

In the formula (1), compounds wherein $R^1$ is a cyclopropylmethyl group.

In the formula (1), compounds wherein $R^2$, $R^3$, $R^4$ and $R^5$ are the same or different and are a C1 to C6 chain hydrocarbon group optionally having one or more atoms or groups selected from group X, a C3 to C6 cycloalkyl group optionally having one or more atoms or groups selected from group Y, or a hydrogen atom.

In the formula (1), compounds wherein $R^2$, $R^3$, $R^4$ and $R^5$ are the same or different and are a phenyl group optionally having one or more atoms or groups selected from group Z, a 5- or 6-membered heterocyclic group optionally having one or more atoms or groups selected from group Z, or a hydrogen atom.

In the formula (1), compounds wherein $R^2$, $R^3$, $R^4$ and $R^5$ are the same or different and are $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group or a halogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom, and $R^3$ is a C1 to C6 chain hydrocarbon group optionally having one or more atoms or groups selected from group X, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-SF_5$, a cyano group, a nitro group, a halogen atom, or a hydrogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom, and $R^3$ is a C1 to C6 chain hydrocarbon group optionally having one or more atoms or groups selected from group X, a phenyl group optionally having one or more atoms or groups selected from group Z, a 5- or 6-membered heterocyclic group optionally having one or more atoms or groups selected from group Z, $-OR^{12}$, $-S(O)_3R^{12}$, $-SF_5$, a cyano group, a nitro group or a halogen atom.

In the formula (1), compounds wherein $R^2$, $R^4$ and $R^5$ are the same or different and are a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^2$, $R^4$ and $R^5$ are the same or different and are a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^2$, $R^4$ and $R^5$ are the same or different and are a halogen atom or a hydrogen atom, and $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group or a halogen atom.

In the formula (1), compounds wherein $R^2$, $R^4$ and $R^5$ are the same or different and are a halogen atom or a hydrogen atom, and $R^3$ is a C1 to C6 chain hydrocarbon group optionally having one or more atoms or groups selected from group X, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-SF_5$, a cyano group, a nitro group, a halogen atom, or a hydrogen atom.

In the formula (1), compounds wherein $R^2$, $R^4$ and $R^5$ are the same or different and are a halogen atom, or a hydrogen atom, $R^3$ is a C1 to C6 chain hydrocarbon group optionally having one or more atoms or groups selected from group X, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-SF_5$, a cyano group, a nitro group or a halogen atom.

In the formula (1), compounds wherein $R^2$, $R^4$ and $R^5$ are the same or different and are a halogen atom or a hydrogen atom, and $R^3$ is a C1 to C6 alkyl group optionally having one or more halogen atoms, a C2 to C6 alkenyl group optionally having one or more halogen atoms, a C2 to C6 alkynyl group optional ly having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, a halogen atom or a hydrogen atom.

In the formula (1), compounds wherein $R^2$, $R^4$ and $R^5$ are the same or different and are a halogen atom or a hydrogen atom, and $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$ or a halogen atom.

In the formula (1), compounds wherein $R^2$, $R^4$ and $R^5$ are a hydrogen atom, and $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, a

halogen atom or a hydrogen atom.

In the formula (1), compounds wherein $R^2$, $R^4$ and $R^5$ are a hydrogen atom, and $R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, a halogen atom or a hydrogen atom.

In the formula (1), compounds wherein $R^2$, $R^4$ and $R^5$ are a hydrogen atom, and $R^3$ is a C1 to C6 alkyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, a halogen atom or a hydrogen atom.

In the formula (1), compounds wherein $R^2$, $R^4$ and $R^5$ are a hydrogen atom, and $R^3$ is a C1 to C6 alkyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, a halogen atom or a hydrogen atom.

In the formula (1), compounds wherein $R^2$, $R^4$ and $R^5$ are a hydrogen atom, and $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.) or a halogen atom.

In the formula (1), compounds wherein $R^2$, $R^4$ and $R^5$ are a hydrogen atom, and $R^3$ is a C1 to C3 alkyl group optionally having one or more halogen atoms.

In the formula (1), compounds wherein $R^2$, $R^4$ and $R^5$ are a hydrogen atom, and $R^3$ is a C1 to C3 alkoxy group optionally having one or more halogen atoms.

In the formula (1), compounds wherein $R^2$, $R^4$ and $R^5$ are a hydrogen atom, and $R^3$ is a C1 to C3 alkylsulfanyl group optionally having one or more halogen atoms, a C1 to C3 alkylsulfinyl group optionally having one or more halogen atoms, or a C1 to C3 alkylsulfonyl group optionally having one or more halogen atoms.

In the formula (1), compounds wherein $R^2$, $R^4$ and $R^5$ are a hydrogen atom, and $R^3$ is a C1 to C6 alkylamino group optionally having one or more halogen atoms.

In the formula (1), compounds wherein $R^2$, $R^4$ and $R^5$ are a hydrogen atom, and $R^3$ is a C2 to C8 dialkylamino group optionally having one or more halogen atoms.

In the formula (1), compounds wherein $R^2$, $R^4$ and $R^5$ are a hydrogen atom, and $R^3$ is a C2 to C6 alkylcarbonylamino group optionally having one or more halogen atoms.

In the formula (1), compounds wherein $R^2$, $R^4$ and $R^5$ are a hydrogen atom, and $R^3$ is a C2 to C6 alkoxycarbonylamino group optionally having one or more halogen atoms.

In the formula (1), compounds wherein $R^2$, $R^4$ and $R^5$ are a hydrogen atom, and $R^3$ is a C2 to C6 alkylcarbonyl group optionally having one or more halogen atoms.

In the formula (1), compounds wherein $R^2$, $R^4$ and $R^5$ are a hydrogen atom, and $R^3$ is a C2 to C6 alkoxycarbonyl group optionally having one or more halogen atoms.

In the formula (1), compounds wherein $R^2$, $R^4$ and $R^5$ are a hydrogen atom, and $R^3$ is a C1 to C3 alkylsulfonylamino group optionally having one or more halogen atoms.

In the formula (1), compounds wherein $R^2$, $R^4$ and $R^5$ are a hydrogen atom, and $R^3$ is an amino group.

In the formula (1), compounds wherein $R^2$, $R^4$ and $R^5$ are a hydrogen atom, and $R^3$ is a C2 to C6 alkylaminocarbonyl group optionally having one or more halogen atoms.

In the formula (1), compounds wherein $R^2$, $R^4$ and $R^5$ are a hydrogen atom, and $R^3$ is a C3 to C8 dialkylaminocarbonyl group optionally having one or more halogen atoms.

In the formula (1), compounds wherein $R^2$, $R^4$ and $R^5$ are a hydrogen atom, and $R^3$ is an aminocarbonyl group.

In the formula (1), compounds wherein $R^2$, $R^4$ and $R^5$ are a hydrogen atom, and $R^3$ is a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.) or a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.).

In the formula (1), compounds wherein $R^2$, $R^4$ and $R^5$ are a hydrogen atom, and $R^3$ is a halogen atom.

In the formula (1), compounds wherein $R^2$, $R^4$ and $R^5$ are a hydrogen atom, and $R^3$ is a methyl group, an ethyl

group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a 2-pyridyl group, a 2-pyrimidinyl group, a 5-trifluoromethyl-2-pyridyl group, a 3-chloro-5-trifluoromethyl-2-pyridyl group, a fluorine atom, a chlorine atom, a bromine atom, or an iodine atom.

In the formula (1), compounds wherein $R^2$, $R^4$ and $R^3$ are a hydrogen atom, and $R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, an acetylamino group, a methoxycarbonylamino group, an ethoxycarbonylamino group, a tert-butyloxycarbonylamino group, a methylsulfonylamino group, an amino group, an acetyl group, a methoxycarbonyl group, an ethoxycarbonyl group, a methylaminocarbonyl group, an ethylaminocarbonyl group, a dimethylamincoarbonyl group, a diethylaminocarbonyl group, an aminocarbonyl group, a fluorine atom, a chlorine atom, a bromine atom, or an iodine atom.

In the formula (1), compounds wherein $R^2$, $R^4$ and $R^5$ are a hydrogen atom, and $R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a fluorine atom, a chlorine atom, a bromine atom, or an iodine atom.

In the formula (1), compounds wherein $R^2$, $R^3$, $R^4$ and $R^5$ are a hydrogen atom.

In the formula (1), compounds wherein $R^2$, $R^4$ and $R^5$ are the same or different and are a C1 to C6 alkyl group optionally having one or more halogen atoms, $-OR^{12}$, $-S(O)_mR^{12}$, a halogen atom, or a hydrogen atom.

In the formula (1), compounds wherein $R^2$, $R^4$ and $R^5$ are the same or different and are a C1 to C6 alkyl group optionally having one or more halogen atoms or a hydrogen atom.

In the formula (1), compounds wherein $R^2$, $R^4$ and $R^5$ are the same or different and are $-OR^{12}$, $-S(O)_mR^{12}$, a halogen atom, or a hydrogen atom.

In the formula (1), compounds wherein $R^2$, $R^4$ and $R^5$ are the same or different and are a halogen atom or a hydrogen atom.

In the formula (1), compounds wherein $R^2$, $R^4$ and $R^5$ are a hydrogen atom.

In the formula (1), compounds wherein $R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group, a halogen atom, or a hydrogen atom.

In the formula (1), compounds wherein $R^9$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has one or more halogen atoms or a C1 to C3 alkyl group optionally having one or more halogen atoms), $-OR^{12}$, $-S(O)_mR^{12}$, a halogen atom, or a hydrogen atom.

In the formula (1), compounds wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.), a halogen atom, or a hydrogen atoms.

In the formula (1), compounds wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.), or a halogen atom.

In the formula (1), compounds wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.), a halogen atom, or a hydrogen atoms.

In the formula (1), compounds wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a C1 to C6 alkylamino group optionally having one or more halogen atoms, a C2 to C8 dialkylamino group optionally having one or more halogen atoms, a C2 to C6 alkylcarbonylamino group optionally having one or more halogen atoms, a C2 to C6 alkoxycarbonylamino group optionally having one or more halogen atoms, a C1 to C3 alkylsulfonylamino group optionally having one or more halogen atoms, an amino group, a C2 to C6 alkylcarbonyl group optionally having one or more halogen atoms, a C2 to C6 alkoxycarbonyl group optionally having one or more halogen atoms, a C2 to C6 alkylaminocarbonyl group optionally having one or more halogen atoms, a C3 to C8 dialkylaminocarbonyl group optionally having one or more halogen atoms, an aminocarbonyl group, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.), a halogen atom, or a hydrogen atom.

In the formula (1), compounds wherein $R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a 2-pyridyl group, a 2-pyrimidinyl group, a 5-trifluoromethyl-2-pyridyl group, a 3-chloro-5-trifluoromethyl-2-pyridyl group, a fluorine atom, a chlorine atom, a bromine atom, or an iodine atom.

In the formula (1), compounds wherein $R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, an acetylamino group, a methoxycarbonylamino group, an ethoxycarbonylamino group, a tert-butcxycarbonylamino group, a methylsulfonylamino group, an amino group, an acetyl group, a methoxycarbonyl group, an ethoxyoarbonyl group, a methylaminocarbonyl group, an ethylaminocarbonyl group, a dimethylaminocarbonyl group, a diethylaminocarbonyl group, an aminocarbonyl group, a fluorine atom, a chlorine atom, a bromine atom, or an iodine atom.

In the formula (1), compounds wherein $R^5$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a fluorine atom, a chlorine atom, a bromine atom, or an iodine atom.

The following examples of $R^6$ are disclosed for reference in as far as they do not fulfill the requirements of $R^6$ in claim 1. Disclosed herein are compounds of formula (1), wherein $R^6$ is a C1 to C6 chain hydrocarbon group optionally having one or more atoms or groups selected from group W or a C3 to C6 cycloalkyl group optionally having one or more atoms or groups selected from group Y.

Disclosed herein are compounds of formula (1), wherein $R^6$ is a C1 to C6 alkyl group having one phenyl group (wherein the phenyl group optionally has one or more atoms or groups selected from group Z) or a C1 to C6 alkyl group having one 5- or 6-membered heterocyclic group (wherein the 5- or 6-membered heterocyclic group optionally has one or more atoms or groups selected from group Z).

Disclosed herein are compounds of formula (1), wherein $R^6$ is a $-C(O)R^{12}$, $-C(O)OR^{12}$ or $-C(O)NR^{12}R^{13}$.

Disclosed herein are compounds of formula (1), wherein $R^6$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, a C2 to C6 alkynyl group optionally

having one or more halogen atoms, a C1 to C6 alkyl group having one thiazolyl group (wherein the thiazolyl group optionally has one or more atoms or substituents selected from the group consisting of halogen atoms, C1 to C3 alkyl groups optionally having a halogen atom, and C1 to C3 alkoxy groups optionally having a halogen atom.), a C1 to C6 alkyl group having one pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a (C1 to C6 alkoxy) C1 to C6 alkyl group optionally having one or more halogen atoms, -C(O)$R^{12}$, or -C(O)O$R^{12}$.

Disclosed herein are compounds of formula (1), wherein $R^6$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, or a C2 to C6 alkynyl group optionally having one or more halogen atoms.

Disclosed herein are compounds of formula (1), wherein $R^6$ is a C1 to C6 alkyl group having one thiazolyl group (wherein the thiazolyl group optionally has one or more atoms or substituents selected from the group consisting of halogen atoms, C1 to C3 alkyl groups optionally having a halogen atom, and C1 to C3 alkoxy groups optionally having a halogen atom.) or a C1 to C6 alkyl group having one pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.).

Disclosed herein are compounds of formula (1), wherein $R^6$ is a (C1 to C6 alkoxy) C1 to C6 alkyl group optionally having one or more halogen atoms, -C(O)$R^{12}$, or -C(O)O$R^{12}$.

Disclosed herein are compounds of formula (1), wherein $R^6$ is a C1 to C6 alkyl group optionally having one cr more halogen atoms, a C2 to C6 alkenyl group optionally having one or more halogen atoms, or a C2 to C6 alkynyl group optionally having one or more halogen atoms.

Disclosed herein are compounds of formula (1), wherein $R^6$ is a C1 to C6 alkyl group, a C2 to C6 alkenyl group, or a C2 to C6 alkynyl group, a (C1 to C6 alkoxy) C1 to C6 alkyl group, -C(O)$R^{12}$, or -C(O)O$R^{12}$.

Disclosed herein are compounds of formula (1), wherein $R^6$ is a C1 to C6 alkyl group, a C2 to C6 alkenyl group, or a C2 to C6 alkynyl group.

Disclosed herein are compounds of formula (1), wherein $R^6$ is a C1 to C6 alkyl group optionally having one cr more atoms or groups selected from the group consisting of halogen atoms and a cyclopropyl group or a propargyl group.

Disclosed herein are compounds of formula (1), wherein $R^6$ is a C1 to C6 alkyl group optionally having one or more atoms or groups selected from the group consisting of halogen atoms and a cyclopropyl group.

Compounds of formula (1), wherein $R^6$ is a methyl group, an ethyl group, a propyl group, an isopropyl group, a butyl group, an isobutyl group, or a hexyl group are according to the invention, whereas a propargyl group, a cyclopropyl group, a cyclobutyl group, a cyclohexyl group, a cyclopropylmethyl group, a cyclobutylmethyl group, a 2,2,2-trifluoroethyl group, a benzyl group, a 6-chicropyridin-3-ylmethyl group, a 2-chlorothiazolyl-5-ylmethyl group, a methoxymethyl group, an ethoxymethyl group, a methoxycarbonyl group, or an ethoxycarbonyl group are disclosed herein.

Compounds of formula (1), wherein $R^6$ is a methyl group, an ethyl group, a propyl group, an isopropyl group, a butyl group, an isobutyl group, or a hexyl group which are according to the invention, while a propargyl group, a cyclopropyl group, a cyclobutyl group, a cyclohexyl group, a cyclopropylmethyl group, a cyclobutylmethyl group, a 2,2,2-trifluoroethyl group, a benzyl group, a 6-chloropyridin-3-ylmethyl group, or a 2-chlorothiazolyl-5-ylmethyl group are disclosed herein.

In the formula (1), compounds according to the invention wherein $R^6$ is a methyl group, an ethyl group, a propyl group, an isopropyl group, a butyl group, an isobutyl group, or a hexyl group.

Disclosed herein are compounds of formula (1), wherein $R^6$ is a cyclopropyl group, a cyclobutyl group, a cyclohexyl group, a cyclopropylmethyl group, or a cyclobutylmethyl group.

Disclosed herein are compounds of formula (1), wherein $R^6$ is a benzyl group, a 6-chloropyridin-3-ylmethyl group, or a 2-chlorothiazolyl-5-ylmethyl group.

Disclosed herein are compounds of formula (1), wherein $R^6$ is a methoxymethyl group, an ethoxymethyl group, a methoxycarbonyl group, or an ethoxycarbonyl group.

In the formula (1), compounds according to the invention wherein $R^6$ is a methyl group.

In the formula (1), compounds according to the invention wherein $R^6$ is an ethyl group.

Disclosed herein are compounds of formula (1), wherein $R^6$ is a cyclopropylmethyl group.

In the formula (1), compounds wherein $R^7$ and $R^{11}$ are the same or different and are a C1 to C6 chain hydrocarbon group optionally having one or more atoms or groups selected from group X or a hydrogen atom.

In the formula (1), compounds wherein $R^1$ and $R^{11}$ are the same or different and are a C1 to C6 chain hydrocarbon

group optionally having one or more atoms or groups selected from. group X, -$OR^{16}$, -$S(O)_mR^{16}$, a fluorine atom, or a hydrogen atom. In the formula (1), compounds wherein $R^7$ and $R^{11}$ are the same or different and are a C1 to C6 alkyl group optionally having one or more halogen atoms, -$OR^{16}$, -$S(O)_mR^{16}$, a fluorine atom, or a hydrogen atom.

In the formula (1), compounds wherein $R^1$ and $R^{11}$ are the same or different and are a fluorine atom or a hydrogen atom.

In the formula (1), compounds wherein $R^7$ and $R^{11}$ are a hydrogen atom.

In the formula (1), compounds wherein $R^8$, $R^9$ and $R^{10}$ are the same or different and are a C1 to C6 chain hydrocarbon group optionally having one or more atoms or groups selected from group X, -$OR^{12}$, -$S(O)_mR^{12}$, -$SF_5$, a halogen atom, or a hydrogen atom.

In the formula (1), compounds wherein $R^8$, $R^9$ and $R^{10}$ are the same or different and are a phenyl group optionally having one or more atoms or groups selected from group Z, a 5- or 6-membered heterocyclic group optionally having one or more atoms or groups selected from group Z, or a hydrogen atom.

In the formula (1), compounds wherein $R^8$, $R^9$ and $R^{10}$ are the same or different and are a C1 to C6 chain hydrocarbon group optionally having one or more atoms or groups selected from group X, a halogen atom, or a hydrogen atom.

In the formula (1), compounds wherein $R^8$, $R^9$ and $R^{10}$ are the same or different and are -$OR^{12}$, -$S(O)_mR^{12}$, a halogen atom, or a hydrogen atom.

In the formula (1), compounds wherein $R^8$, $R^9$ and $R^{10}$ are the same or different and are a C1 to C6 alkyl group optionally having one or more halogen atoms, a C2 to C6 alkenyl group optionally having one or more halogen atoms, a C2 to C6 alkynyl group optionally having one or more halogen atoms, -$OR^{12}$, -$S(O)_mR^{12}$, a halogen atom, or a hydrogen atom.

In the formula (1), compounds wherein $R^8$, $R^9$ and $R^{10}$ are the same or different and are a C1 to C6 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^8$, $R^9$ and $R^{10}$ are the same or different and are a C1 to C2 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^8$, $R^9$ and $R^{10}$ are the same or different and are a C1 to C2 haloalkyl group, a C2 to C6 haloalkenyl group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^8$, $R^9$ and $R^{10}$ are the same or different and are a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^8$, $R^9$ and $R^{10}$ are the same or different and are a C1 to C2 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^8$, $R^9$ and $R^{10}$ are the same or different and are a C1 to C2 perfluoroalkyl group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^8$, $R^9$ and $R^{10}$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^8$, $R^9$ and $R^{10}$ are the same or different and are a trifluoromethyl group, a pentafluoroethyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^8$, $R^9$ and $R^{10}$ are the same or different and are a trifluoromethyl group, a pentafluoroethyl group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^7$ and $R^{11}$ are the same or different and are a C1 to C6 alkyl group optionally having one or more halogen atoms, -$OR^{16,}$ -$S(O)_mR^{16}$, a fluorine atom, or a hydrogen atom, and $R^8$, $R^9$ and $R^{10}$ are the same or different and are a C1 to C6 chain hydrocarbon group optionally having one or more atoms or groups selected from group X, -$OR^{12}$, -$S(O)_mR^{12}$, -$SF_5$, a halogen atom, or a hydrogen atom.

In the formula (1), compounds wherein $R^7$ and $R^{11}$ are the same or different and are a fluorine atom, or a hydrogen atom, and $R^8$, $R^9$ and $R^{10}$ are the same or different and are a C1 to C6 alkyl group optionally having one or more halogen atoms, a C2 to C6 alkenyl group optionally having one or more halogen atoms, a C2 to C6 alkynyl group optionally having one or more halogen atoms, -$OR^{12}$, -$S(O)_mR^{12}$, a halogen atom, or a hydrogen atom.

In the formula (1), compounds wherein $R^7$ and $R^{11}$ are a hydrogen atom, and $R^8$, $R^9$ and $R^{10}$ are the same or

different and are a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{14}$, $-C(O)NR^{15}R^{16}$, $-SF_5$, a cyano group, a nitro group, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C6 chain hydrocarbon group optionally having one or more atoms or groups selected from group X, $-OR^{12}$, $-S(O)_mR^{12}$, $-SF_5$, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C6 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C2 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^1$ and $R^{11}$ are a hydrogen atom, and $R^8$, $R^9$ and $R^{10}$ are the same or different and are a C1 to C2 haloalkyl group, a C2 to C6 haloalkenyl group, a C1 to C6 haloalkylsulfanyl group, a C1 to C6 haloalkylsulfinyl group, a C1 to C6 haloalkylsulfonyl group, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C2 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^7$ and $R^{11}$ are a hydrogen atom, and $R^8$, $R^9$ and $R^{10}$ are the same or different and are a C1 to C2 perfluoroalkyl group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

In the formula (1), compounds wherein $R^7$ and $R^{11}$ are a hydrogen atom, and $R^8$, $R^9$ and $R^{10}$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^7$ and $R^{11}$ are a hydrogen atom, and $R^8$, $R^9$ and $R^{10}$ are the same or different and are a trifluoromethyl group, a pentafluoroethyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^1$ and $R^{11}$ are a hydrogen atom, and $R^5$, $R^9$ and $R^{10}$ are the same or different and are a trifluoromethyl group, a pentafluoroethyl group, a trifluoromethylsulfanyl group, a trifluoromethylsulfinyl group or a trifluoromethylsulfonyl group, a fluorine atom, a chlorine atom, a bromine atom, or a hydrogen atom.

In the formula (1), compounds wherein $R^7$ and $R^{11}$ are a hydrogen atom, and $R^8$, $R^9$ and $R^{10}$ are the same or different and are a pentafluoroethyl group, a heptafluoroisopropyl group, a trifluoromethoxy group, a trifluoromethylsulfanyl group, a trifluoromethylsulfinyl group or a trifluoromethylsulfonyl group, or a hydrogen atom.

In the formula (1), compounds wherein $R^7$ and $R^{11}$ are a hydrogen atom, and $R^8$, $R^9$ and $R^{10}$ are the same or different and are a pentafluoroethyl group, a trifluoromethoxy group, a trifluoromethylsulfanyl group, a trifluoromethylsulfinyl group or a trifluoromethylsulfonyl group, or a hydrogen atom.

In the formula (1), compounds wherein $R^7$ and $R^{11}$ are a hydrogen atom, and $R^8$, $R^9$ and $R^{10}$ are the same or different and are a pentafluoroethyl group, a trifluoromethylsulfanyl group, a trifluoromethylsulfinyl group or a trifluoromethylsulfonyl group, or a hydrogen atom.

In the formula (1), compounds wherein $R^7$, $R^8$, $R^{10}$ and $R^{11}$ are a hydrogen atom.

In the formula (1), compounds wherein $R^7$, $R^9$ and $R^{11}$ are a hydrogen atom.

In the formula (1), compounds wherein $R^7$, $R^8$, $R^{10}$ and $R^{11}$ are a hydrogen atom, and $R^9$ is a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group or a halogen atom.

In the formula (1), compounds wherein $R^7$, $R^9$ and $R^{11}$ are a hydrogen atom, and $R^1$ and $R^{10}$ are the same or different and are a C1 to C3 perfluoroalkyl group, a C1 to C3 perfluoroalkoxy group, a C1 to C3 perfluoroalkylsulfanyl

group, a C1 to C3 perfluoroalkylsulfinyl group, a C1 to C3 perfluoroalkylsulfonyl group, a halogen atom, or a hydrogen atom.

In the formula (1), compounds wherein Q is an oxygen atom.

In the formula (1), compounds wherein Q is a sulfur atom.

In the formula (1), compounds wherein m is 0.

In the formula (1), compounds wherein m is 1.

In the formula (1), compounds wherein m is 2.

In the formula (1), compounds wherein n is 0.

In the formula (1), compounds wherein n is 1.

In the formula (1), compounds wherein n is 2.

In the formula (1), compounds, wherein

$R^1$ is a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, or a C3 to C6 cycloalkyl group optionally having one or more atoms or groups selected from the group consisting of halogen atoms and C1 to C3 alkyl groups,

$R^2$, $R^4$ and $R^5$ are the same or different and are a C1 to C6 alkyl group optionally having one or more halogen atoms, $-OR^{12}$, $-S(O)_mR^{12}$, a halogen atom or a hydrogen atom,

$R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-C(O)R^{12}$, $-CO_2R^{12}$, $-C(O)NR^{12}R^{13}$, $-SF_5$, a cyano group, a nitro group, a halogen atom, or a hydrogen atom,

$R^6$ represents a methyl group, an ethyl group, a propyl group, an isopropyl group, a butyl group, an isobutyl group, or a hexyl group,

$R^1$ and $R^{11}$ are the same or different and are a C1 to C6 alkyl group optionally having one or more halogen atoms, $-OR^{16}$, $-S(O)_mR^{16}$, a fluorine atom or a hydrogen atom,

$R^8$, $R^9$ and $R^{10}$ are the same or different and are a C1 to C6 chain hydrocarbon group optionally having one or more atoms cr groups selected from group X, $-OR^{12}$, $-S(O)_mR^{12}$, $-SF_5$, a halogen atom or a hydrogen atom,

$R^{12}$ and $R^{13}$ are the same or different and are a C1 to C6 alkyl group optionally having one or more halogen atoms, a phenyl group (wherein the phenyl group optionally has a C1 to C3 alkyl group optionally having one or more halogen atoms or one or more halogen atoms), a 5- or 6-membered heterocyclic group (wherein the 5- or 6-membered heterocyclic group optionally has a C1 to C3 alkyl group optionally having one or more halogen atoms or one or more halogen atoms) or a hydrogen atom, and

Q is an oxygen atom.

In the formula (1), compounds according to [1], wherein

$R^1$ is a C3 to C6 cycloalkyl group optionally having one cr more atoms or groups selected from the group consisting of halogen atoms and C1 to C3 alkyl groups, a C2 to C6 alkyl group optionally having one or more halogen atoms or a (C3 to C6 cycloalkylalkyl) C1 to C3 alkyl group optionally having one or more halogen atoms,

$R^2$, $R^4$ and $R^5$ are the same or different and are a halogen atom or a hydrogen atom,

$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- cr 6-membered heterocyclic group optionally has a C1 to C3 alkyl group optionally having one or more halogen atoms or one or more halogen atoms), $-OR^{12}$, $-S(O)_mR^{12}$, a halogen atom, or a hydrogen atom,

$R^6$ represents a methyl group, an ethyl group, a propyl group, an isopropyl group, a butyl group, an isobutyl group, or a hexyl group,

$R^7$ and $R^{11}$ are the same or different and are a fluorine atom or a hydrogen atom,

$R^8$, $R^9$ and $R^{10}$ are the same or different and are a C1 to C6 alkyl group optionally having one or more halogen atoms, a C2 to C6 alkenyl group optionally having one or more halogen atoms, a C2 to C6 alkynyl group optionally having one or more halogen atoms, $-OR^{12}$, $-S(O)_mR^{12}$, a halogen atom or a hydrogen atom,

$R^{12}$ is a C1 to C6 alkyl group optionally having one or more halogen atoms or a hydrogen atom, and

Q is an oxygen atom.

In the formula (1), compounds, wherein

$R^1$ is a C2 to C6 alkyl group, a cyclopropyl group or a cyclopropylmethyl group,

$R^2$, $R^4$ and $R^5$ are a hydrogen atom,

$R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3

alkylsulfinyl group optionally having one or more halogen atoms, a C1 to C3 alkylsulfonyl group optionally having one or more halogen atoms, a pyridyl group (wherein the pyridyl group optionally has one or more atoms or substituents selected from the group consisting of halogen atoms, C1 to C3 alkyl groups optionally having a halogen atom, and C1 to C3 alkoxy groups optionally having a halogen atom.), a pyrimidinyl group (wherein the pyrimidinyl group optionally has one or more atoms or substituents selected from the group consisting of halogen atoms, C1 to C3 alkyl groups optionally having a halogen atom, and C1 to C3 alkoxy groups optionally having a halogen atom.), a halogen atom or a hydrogen atom,
$R^6$ represents a methyl group, an ethyl group, a propyl group, an isopropyl group, a butyl group, an isobutyl group, or a hexyl group,
$R^1$ and $R^{11}$ are a hydrogen atom,
$R^8$, $R^9$ and $R^{10}$ are the same or different and are a C1 to C2 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom or a hydrogen atom,
$R^{12}$ is a C1 to C6 alkyl group optionally having one or more halogen atoms or a hydrogen atom, and
Q is an oxygen atom.

In the formula (1), compounds, wherein
$R^1$ is an ethyl group, a cyclopropyl group or a cyclopropylmethyl group,
$R^2$, $R^4$ and $R^5$ are a hydrogen atom,
$R^3$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z, $-OR^{12}$, $-S(O)mR^{14}$ or a halogen atom,
$R^6$ represents a methyl group, an ethyl group, a propyl group, an isopropyl group, a butyl group, an isobutyl group, or a hexyl group,
$R^7$ and $R^{11}$ are a hydrogen atom,
$R^8$, $R^9$ and $R^{10}$ are the same or different and are a C1 to C6 perfluoroalkyl group, a C1 to C6 perfluoroalkoxy group, a C1 to C6 perfluoroalkylsulfanyl group, a C1 to C6 perfluoroalkylsulfinyl group, a C1 to C6 perfluoroalkylsulfonyl group, $-SF^5$, a cyano group, a nitro group, a halogen atom, or a hydrogen atom,
$R^{12}$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z or a hydrogen atom,
$R^{14}$ is a C1 to C6 alkyl group optionally having one or more halogen atoms, a phenyl group optionally having one or more atoms or groups selected from group Z or a 5- or 6-membered heterocyclic group optionally having one or more atoms or groups selected from group Z, and
Q is an oxygen atom.

In the formula (1), compounds, wherein
$R^1$ is an ethyl group, a cyclopropyl group or a cyclopropylmethyl group,
$R^2$, $R^4$ and $R^5$ are a hydrogen atom,
$R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsufanyl group, a methylsulfinyl group, a trifluoromethylsulfinyl group, a methylsulfonyl group, a trifluoromethylsulfonyl group, an acetylamino group, a methoxycarbonylamino group, an ethoxyoarbonylamino group, a tert-butoxycarbonylamino group, a methylsulfonylamino group, an amino group, an acetyl group, a methoxycarbonyl group, an ethoxycarbonyl group, a methylaminocarbonyl group, an ethylaminocarbonyl group, a dimethylaminocarbonyl group, a diethylaminocarbonyl group, an aminocarbonyl group, a 2-pyridyl group, a 2-pyrimidinyl group, a 5-trifluoromethyl-2-pyridyl group, a 3-chloro-5-trlfluoromethyl-2-pyridyl group, a fluorine atom, a chlorine atom, a bromine atom, an iodine atom, or a hydrogen atom,
$R^6$ is a methyl group, an ethyl group, a propyl group, an isopropyl group, a butyl group, an isobutyl group, or a hexyl group, while disclosed examples are a propargyl group, a cyclopropyl group, a cyclobutyl group, a cyclohexyl group, a cyclopropylmethyl group, a cyclobutylmethyl group, a 2,2,2-trifluoroethyl group, a benzyl group, a 6-chloropyridin-3-ylmethyl group, a 2-chlorothiazolyl-5-ylmethyl group, a methoxymethyl group, an ethoxymethyl group, a methoxycarbonyl group, or an ethoxycarbonyl group,
$R^7$ and $R^{11}$ are a hydrogen atom,
$R^8$, $R^9$ and $R^{10}$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom, and
Q is an oxygen atom.

In the formula (1), compounds, wherein
$R^1$ is an ethyl group, a cyclopropyl group or a cyclopropylmethyl group,

$R^2$, $R^4$ and $R^8$ are a hydrogen atom,
$R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a 2-pyridyl group, a 2-pyrimidinyl group, a 5-trifluoromethyl-2-pyridyl group, a 3-chloro-5-trifluoromethyl-2-pyridyl group, a fluorine atom, a chlorine atom, a bromine atom, an iodine atom, or a hydrogen atom,
$R^6$ is a methyl group, an ethyl group, a propyl group, an isopropyl group, a butyl group, an isobutyl group, or a hexyl group, while disclosed examples are a propargyl group, a cyclopropyl group, a cyclobutyl group, a cyclohexyl group, a cyclopropylmethyl group, a cyclobutylmethyl group, a 2,2,2-trifluoroethyl group, a benzyl group, a 6-chloropyridin-3-ylmethyl group, a 2-chlorothiazolyl-5-ylmethyl group, a methoxymethyl group, an ethoxymethyl group, a methoxycarbonyl group, or an ethoxycarbonyl group,
$R^7$ and $R^{11}$ are a hydrogen atom,
$R^8$, $R^9$ and $R^{10}$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom, and
Q is an oxygen atom.

In the formula (1), compounds wherein
$R^1$ is an ethyl group, a cyclopropyl group or a cyclopropylmethyl group,
$R^2$, $R^4$ and $R^5$ are a hydrogen atom,
$R^3$ is a C1 to C3 perfluoroalkyl group, a C1 to C6 alkylcarbonylamino group optionally having one or more halogen atoms, a C1 to C6 alkoxycarbonylamino group optionally having one or more halogen atoms, a C2 to C6 alkoxycarbonyl group optionally having one or more halogen atoms, a C2 to C6 alkylaminocarbonyl group optionally having one or more halogen atoms, a halogen atom or a hydrogen atom,
$R^6$ is a C1 to C3 alkyl group or, in disclosed examples, a cyclopropylmethyl group,
$R^7$ and $R^{11}$ are a hydrogen atom,
$R^8$ and $R^{10}$ are the same or different and are a C1 to C3 alkyl group optionally having one or more halogen atoms, a halogen atom or a hydrogen atom,
$R^9$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a halogen atom or a hydrogen atom, and
Q is an oxygen atom.

In the formula (1), compounds wherein
$R^1$ is an ethyl group, a cyclopropyl group or a cyclopropylmethyl group,
$R^3$, $R^4$ and $R^5$ are a hydrogen atom,
$R^3$ is a C1 to C3 perfluoroalkyl group, a halogen atom or a hydrogen atom,
$R^6$ is a C1 to C3 alkyl group or, in disclosed examples, a cyclopropylmethyl group,
$R^7$ and $R^{11}$ are a hydrogen atom,
$R^8$ and $R^{10}$ are the same or different and are a C1 to C3 alkyl group optionally having one or more halogen atoms, a halogen atom or a hydrogen atom,
$R^9$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a halogen atom or a hydrogen atom, and
Q is an oxygen atom.
Next, the method for producing the compound of the present invention will be described.

The compound of the present invention and an intermediate compound can be produced, for example, according to the following (Production Method 1) to (Production Method 13).

(Production Method 1)

The compound of the present invention (1-n1) or the compound of the present invention (1-n2) in which n is 1 or 2 in the formula (1) can be produced by oxidizing the compound of the present invention (1-n0) in which n is 0:

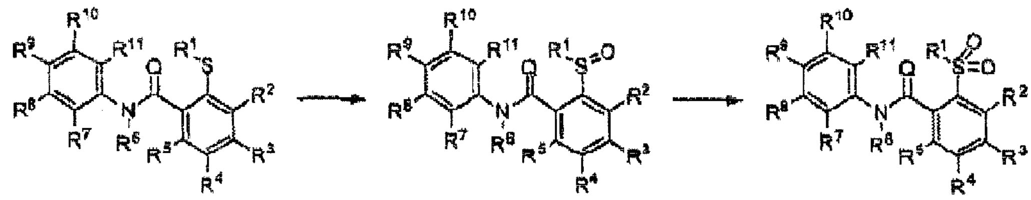

(1-n0) (1-n1) (1-n2)

wherein symbols represent the same meaning as described above.

The compound of the present invention (1-n1) in which n is 1 in the formula (1) can be produced by subjecting the compound of the present invention (1-n0) in which n is 0 to an oxidation reaction.

The oxidation reaction is usually carried out in the presence of a solvent.

Examples of the solvent include aliphatic halogenated hydrocarbons such as dichloromethane and chloroform, alcohols such as methanol and ethanol, acetic acid, water, and mixtures thereof.

The oxidizing agent used in the oxidation reaction is, for example, sodium periodate or m-chloroperbenzoic acid.

In the reaction, the oxidizing agent is usually used in a ratio of 1 to 3 mol, based on 1 mol of the compound of the present invention (1-n0). Preferably, the oxidizing agent is used in a ratio of 1 to 1.2 mol, based on 1 mol of the compound of the present invention (1-n0).

The reaction temperature is usually within the range of -20 to 80°C. The reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n1) can be isolated. The isolated compound of the present invention (1-n1) also can be further purified by chromatography, recrystallization, or the like. Alternatively, the compound of the present invention (1-n1) can be used in the next reaction as it is without isolation.

The compound of the present invention (1-n2) in which n is 2 in the formula (1) can be produced by subjecting the compound of the present invention (1-nl) in which n is 1 to an oxidation reaction.

The oxidation reaction is usually carried out in the presence of a solvent.

Examples of the solvent include aliphatic halogenated hydrocarbons such as dichloromethane and chloroform, alcohols such as methanol and ethanol, acetic acid, water, and mixtures thereof.

The oxidizing agent used in the oxidation reaction is, for example, m-chloroperbenzoic acid or aqueous hydrogen peroxide.

In the reaction, the oxidizing agent is usually used in a ratio of 1 to 4 mol, based on 1 mol of the compound of the present invention (1-n1). Preferably, the oxidizing agent is used in a ratio of 1 to 1.2 mol, based on 1 mol of the compound of the present invention (1-n1).

The reaction temperature is usually within the range of -20 to 120°C. The reaction time is usually within the range of 0.1 to 12 hours.

After completion of the reaction, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2) can be isolated. The compound of the present invention (1-n2) also can be further purified by chromatography, recrystallization, or the like.

The compound of the present invention (1-n2) in which n is 2 in the formula (1) can be produced by a one step reaction (one pot) by allowing the compound of the present invention (1-n0) in which n is 0 to react with an oxidizing agent.

The reaction is usually carried out in the presence of a solvent.

Examples of the solvent include aliphatic halogenated hydrocarbons such as dichloromethane and chloroform, alcohols such as methanol and ethanol, acetic acid, water, and mixtures thereof.

The oxidizing agent used in the reaction is, for example, m-chloroperbenzoic acid or aqueous hydrogen peroxide.

The reaction can be also carried out, in the presence of a catalyst, as necessary.

Examples of the catalyst used in the reaction include sodium tungstate.

In the reaction, the oxidizing agent is usually used in a ratio of 2 to 5 mol, and the catalyst is usually used in a ratio of 0.01 to 0.5 mol, based on 1 mol of the compound of the present invention (1-n0). Preferably, the oxidizing agent is used in a ratio of 2 to 2.2 mol, based on 1 mol of the compound of the present invention (1-n0).

The reaction temperature is usually within the range of 0 to 120°C. The reaction time is usually within the range of 0.1 to 12 hours.

After completion of the reaction, the reaction mixture is extracted with an organic solvent, and the organic layer

is washed with an aqueous solution of a reducing agent (for example, sodium sulfite, sodium thiosulfate) and an aqueous solution of a base (for example, sodium bicarbonate) as necessary, and subjected to post-treatment operations such as drying and concentration, whereby the compound of the present invention (1-n2) can be isolated. The isolated compound of the present invention (1-n2) also can be further purified by chromatography, recrystallization, or the like.

(Production Method 2)

The compound of the present invention (P1) in which Q is an oxygen atom in the formula (1) can be produced by reacting the intermediate compound (M1) with the intermediate compound (M2) :

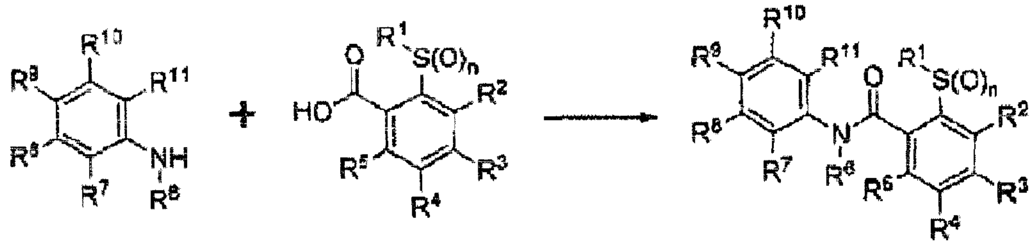

(M 1) (M 2) (P 1)

wherein symbols represent the same meaning as described above.

The compound of the present invention (P1) can be produced by reacting the intermediate compound (M1) with the intermediate compound (M2), in the presence of a condensing agent.

The reaction is usually carried out in the presence of a solvent. Examples of the solvent include ethers such as 1,4-dioxane, diethyl ether, tetrahydrofuran (hereinafter, referred to as THF) and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as N,N-dimethylformamide (hereinafter, referred to as DMF), N-methyl pyrrolidone (hereinafter, referred to as NMP), 1,3-dimethyl-2-imidazolidinone and dimethyl sulfoxide (hereinafter, referred to as DMSO), nitrogen-containing aromatic compounds such as pyridine and quinolone, and mixtures thereof.

Examples of the condensing agent include carbodiimides such as 1-ethyl-3-(3-dimethylaminopropyl) carbodiimide hydrochloride (hereinafter, referred to as EDCI hydrochloride) and 1,3-dicyclohexylcarbodiimide.

The reaction can be also carried out by adding a catalyst, as necessary. Examples of the catalyst include 1-hydroxybenzotriazole (hereinafter, referred to as HOBt).

In the reaction, the intermediate compound (M2) is usually used in a ratio of 0.8 to 1.2 mol, the condensing agent is usually used in a ratio of 1 to 2 mol, and the catalyst is usually used in a ratio of 0.01 to 1 mol, based on 1 mol of the intermediate compound (M1).

The reaction temperature is usually within the range of 0 to 200°C. The reaction time is usually within the range of 0.1 to 24 hours.

After completion of the reaction, the compound of the present invention (P1) can be isolated by adding the reaction mixture to water, then extracting the mixture with an organic solvent, and concentrating the organic layer; collecting a solid generated by adding the reaction mixture to water by filtration; or collecting a solid generated in the reaction mixture by filtration. The isolated compound of the present invention (P1) also can be further purified by recrystallization, chromatography, or the like.

(Production Method 3)

The compound of the present invention (P1) in which Q is an oxygen atom in the formula (1) can be produced by reacting the intermediate compound (M1) with the intermediate compound (M3) :

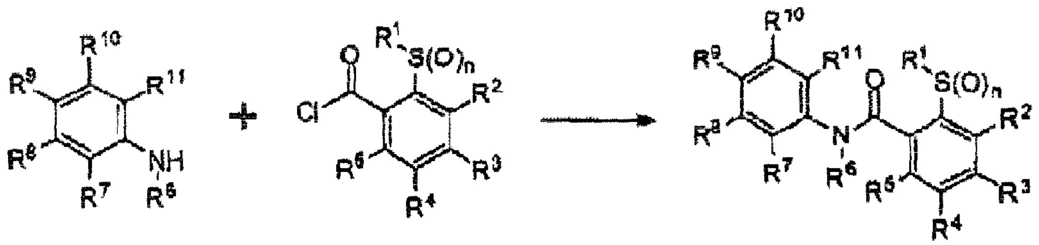

(M 1) (M 3) (P 1)

wherein symbols represent the same meaning as described above.

The compound of the present invention (P1) can be produced by reacting the compound (M1) with the intermediate compound (M3).

The reaction is usually carried out in the presence of a solvent.

Examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, eaters such as ethyl acetate and butyl acetate, nitriles such as acetonitrile, aprotic polar solvents such as DMF, NMP, and DMSO, and mixtures thereof.

The reaction can be also carried out by adding a base, as necessary. The base includes alkali metal carbonates such as sodium carbonate and potassium carbonate, tertiary amines such as triethylamine and N,N-diisopropylethylamine, and nitrogen-containing aromatic compounds such as pyridine, 4-dimethylaminopyridine, and the like.

In the reaction, the intermediate compound (M3) is usually used in a ratio of 0.8 to 1.2 mol, and the base is usually used in a ratio of 1 to 2 mol, based on 1 mol of the intermediate compound (M1).

The reaction temperature is usually within the range of -20 to 200°C. The reaction time is usually within the range of 0.1 to 24 hours.

After completion of the reaction, water is added to the reaction mixture, then the reaction mixture is extracted with an organic solvent, and the organic layer is subjected to post-treatment operations such as drying and concentration, whereby the compound of the present invention (P1) can be isolated. The isolated compound of the present invention (P1) also can be further purified by chromatography, recrystallization, or the like.

(Production Method 4)

The compound of the present invention (P1) in which Q is an oxygen atom in the formula (1) can be produced by reacting the intermediate compound (M4) with the intermediate compound (M5) :

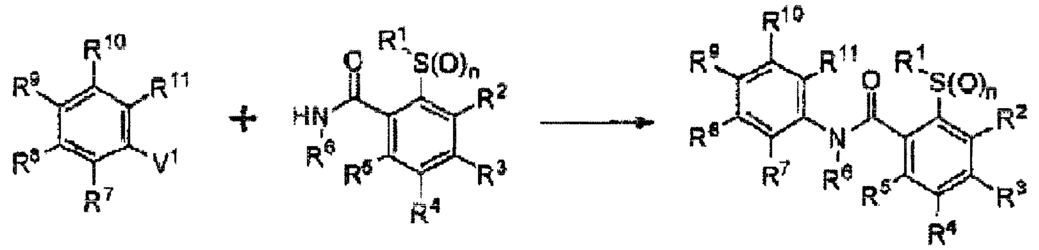

(M 4) (M 5) (P 1)

wherein $V^1$ represents a halogen atom, and other symbols represent the same meaning as described above.

When $V^1$ is a fluorine atom, the compound of the present invention (P1) in which Q is an oxygen atom in the formula (1) can be produced by reacting the intermediate compound (M4) with the intermediate compound (M5), in the presence of a base.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, water, and mixtures thereof.

Examples of the base include alkali metal carbonates such as sodium carbonate, cesium carbonate and potassium carbonate, and alkali metal hydrides such as sodium hydride.

In the reaction, the intermediate compound (M5) is usually used in a ratio of 0.8 to 1.2 mol, and the base is

usually used in a ratio of 1 to 2 mol, based on 1 mol of the intermediate compound (M4).

The reaction temperature is usually within the range of 0 to 200°C. The reaction time is usually within the range of 0.5 to 24 hours.

After completion of the reaction, the reaction mixture is extracted with an organic solvent, and the organic layer is subjected to post-treatment operations such as drying and concentration, whereby the compound of the present invention (P1) can be isolated. The isolated compound of the present invention (P1) also can be further purified by chromatography, recrystallization, or the like.

When $V^1$ is a chlorine atom, a bromine atom or an iodine atom, the compound of the present invention (P1) in which Q is an oxygen atom in the formula (1) can be produced by reacting the intermediate compound (M4) with the intermediate compound (M5) and a base, in the presence of a copper catalyst or a palladium catalyst.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, water, and mixtures thereof.

Examples of the base include alkali metal carbonates such as sodium carbonate and potassium carbonate, phosphates such as trisodium phosphate and tripotassium phosphate, alkali metal hydrides such as sodium hydride, tertiary amines such as triethylamine and N,N-diisopropylethylamine, and cyclic amines such as 1,4-diazabicyclo[2.2.2]octane (hereinafter, referred to as DABCO) and diazabicycloundecene (hereinafter, referred to as DBU).

Examples of the copper catalyst include copper (I) iodide, copper(I) bromide, copper(I) chloride, and copper(I) oxide.

Examples of the palladium catalyst include palladium(II) acetate and tris(dibenzylideneacetone) dipalladium(0).

The reaction can be also carried out by adding a ligand as necessary. Examples of the ligand include acetylacetone, salen, phenanthroline, triphenylphosphine, and 4,5'-bia(diphenylphosphino)-9,9'-dimethylxanthene.

In the reaction, the intermediate compound (M5) is usually used in a ratio of 0.8 to 1.2 mol, the base is usually used in a ratio of 1 to 2 mol, the copper catalyst is usually used in a ratio of 0.01 to 0.5 mol, the palladium catalyst is usually used in a ratio of 0.01 to 0.2 mol, and the ligand is usually used in a ratio of 0.01 to 0.5 mol, based on 1 mol of the intermediate compound (M4).

The reaction temperature is usually within the range of 0 to 200°C. The reaction time is usually within the range of 0.5 to 24 hours.

After completion of the reaction, the reaction mixture is extracted with an organic solvent, and the organic layer is subjected to post-treatment operations such as drying and concentration, whereby the compound of the present invention (P1) can be isolated. The isolated compound of the present invention (P1) also can be further purified by chromatography, recrystallization, or the like.

(Production Method 5)

The compound of the present invention (1) can be produced by reacting the intermediate compound (M6) with the intermediate compound (M7), in the presence of a base:

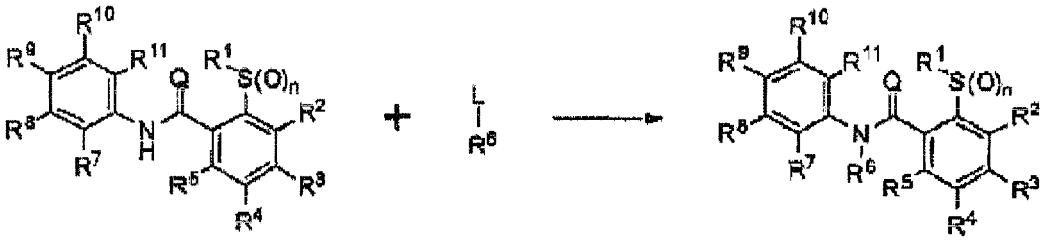

(M 6) (M 7) (1)

wherein L represents a leaving group such as a chlorine atom, a bromine atom, an iodine atom, a trifluoromethanesulfonyloxy group or a methanesulfonyloxy group, and other symbols represent the same meaning as described above.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, and mixtures thereof.

Examples of the base include hydrides of alkali metals or alkaline earth metals such as sodium hydride, potassium hydride and calcium hydride, inorganic bases such as sodium carbonate and potassium carbonate, organic bases such as triethylamine, and the like.

In the reaction, the intermediate compound (M7) is usually used in a ratio of 1 to 5 mol, and the base is usually used in a ratio of 1 to 2 mol, based on 1 mol of the intermediate compound (M6).
The reaction temperature is usually within the range of 0 to 100°C. The reaction time is usually within the range of 0.1 to 24 hours.
After completion of the reaction, the reaction mixture is extracted with an organic solvent, and the organic layer is subjected to post-treatment operations such as drying and concentration, whereby the compound of the present invention (1) can be isolated. The isolated compound of the present invention (1) also can be further purified by chromatography, recrystallization, or the like.

(Production Method 6)

The compound of the present invention (1) can be produced by reacting the intermediate compound (M6) with the intermediate compound (M8), in the presence of a condensing agent:

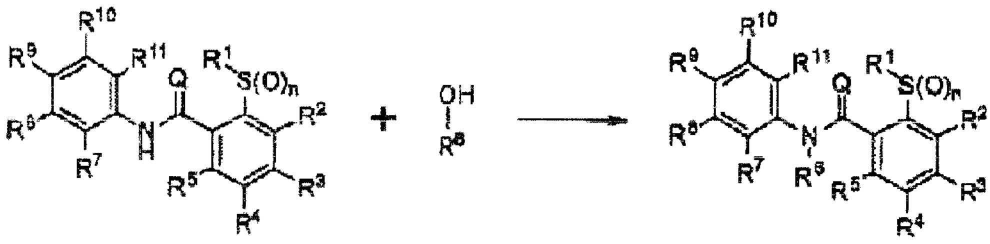

(M 6) (M 8) (1)

wherein symbols represent the same meaning as described above.
The reaction is usually carried out in the presence of a solvent.
Examples of the solvent include ethers such as THF, ethylene glycol dimethyl ether, tert-butyl methyl ether and 1,4-dioxane, aromatic hydrocarbons such as toluene and xylene, nitriles such as acetonitrile, aliphatic halogenated hydrocarbons such as dichloromethane and chloroform, aprotic polar solvents such as DMF, NMP and DMSO, and mixtures thereof.
Examples of the condensing agent include a mixture of diethyl azodicarboxylate and triphenylphosphine.
In the reaction, the intermediate compound (M8) is usually used in a ratio of 1 to 5 mol, and the condensing agent is usually used in a ratio of 1 to 2 mol, based on 1 mol of the intermediate compound (M6).
The reaction temperature is usually within the range of 0 to 150°C. The reaction time is usually within the range of 0.1 to 24 hours.
After completion of the reaction, the reaction mixture is extracted with an organic solvent, and the organic layer is subjected to post-treatment operations such as drying and concentration, whereby the compound of the present invention (1) can be isolated. The isolated compound of the present invention (1) also can be further purified by chromatography, recrystallization, or the like.

(Production Method 7)

The compound of the present invention (P2) in which Q is an oxygen atom and n is 0 in the formula (1) can be produced by reacting the intermediate compound (M11) with the intermediate compound (M12), in the presence of a base:

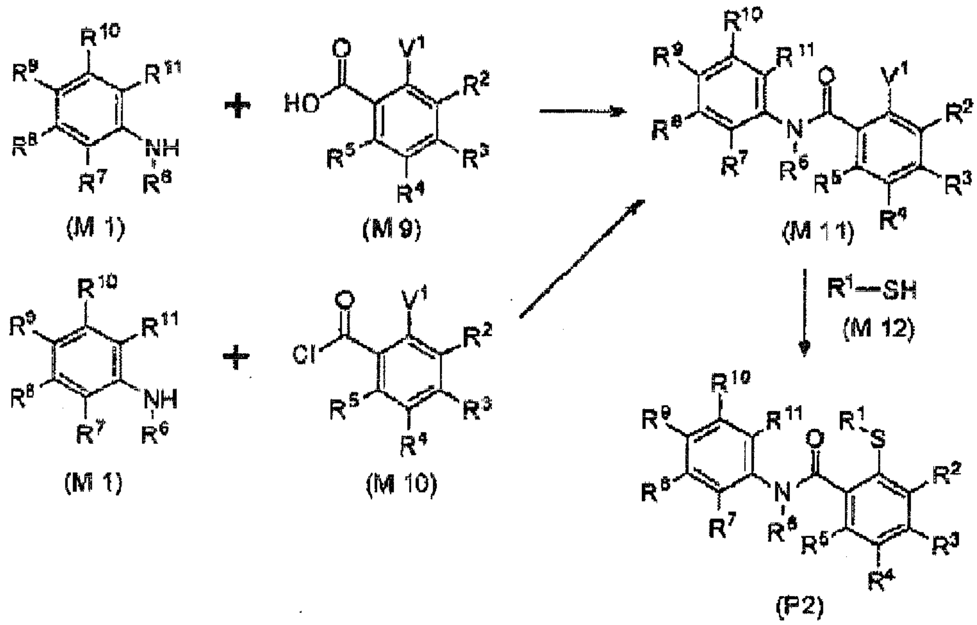


wherein symbols represent the same meaning as described above.

The intermediate compound (M11) can be produced, using the intermediate compound (M9) in place of the intermediate compound (M2), in accordance with the method of Production Method 2.

The intermediate compound (M11) oan be produced, using the intermediate compound (M10) in place of the intermediate compound (M2), in accordance with the method of Production Method 3.

The compound of the present invention (P2) in which Q is an oxygen atom and n is 0 in the formula (1) can be produced by reacting the intermediate compound (M11) with the intermediate compound (M12), in the presence of a base.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, water, and mixtures thereof.

Examples of the base include alkali metal carbonates such as sodium carbonate and potassium carbonate, and alkali metal hydrides such as sodium hydride.

In the reaction, the compound (M12) is usually used in a ratio of 0.8 to 1.2 mol, and the base is usually used in a ratio of 1 to 2 mol, based on 1 mol of the intermediate compound (M11).

The reaction temperature is usually within the range of 0 to 150°C. The reaction time is usually within the range of 0.5 to 24 hours.

After completion of the reaction, the reaction mixture is extracted with an organic solvent, and the organic layer is subjected to post-treatment operations such as drying and concentration, whereby the compound of the present invention (P2) can be isolated. The isolated compound of the present invention (P2) also can be further purified by chromatography, recrystallization, or the like.

In the reaction, $V^1$ is preferably a fluorine atom or a chlorine atom.

(Production Method 8)

The compound of the present invention (P2) in which Q is an oxygen atom and n is 0 in the formula (1) can be produced by producing the intermediate compound (M13) using sodium sulfide, sodium hydrogen sulfide or hydrogen sulfide in place of the intermediate compound (M12), in accordance with the method of Production Method 7, and reacting the intermediate compound (M13) with the intermediate compound (M14), in the presence of a base:

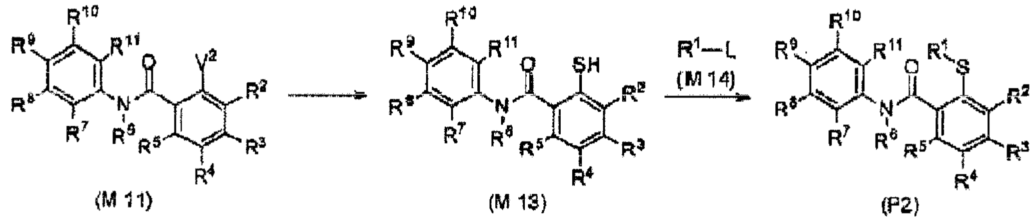


wherein symbols represent the same meaning as described above.

The intermediate compound (M13) can be produced, using sodium sulfide, sodium hydrogen sulfide or hydrogen sulfide in place of the intermediate compound (M12), in accordance with the method of Production Method 7. Here, when using sodium sulfide or sodium hydrogen sulfide in place of the intermediate compound (M12), the intermediate compound (M13) can be usually produced by the reaction in the absence of a base. Also, when using hydrogen sulfide in place of the intermediate compound (M12), the intermediate compound (M13) can be usually produced by the reaction in the presence of a base.

The compound of the present invention (P2) in which Q is an oxygen atom and n is 0 in the formula (1) can be produced by reacting the intermediate compound (M13) with the intermediate compound (M14), in the presence of a base.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, and mixtures thereof.

Examples of the base include hydrides of alkali metals or alkaline earth metals such as sodium hydride, potassium hydride and calcium hydride, inorganic bases such as sodium carbonate and potassium carbonate, and organic bases such as triethylamine.

In the reaction, the intermediate compound (M14) is usually used in a ratio of 1 to 5 mol, and the base is usually used in a ratio of 1 to 2 mol, based on 1 mol of the intermediate compound (M13).

The reaction temperature is usually within the range of 0 to 100°C. The reaction time is usually within the range of 0.1 to 24 hours.

After completion of the reaction, the reaction mixture is extracted with an organic solvent, and the organic layer is subjected to post-treatment operations such as drying and concentration, whereby the compound of the present invention (P2) can be isolated. The isolated compound of the present invention (P2) also can be further purified by chromatography, recrystallization, or the like.

(Production Method 9)

The compound of the present invention (P3) in which Q is a sulfur atom in the formula (1) can be produced by reacting the compound of the present invention (P1) in which Q is an oxygen atom in the formula (1) with a sulfurizing agent:

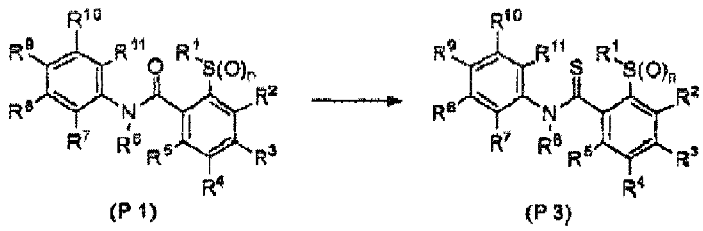


wherein symbols represent the same meaning as described above.

The reaction is usually carried out in the presence or absence of a solvent.

Examples of the solvent include ethers such as 1,4-dioxane, diethyl ether, tetrahydrofuran, tert-butyl methyl ether and diglyme, halogenated hydrocarbons such as dichloromethane, chloroform, carbon tetrachloride, 1,2-dichloroethane and chlorobenzene, aromatic hydrocarbons such as toluene, benzene and xylene, nitriles such as acetonitrile, nitrogen-containing aromatic compounds such as pyridine, picoline, lutidine and quinoline, and mixtures thereof.

The sulfurizing agent includes diphosphorus pentasulfide, Lawesson's reagent (2,4-bis-(4-methoxyphenyl)-1,3-dithia-2,4-diphoephetane-2,4 -disulfide), and the like.

In the reaction, the sulfurizing agent is usually used in a ratio of 1 to 3 mol, based on 1 mol of the compound of the present invention (P1).

The reaction temperature is usually within the range of 0°C to 200°C, and the reaction time is usually within the range of 1 to 24 hours.

After completion of the reaction, the compound of the present invention (P3) can be isolated by adding the reaction mixture to water, then extracting the mixture with an organic solvent, and concentrating the organic layer; collecting a solid generated by adding the reaction mixture to water by filtration; or collecting a solid generated in the reaction mixture by filtration. The isolated compound of the present invention (P3) also can be further purified by recrystallization, chromatography, or the like.

(Production Method 10)

The intermediate compound (M2) can be produced by hydrolyzing the intermediate compound (M15). Also, the intermediate compound (M3) can be produced by reacting the intermediate compound (M2) with a chlorinating agent:

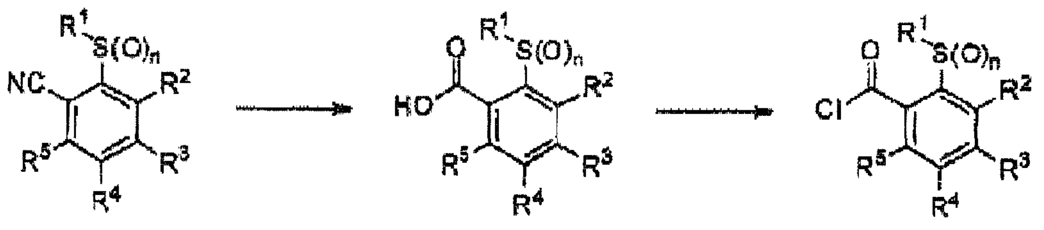

(M 15) (M 2) (M 3)

wherein symbols represent the same meaning as described above.

The intermediate compound (M2) can be produced by hydrolyzing the intermediate compound (M15).

When hydrolyzed by an acid, the hydrolysis is usually carried out using an aqueous solution of an acid as a solvent.

Examples of the acid used in the hydrolysis include mineral acids such as hydrochloric acid, nitric acid, phosphoric acid and sulfuric acid, carboxylic acids such as acetic acid and trifluoroacetic acid.

The reaction temperature is usually within the range of 0 to 15C°C. The reaction time is usually within the range of 0.1 to 24 hours.

After completion of the reaction, the reaction mixture is extracted with an organic solvent, and the organic layer is subjected to post-treatment operations such as drying and concentration, whereby the intermediate compound (M2) can be isolated. The isolated intermediate compound (M2) also can be further purified by chromatography, recrystallization, or the like.

When hydrolyzed by a base, the hydrolysis is usually carried out in the presence of a solvent.

Examples of the solvent include ethers such as THF, ethylene glycol dimethyl ether, tert-butyl methyl ether and 1,4-dioxane, alcohols such as methanol and ethanol, water, and mixtures thereof.

Examples of the base include alkali metal hydroxides such as sodium hydroxide and potassium hydroxide.

In the hydrolysis, the base is usually used in a ratio of 1 to 10 mol, based on 1 mol of the intermediate compound (M15).

The reaction temperature is usually within the range of 0 to 120°C. The reaction time is usually within the range of 0.1 to 24 hours.

After completion of the reaction, the reaction solution is acidified, then the reaction mixture is extracted with an organic solvent, and the organic layer is subjected to post-treatment operations such as drying and concentration, whereby the intermediate compound (M2) can be isolated. The isolated intermediate compound (M2) also can be further purified by chromatography, recrystallization, or the like.

The intermediate compound (M3) can be produced by allowing the intermediate compound (M2) to react with a chlorinating agent.

The reaction is usually carried out in the presence of a solvent.

Examples of the solvent include ethers such as THF, ethylene glycol dimethyl ether, tert-butyl methyl ether and 1,4-dioxane, aromatic hydrocarbons such as toluene and xylene, aliphatic halogenated hydrocarbons such as dichloromethane and chloroform, and mixtures thereof.

The chlorinating agent includes thionyl chloride, oxalyl dichloride, phosphorus oxychloride, and the like.

The reaction can be also carried out by adding DMF as necessary.

In the reaction, the chlorinating agent is usually used in a ratio of 1 to 5 mol, and DMF is usually used in a ratio of 0.001 to 0.1 mol, based on 1 mol of the intermediate compound (M2).

The reaction temperature is usually within the range of 0 to 100°C. The reaction time is usually within the range

of 0.1 to 24 hours.

After completion of the reaction, the intermediate compound (M3) oan be isolated by distilling the solvent. Alternatively, the intermediate compound (M3) can be used in the next reaction as it is without isolation.

(Production Method 11)

The intermediate compound (M2-n0) in which n is 0 in the intermediate compound (M2) can be produced by reacting the intermediate compound (M16) with the intermediate compound (M12), in the presence of a base. Also, the intermediate compound (M2-n1) or intermediate compound (M2-n2) in which n is 1 or 2 in the intermediate compound (M2) can be produced by oxidizing the intermediate compound (M2) in which n is 0:

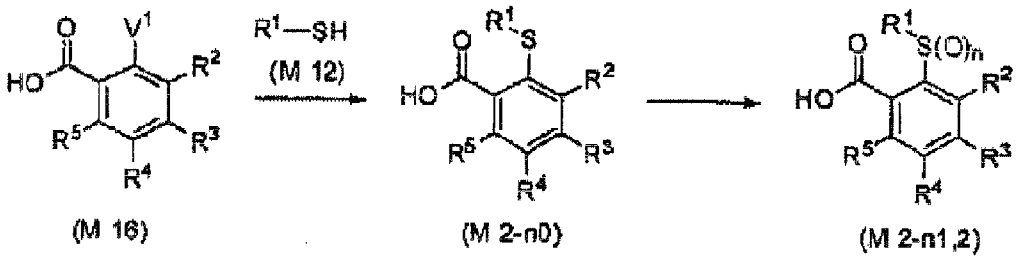


(M 16) (M 2-n0) (M 2-n1,2)

wherein symbols represent the same meaning as described above.

The intermediate compound (M2-n0) in which n is 0 can be produced, using the intermediate compound (M16) in place of the intermediate compound (M11), in accordance with the method of Production Method 7.

In the reaction, $V^1$ is preferably a fluorine atom or a chlorine atom.

The intermediate compound (M2-n1) or intermediate compound (M2-n2) in which n is 1 or 2 can be produced, using the intermediate compound (M2-n0) in which n is 0 in place of the compound of the present invention (1-n0) in which n is 0, in accordance with the method of Production Method 1.

(Production Method 12)

The intermediate compound (M6-QO) in which Q is an oxygen atom in the intermediate compound (M6) can be produced, using the intermediate compound (M17) in place of the intermediate compound (M1), in accordance with the method of Production Method 2. Also, the intermediate compound (M6) can be produced, using the intermediate compound (M17) in place of the intermediate compound (M1), in accordance with the method of Production Method 3.

The intermediate compound (M6-QS) in which Q is a sulfur atom in the intermediate compound (M6) can be produced, using the intermediate compound (M6-QO) in which Q is an oxygen atom in place of the compound of the present invention (P1), in accordance with the method of Production Method 9:

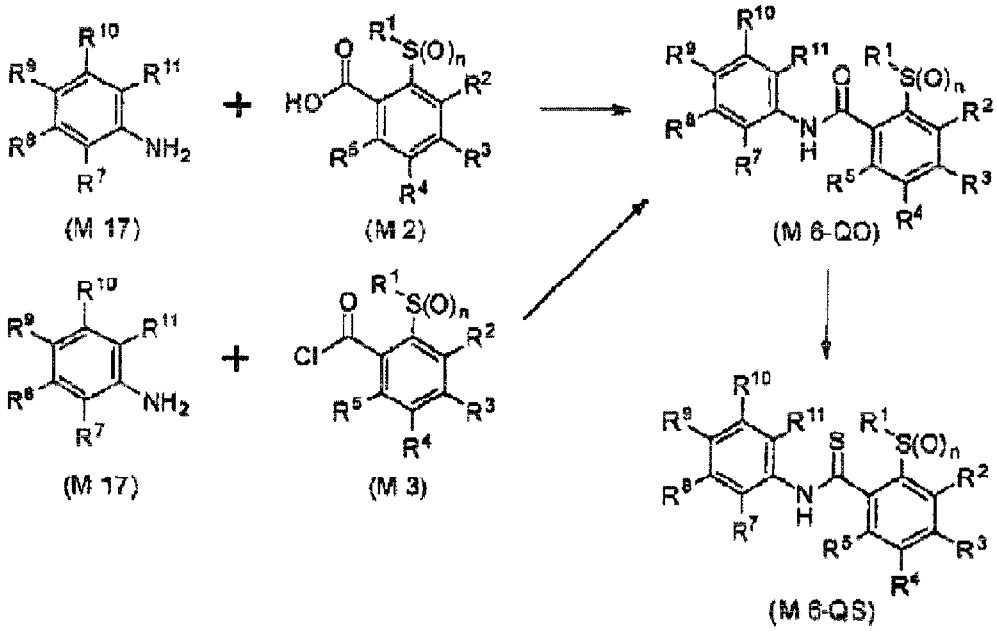

(M 17) (M 2) (M 6-QO) (M 17) (M 3) (M 6-QS)

wherein symbols represent the same meaning as described above.

(Production Method 13)

Among the compounds of the present invention and the above-described intermediate compounds, a compound having a nitrogen-containing heterocyclic group having a lone pair of electrons on the nitrogen atom is reacted with an oxidizing agent, whereby an N-oxide in which the nitrogen atom is oxidized can be manufactured in some cases.

Examples of the nitrogen-containing heterocyclic group include a pyridine ring.

The reaction can be carried out by a known method, and is carried out using an oxidizing agent such as m-chloroperbenzoic acid or hydrogen peroxide, in a solvent, for example, a halogenated hydrocarbon such as dichloromethane, chloroform or chlorobenzene, an alcohol such as methanol or ethanol, acetic acid, water, and mixtures thereof.

Examples of the intermediate compound (M6) include following compounds.

Compounds represented by formula (M6):

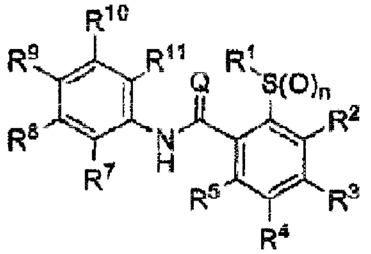

(M 6)

wherein symbols represent the same meaning as described above, or N-oxides thereof.

In the formula (M6), compounds, wherein

$R^1$ is an ethyl group, a cyclopropyl group or a cyclopropylmethyl group,

$R^2$, $R^4$ and $R^5$ are a hydrogen atom,

$R^3$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z, $-OR^{12}$, $-S(O)_mR^{14}$ $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, a halogen atom or a hydrogen atom,

$R^7$ and $R^{11}$ are a hydrogen atom,

$R^8$, $R^9$ and $R^{10}$ are the same or different and are a C1 to C6 perfluoroalkyl group, a C1 to C6 perfluoroalkoxy group, a C1 to C6 perfluoroalkylsulfanyl group, a C1 to C6 perfluoroalkylsulfinyl group, a C1 to C6 perfluoroalkylsulfonyl group, $-SF_5$, a cyano group, a nitro group, a halogen atom, or a hydrogen atom (wherein $R^8$, $R^9$ and $R^{10}$ do not simultaneously

represent a hydrogen atom),
$R^{12}$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z or a hydrogen atom,
$R^{14}$ is a C1 to C6 alkyl group optionally having one or more halogen atoms, a phenyl group optionally having one or more atoms or groups selected from group Z or a 5- or 6-membered heterocyclic group optionally having one or more atoms or groups selected from group Z,
Q is an oxygen atom or a sulfur atom,
m is 0, 1 or 2, and
n is 0, 1 or 2.

In the formula (M6), compounds, wherein
$R^1$ is an ethyl group, a cyclopropyl group or a cyclopropylmethyl group,
$R^2$, $R^4$ and $R^5$ are a hydrogen atom,
$R^3$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z, $-OR^{12}$, $-S(O)_mR^{14}$ or a halogen atom,
$R^7$ and $R^{11}$ are a hydrogen atom,
$R^8$, $R^9$ and $R^{10}$ are the same or different and are a C1 to C6 perfluoroalkyl group, a C1 to C6 perfluoroalkoxy group, a C1 to C6 perfluoroalkylsulfanyl group, a C1 to C6 perfluoroalkylsulfinyl group, a C1 to C6 perfluoroalkylsulfonyl group, $-SF_5$, a oyano group, a nitro group, a halogen atom, or a hydrogen atom (wherein $R^8$, $R^9$ and $R^{10}$ do not simultaneously represent a hydrogen atom),
$R^{12}$ is a C1 to C6 alkyl group optionally having one or more halogen atoms, a phenyl group optionally having one or more atoms or groups selected from group Z, a 5- or 6-membered heterocyclic group optionally having one or more atoms or groups selected from group Z or a hydrogen atom,
$R^{14}$ is a C1 to C6 alkyl group optionally having one or more halogen atoms, a phenyl group optionally having one or more atoms or groups selected from group Z or a 5- or 6-membered heterocyclic group optionally having one or more atoms or groups selected from group Z,
Q is an oxygen atom or a sulfur atom,
m is 0, 1 or 2, and
n is 0, 1 or 2.

In the formula (M6), compounds wherein Q is an oxygen atom.

In the formula (M6), compounds, wherein
$R^1$ is an ethyl group, a cyclopropyl group or a cyclopropylmethyl group,
$R^2$, $R^4$ and $R^5$ are a hydrogen atom,
$R^3$ is a methyl group, an ethyl group, a vinyl group, a propyl group, an isopropyl group, a cyclopropyl group, a propargyl group, a trifluoromethyl group, a pentafluoroethyl group, a heptafluoropropyl group, a heptafluoroisopropyl group, a methoxy group, a trifluoromethoxy group, a methylsulfanyl group, a trifluoromethylsulfanyl group, a methylsulfinyl group, a trifluoromethylsulfinyl group, a methylsulfonyl group, a trifluoromethylsulfonyl group, a 2-pyridyl group, a 2-pyrimidinyl group, a 5-trifluoromethyl-2-pyridyl group, a 3-chloro-5-trifluoromethyl-2-pyridyl group, a fluorine atom, a chlorine atom, a bromine atom, or an iodine atom,
$R^1$ and $R^{11}$ are a hydrogen atom,
$R^8$, $R^9$ and $R^{10}$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group or a trifluoromethylsulfonyl group, a fluorine atom, a chlorine atom, a bromine atom, or a hydrogen atom (wherein $R^8$, $R^9$ and $R^{10}$ do not simultaneously represent a hydrogen atom), and
Q is an oxygen atom.

Next, specific examples of the compound of the present invention are shown below.

In the formula (1):

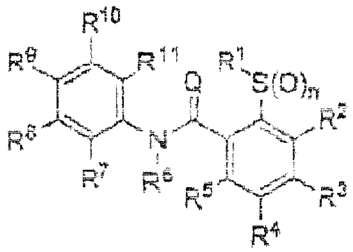
(1)

wherein n is 0, $R^2$, $R^3$, $R^4$, $R^5$, $R^7$ and $R^{11}$ are a hydrogen atom,
Q is an oxygen atom, and $R^1$, $R^6$, $R^8$, $R^9$ and $R^{10}$ are the combinations shown in [Table 1] to [Table 24].
In Tables 2, 3, 5, 6, 8, 9, 11, 12, 14, 15, 17, 18, 20, 21, 23 and 24, all examples of $R^6$ are reference examples.
In Tables 1, 4, 7, 10, 13, 16, 19 and 22, reference examples are identified by an asterisk (*).

[Table 1]

| $R^1$ | $R^6$ | $R^8$ | $R^9$ | $R^{10}$ |
|---|---|---|---|---|
| Me | Me | H | $CF_3$ $CF_3$ | H |
| Et | Me | H | $CF_3$ | H |
| Pr | Me | H | $CF_3$ | H |
| $CH_2CF_3$ | Me | H | $CF_3$ | H |
| $CH_2C{\equiv}CH$ | Me | H | $CF_3$ | H |
| CycPr | Me | H | $CF_3$ | H |
| $CH_2$CycPr | Me | H | $CF_3$ | H |
| Me | Et | H | $CF_3$ | H |
| Et | Et | H | $CF_3$ | H |
| Pr | Et | H | $CF_3$ | H |
| $CH_2CF_3$ | Et | H | $CF_3$ | H |
| $CH_2C{\equiv}CH$ | Et | H | $CF_3$ | H |
| CycPr | Et | H | $CF_3$ | H |
| $CH_2$CycPr | Et | H | $CF_3$ | H |
| Me | CycPr * | H | $CF_3$ | H |
| Et | CycPr * | H | $CF_3$ | H |
| Pr | CycPr * | H | $CF_3$ | H |
| $CH_2CF_3$ | CycPr * | H | $CF_3$ | H |
| $CH_2C{\equiv}CH$ | CycPr * | H | $CF_3$ | H |
| CycPr | CycPr * | H | $CF_3$ | H |
| $CH_2$CycPr | CycPr * | H | $CF_3$ | H |

[Table 2]

| $R^1$ | $R^6$ | $R^8$ | $R^9$ | $R^{10}$ |
|---|---|---|---|---|
| Me | $CH_2$CycPr | H | $CF_3$ | H |
| Et | $CH_2$CycPr | H | $CF_3$ | H |
| Pr | $CH_2$CycPr | H | $CF_3$ | H |
| $CH_2CF_3$ | $CH_2$CycPr | H | $CF_3$ | H |
| $CH_2O{\equiv}CH$ | $CH_2$CycPr | H | $CF_3$ | H |
| CycPr | $CH_2$CycPr | H | $CF_3$ | H |
| $CH_2$CycPr | $CH_2$CycPr | H | $CF_3$ | H |
| Me | $CH_2C{\equiv}CH$ | H | $CF_3$ | H |
| Et | $CH_2C{\equiv}CH$ | H | $CF_3$ | H |
| Pr | $CH_2C{\equiv}MCH$ | H | $CF_3$ | H |

(continued)

| $R^1$ | $R^6$ | $R^8$ | $R^9$ | $R^{10}$ |
|---|---|---|---|---|
| $CH_3CF_3$ | $CH_2C{\equiv}CH$ | H | $CF_3$ | H |
| $CH_2C{\equiv}CH$ | $CH_2{\equiv}CH$ | H | $CF_3$ | H |
| CycPr | $CH_2C{\equiv}CH$ | H | $CF_3$ | H |
| $CH_2$CycPr | $CH_2C{\equiv}CH$ | H | $CF_3$ | H |
| Me | $CH_2OCH_3$ | H | $CF_3$ | H |
| Et | $CH_3CCH_3$ | H | $CF_3$ | H |
| Pr | $CH_2OCH_3$ | H | $CF_3$ | H |
| $CH_2CF_3$ | $CH_2OCH_3$ | H | $CF_3$ | H |
| $CH_2C{\equiv}CH$ | $CH_2OCH_3$ | H | $CF_3$ | H |
| CycPr | $CH_2OCH_3$ | H | $CF_3$ | H |
| $CH_2$CycPr | $CH_2OCH_3$ | H | $CF_3$ | H |

[Table 3]

| $R^1$ | $R^6$ | $R^8$ | $R^9$ | $R^{10}$ |
|---|---|---|---|---|
| Me | $CO_2CH_3$ | H | $CF_3$ | H |
| Et | $CO_2CH_3$ | H | $CF_3$ | H |
| Pr | $CO_2CH_3$ | H | $CF_3$ | H |
| $CH_3CF_3$ | $CO_3CH_3$ | H | $CF_3$ | H |
| $CH_2C{\equiv}CH$ | $CO_2CH_3$ | H | $CF_3$ | H |
| CycPr | $CO_2CH_3$ | H | $CF_3$ | H |
| $CH_3$CycPr | $CO_2CH_3$ | H | $CF_3$ | H |
| Me | 6-chloro-pyridin-3-ylmethyl | H | $CF_3$ | H |
| Et | 6-chloro-pyridin-3-ylmethyl | H | $CF_3$ | H |
| Pr | 6-chloro-pyridin-3-ylmethyl | H | $CF_3$ | H |
| $CH_2CF_3$ | 6-chloro-pyridin-3-ylmethyl | H | $CF_3$ | H |
| $CH_2C{\equiv}CH$ | 6-chloro-pyridin-3-ylmethyl | H | $CF_3$ | H |
| CycPr | 6-chloro-pyridin-3-ylmethyl | H | $CF_3$ | H |
| $CH_2$CycPr | 6-chloro-pyridin-3-ylmethyl | H | $CF_3$ | H |
| Me | 2-chloro-thiazol-5-ylmethyl | H | $CF_3$ | H |
| Et | 2-chloro-thiazol-5-ylmethyl | H | $CF_3$ | H |
| Pr | 2-chloro-thiazol-5-ylmethyl | H | $CF_3$ | H |
| $CH_2CF_3$ | 2-chloro-thiazol-5-ylmethyl | H | $CF_3$ | H |
| $CH_2C{\equiv}CH$ | 2-chloro-thiazol-5-ylmethyl | H | $CF_3$ | H |
| CycPr | 2-chloro-thiazol-5-ylmethyl | H | $CF_3$ | H |
| $CH_3$CycPr | 2-chloro-thiazol-5-ylmethyl | H | $CF_3$ | H |

[Table 4]

| $R^1$ | $R^6$ | $R^8$ | $R^5$ | $R^{10}$ |
|---|---|---|---|---|
| Me | Me | H | $CF_3CF_3$ | H |
| Et | Me | H | $CF_2CF_3$ | H |
| Pr | Me | H | $CF_2CF_3$ | H |
| $CH_3CF_3$ | Me | H | $CF_2CF_3$ | H |
| $CH_2C{\equiv}CH$ | Me | H | $CF_2F_3$ | H |
| CycPr | Me | H | $CF_2CF_3$ | H |
| $CH_2$CycPr | Me | H | $CF_2CF_3$ | H |
| Me | Et | H | $CF_2CF_3$ | H |
| Et | Et | H | $CF_2CF_3$ | H |
| Pr | Et | H | $CF_2CF_3$ | H |
| $CH_2CF_3$ | Et | H | $CF_2CF_3$ | H |
| $CH_2C{\equiv}CH$ | Et | H | $CF_2CF_3$ | H |
| CycPr | Et | H | $CF_2CF_3$ | H |
| $CH_2$CycPr | Et | H | $CF_2CF_3$ | H |
| Me | CycPr * | H | $CF_2CF_3$ | H |
| Et | CycPr * | H | $CF_2CF_3$ | H |
| Pr | CycPr * | H | $CF_2CF_3$ | H |
| $CH_2CF_3$ | CycPr * | H | $CF_3CF_3$ | H |
| $CH_2C{\equiv}CH$ | CycPr * | H | $CF_2CF_3$ | H |
| CycPr | CycPr * | H | $CF_2CF_3$ | H |
| $CH_2$CycPr | CycPr * | H | $CF_2CF_3$ | H |

[Table 5]

| $R^1$ | $R^6$ | $R^8$ | $R^9$ | $R^{10}$ |
|---|---|---|---|---|
| Me | $CH_3$CycPr | H | $CF_2CF_3$ | H |
| Et | $CH_2$CycPr | H | $CF_2CF_3$ | H |
| Pr | $CH_2$CycPr | H | $CF_2CF_3$ | H |
| $CH_3CF_3$ | $CH_3$CycPr | H | $CF_2CF_3$ | H |
| $CH_2C{\equiv}CH$ | $CH_2$CycPr | H | $CF_2CF_3$ | H |
| CycPr | $CH_2$CycPr | H | $CF_2CF_3$ | H |
| $CH_2$CycPr | $CH_2$CycPr | H | $CF_2CF_3$ | H |
| Me | $CH_2C{\equiv}CH$ | H | $CF_2CF_3$ | H |
| Et | $CH_2C{\equiv}CH$ | H | $CF_2CF_3$ | H |
| Pr | $CH_2C{\equiv}CH$ | H | $CF_2CF_3$ | H |
| $CH_2CF_3$ | $CH_2C{\equiv}CH$ | H | $CF_2CF_3$ | H |
| $CH_3C{\equiv}CH$ | $CH_2C{\equiv}CH$ | H | $CF_2CF_3$ | H |
| CycPr | $CH_2C{\equiv}CH$ | H | $CF_2CF_3$ | H |
| $CH_2$CycPr | $CH_2C{\equiv}CH$ | H | $CF_2CF_3$ | H |

(continued)

| $R^1$ | $R^6$ | $R^8$ | $R^9$ | $R^{10}$ |
|---|---|---|---|---|
| Me | $CH_2OCH_3$ | H | $CF_2CF_3$ | H |
| Et | $CH_2OCH_3$ | H | $CF_2CF_3$ | H |
| Pr | $CH_2OCH_3$ | H | $CF_2CF_3$ | H |
| $CH_3CF_3$ | $CH_2OCH_3$ | H | $CF_2CF_3$ | H |
| $CH_2C{\equiv}CH$ | $CH_2OCH_3$ | H | $CF_2CF_3$ | H |
| CycPr | $CH_2OCH_3$ | H | $CF_2CF_3$ | H |
| $CH_2$CycPr | $CH_2OCH_3$ | H | $CF_2CF_3$ | H |

[Table 6]

| $R^1$ | $R^6$ | $R^8$ | $R^9$ | $R^{10}$ |
|---|---|---|---|---|
| Me | $CO_2CH_3$ | H | $CF_2CF_3$ | H |
| Et | $CO_2CH_3$ | H | $CF_2CF_3$ | H |
| Pr | $CO_2CH_3$ | H | $CF_2CF_3$ | H |
| $CH_2CF_3$ | $CO_2CH_3$ | H | $CF_2CF_3$ | H |
| $CH_2C{\equiv}CH$ | $CO_2CH_3$ | H | $CF_2CF_3$ | H |
| CycPr | $CO_2CH_3$ | H | $CF_2CF_3$ | H |
| $CH_2$CycPr | $CO_2CH_3$ | H | $CF_2CF_3$ | H |
| Me | 6-chloro-pyridin-3-ylmethyl | H | $CF_2CF_3$ | H |
| Et | 6-chloro-pyridin-3-ylmethyl | H | $CF_2CF_3$ | H |
| Pr | 6-chloro-pyridin-3-ylmethyl | H | $CF_2CF_3$ | H |
| $CH_2CF_3$ | 6-chloro-pyridin-3-ylmethyl | H | $CF_2CF_3$ | H |
| $CH_2C{\equiv}CH$ | 6-chloro-pyridin-3-ylmethyl | H | $CF_2CF_3$ | H |
| CycPr | 6-chloro-pyridin-3-ylmethyl | H | $CF_2CF_3$ | H |
| $CH_2$CycPr | 6-chloro-pyridin-3-ylmethyl | H | $CF_2CF_3$ | H |
| Me | 2-chloro-thiazol-5-ylmethyl | H | $CF_2CF_3$ | H |
| Et | 2-chloro-thiazol-5-ylmethyl | H | $CF_2CF_3$ | H |
| Pr | 2-chloro-thiazol-5-ylmethyl | H | $CF_2CF_3$ | H |
| $CH_2CF_3$ | 2-chloro-thiazol-5-ylmethyl | H | $CF_2CF_3$ | H |
| $CH_2C{\equiv}CH$ | 2-chloro-thiazol-5-ylmethyl | H | $CF_2CF_3$ | H |
| CycPr | 2-chloro-thiazol-5-ylmethyl | H | $CF_2CF_3$ | H |
| $CH_2$CycPr | 2-chloro-thiazol-5-ylmethyl | H | $CF_2CF_3$ | H |

[Table 7]

| $R^1$ | $R^6$ | $R^8$ | $R^9$ | $R^{10}$ |
|---|---|---|---|---|
| Me | Me | H | $CF(CF_3)_2$ | H |
| Et | Me | H | $CF(CF_3)_2$ | H |
| Pr | Me | H | $CF(CF_3)_2$ | H |

(continued)

| $R^1$ | $R^6$ | $R^8$ | $R^9$ | $R^{10}$ |
|---|---|---|---|---|
| $CH_2CF_3$ | Me | H | $CF(CF_3)_2$ | H |
| $CH_2C{\equiv}CH$ | Me | H | $CF(CF_3)_2$ | H |
| CycPr | Me | H | $CF(CF_3)_2$ | H |
| $CH_2$CycPr | Me | H | $CF(CF_3)_2$ | H |
| Me | Et | H | $CF(CF_3)_2$ | H |
| Et | Et | H | $CF(CF_3)_2$ | H |
| Pr | Et | H | $CF(CF_3)_2$ | H |
| $CH_2CF_3$ | Et | H | $CF(CF_3)_2$ | H |
| $CH_2C{\equiv}CH$ | Et | H | $CF(CF_3)_2$ | H |
| CycPr | Et | H | $CF(CF_3)_2$ | H |
| $CH_2$CycPr | Et | H | $CF(CF_3)_2$ | H |
| Me | CycPr * | H | $CF(CF_3)_2$ | H |
| Et | CycPr * | H | $CF(CF_3)_2$ | H |
| Pr | CycPr * | H | $CF(CF_3)_2$ | H |
| $CH_2CF_3$ | CycPr * | H | $CF(CF_3)_2$ | H |
| $CH_2C{\equiv}CH$ | CycPr * | H | $CF(CF_3)_2$ | H |
| CycPr | CycPr * | H | $CF(CF_3)_2$ | H |
| $CH_2$CycPr | CycPr * | H | $CF(CF_3)_2$ | H |

[Table 8]

| $R^1$ | $R^6$ | $R^8$ | $R^9$ | $R^{10}$ |
|---|---|---|---|---|
| Me | $CH_3$CycPr | H | $CF(CF_3)_2$ | H |
| Et | $CH_2$CycPr | H | $CF(CF_3)_2$ | H |
| Pr | $CH_3$CycPr | H | $CF(CF_3)_2$ | H |
| $CH_3CF_3$ | $CH_2$CycPr | H | $CF(CF_3)_2$ | H |
| $CH_2C{\equiv}CH$ | $CH_2$CycPr | H | $CF(CF_3)_2$ | H |
| CycPr | $CH_2$CycPr | H | $CF(CF_3)_2$ | H |
| $CH_2$CycPr | $CH_2$CycPr | H | $CF(CF_3)_2$ | H |
| Me | $CH_2C{\equiv}CH$ | H | $CF(CF_3)_2$ | H |
| Et | $CH_2C{\equiv}CH$ | H | $CF\ (CF_3)_2$ | H |
| Pr | $CH_2C{\equiv}CH$ | H | $CF(CF_3)_2$ | H |
| $CH_2CF_3$ | $CH_2C{\equiv}CH$ | H | $CF(CF_3)_2$ | H |
| $CH_2C{\equiv}CH$ | $CH_2C{\equiv}CH$ | H | $CF(CF_3)$ | H |
| CycPr | $CH_2C{\equiv}CH$ | H | $CF(CF_3)_2$ | H |
| $CH_2$CycPr | $CH_2C{\equiv}CH$ | H | $CF(CF_3)_2$ | H |
| Me | $CH_2OCH_3$ | H | $CF(CF_3)_2$ | H |
| Et | $CH_2OCH_3$ | H | $CF(CF_3)_2$ | H |

(continued)

| $R^1$ | $R^6$ | $R^8$ | $R^9$ | $R^{10}$ |
|---|---|---|---|---|
| Pr | $CH_2OCH_3$ | H | $CF(CF_3)_2$ | H |
| $CH_2CF_3$ | $CH_2OCH_3$ | H | $CF(CF_3)_2$ | H |
| $CH_2C{\equiv}CH$ | $CH_2OCH_3$ | H | $CF(CF_3)_2$ | H |
| CycPr | $CH_3OCH_3$ | H | $CF(CF_3)_2$ | H |
| $CH_2CycPr$ | $CH_2OCH_3$ | H | $CF(CF_3)_2$ | H |

[Table 9]

| $R^1$ | $R^6$ | $R^8$ | $R^9$ | $R^{10}$ |
|---|---|---|---|---|
| Me | $CO_2CH_3$ | H | $CF(CF_3)_2$ | H |
| Et | $CO_2CH_3$ | H | $CF(CF_3)_2$ | H |
| Pr | $CO_2CH_3$ | H | $CF(CF_3)_2$ | H |
| $CH_2CF_3$ | $CO_2CH_3$ | H | $CF(CF_3)_2$ | H |
| $CH_2C{\equiv}CH$ | $CO_2CH_3$ | H | $CF(CF_3)_2$ | H |
| CycPr | $CO_2CH_3$ | H | $CF(CF_3)_2$ | H |
| $CH_2CycPr$ | $CO_2CH_3$ | H | $CF(CF_3)_2$ | H |
| Me | 6-chloro-pyridin-3-ylmethyl | H | $CF(CF_3)_2$ | H |
| Et | 6-chloro-pyridin-3-ylmethyl | H | $CF(CF_3)_2$ | H |
| Pr | 6-chloro-pyridin-3-ylmethyl | H | $CF\ (CF_3)\ _2$ | H |
| $CH_2CF_3$ | 6-chloro-pyridin-3-ylmethyl | H | $CF(CF_3)_2$ | H |
| $CH_3C{\equiv}CH$ | 6-chloro-pyridin-3-ylmethyl | H | $CF(CF_3)_2$ | H |
| CycPr | 6-chloro-pyridin-3-ylmethyl | H | $CF(CF_3)_2$ | H |
| $CH_2CycPr$ | 6-chloro-pyridin-3-ylmethyl | H | $CF(CF_3)_2$ | H |
| Me | 2-chloro-thiazol-5-ylmethyl | H | $CF(CF_3)_2$ | H |
| Et | 2-chloro-thiazol-5-ylmethyl | H | $CF(CF_3)_2$ | H |
| Pr | 2-chloro-thiazol-5-ylmethyl | H | $CF(CF_3)_2$ | H |
| $CH_2CF_3$ | 2-chloro-thiazol-5-ylmethyl | H | $CF(CF_3)_2$ | H |
| $CH_2C{\equiv}CH$ | 2-chloro-thiazol-5-ylmethyl | H | $CF(CF_3)_2$ | H |
| CycPr | 2-chloro-thiazol-5-ylmethyl | H | $CF(CF_3)_2$ | H |
| $CH_2CycPr$ | 2-chloro-thiazol-5-ylmethyl | H | $CF(CF_3)_2$ | H |

[Table 10]

| $R^1$ | $R^6$ | $R^8$ | $R^9$ | $R^{10}$ |
|---|---|---|---|---|
| Me | Me | H | $OCF_3$ | H |
| Et | Me | H | $OCF_3$ | H |
| Pr | Me | H | $OCF_3$ | H |
| $CH_2CF_3$ | Me | H | $OCF_3$ | H |
| $CH_2C{\equiv}CH$ | Me | H | $OCF_3$ | H |

(continued)

| $R^1$ | $R^6$ | $R^8$ | $R^9$ | $R^{10}$ |
|---|---|---|---|---|
| CycPr | Me | H | $OCF_3$ | H |
| $CH_2$CycPr | Me | H | $OCF_3$ | H |
| Me | Et | H | $OCF_3$ | H |
| Et | Et | H | $OCF_3$ | H |
| Pr | Et | H | $CCF_3$ | H |
| $CH_2CF_3$ | Et | H | $OCF_3$ | H |
| $CH_2C{\equiv}CH$ | Et | H | $OCF_3$ | H |
| CycPr | Et | H | $OCF_3$ | H |
| $CH_2$CycPr | Et | H | $OCF_2$ | H |
| Me | CycPr * | H | $OCF_3$ | H |
| Et | CycPr * | H | $OCF_3$ | H |
| Fr | CycPr * | H | $OCF_3$ | H |
| $CH_2CF_3$ | CycPr * | H | $OCF_3$ | H |
| $CH_2C{\equiv}CH$ | CycPr * | H | $OCF_3$ | H |
| CycPr | CycPr * | H | $OCF_3$ | H |
| $CF_2$CycPr | CycPr * | H | $OCF_3$ | H |

[Table 11]

| $R^1$ | $R^6$ | $R^8$ | $R^9$ | $R^{10}$ |
|---|---|---|---|---|
| Me | $CH_2$CycPr | H | $OCF_3$ | H |
| Et | $CH_2$CycPr | H | $OCF_3$ | H |
| Pr | $CH_2$CycPr | H | $OCF_3$ | H |
| $CH_2CF_3$ | $CH_2$CycPr | H | $OCF_3$ | H |
| $CH_2C{\equiv}CH$ | $CH_2$CycPr | H | $OCF_3$ | H |
| CycPr | $CH_2$CycPr | H | $OCF_3$ | H |
| $CH_2$CycPr | $CH_2$CycPr | H | $OCF_3$ | H |
| Me | $CH_2C{\equiv}CH$ | H | $OCF_3$ | H |
| Et | $CH_2C{\equiv}CH$ | H | $OCF_3$ | H |
| Pr | $CH_2C{\equiv}CH$ | H | $OCF_3$ | H |
| $CH_2CF_3$ | $CH_2C{\equiv}CH$ | H | $OCF_3$ | H |
| $CH_2C{\equiv}CH$ | $CH_2C{\equiv}CH$ | H | $OCF_3$ | H |
| CycPr | $CH_2C{\equiv}CH$ | H | $OCF_3$ | H |
| $CH_2$CycPr | $CH_2C{\equiv}CH$ | H | $OCF_3$ | H |
| Me | $CH_2OCH_3$ | H | $OCF_3$ | H |
| Et | $CH_2OCH_3$ | H | $OCF_3$ | H |
| Pr | $CH_2OCH_3$ | H | $OCF_3$ | H |
| $CH_2CF_3$ | $CH_3OCH_3$ | H | $OCF_3$ | H |

(continued)

| $R^1$ | $R^6$ | $R^8$ | $R^9$ | $R^{10}$ |
|---|---|---|---|---|
| $CH_2C\equiv CH$ | $CH_3OCH_3$ | H | $OCF_3$ | H |
| CycPr | $CH_2OCH_3$ | H | $OCF_3$ | H |
| $CH_2$CycPr | $CH_2OCH_3$ | H | $OCF_3$ | H |

[Table 12]

| $R^1$ | $R^6$ | $R^8$ | $R^9$ | $R^{10}$ |
|---|---|---|---|---|
| Me | $CO_2CH_3$ | H | $OCF_3$ | H |
| Et | $CO_2CH_3$ | H | $OCF_3$ | H |
| Pr | $CO_2CH_3$ | H | $OCF_3$ | H |
| $CH_2CF_3$ | $CO_2CH_3$ | H | $OCF_3$ | H |
| $CH_2C\equiv H$ | $CO_2CH_3$ | H | $OCF_3$ | H |
| CycPr | $CO_2CH_3$ | H | $OCF_3$ | H |
| $CH_2$CycPr | $CO_2CH_3$ | H | $OCF_3$ | H |
| Me | 6-chloro-pyridin-3-ylmethyl | H | $OCF_3$ | H |
| Et | 6-chloro-pyridin-3-ylmethyl | H | $OCF_3$ | H |
| Pr | 6-chloro-pyridin-3-ylmethyl | H | $OCF_3$ | H |
| $CH_2CF_3$ | 6-chloro-pyridin-3-ylmethyl | H | $OCF_3$ | H |
| $CH_2C\equiv CH$ | 6-chloro-pyridin-3-ylmethyl | H | $OCF_3$ | H |
| CycPr | 6-chloro-pyridin-3-ylmethyl | H | $OCF_3$ | H |
| $CH_2$CycPr | 6-chloro-pyridin-3-ylmethyl | H | $OCF_3$ | H |
| Me | 2-chloro-thiazol-5-ylmethyl | H | $OCF_3$ | H |
| Et | 2-chloro-thiazol-5-ylmethyl | H | $OCF_3$ | H |
| Pr | 2-chloro-thiazol-5-ylmethyl | H | $OCF_3$ | H |
| $CH_2CF_3$ | 2-chloro-thiazol-5-ylmethyl | H | $OCF_3$ | H |
| $CH_2C\equiv CH$ | 2-chloro-thiazol-5-ylmethyl | H | $OCF_3$ | H |
| CycPr | 2-chloro-thiazol-5-ylmethyl | H | $OCF_3$ | H |
| $CH_2$CycPr | 2-chloro-thiazol-5-ylmethyl | H | $OCF_3$ | H |

[Table 13]

| $R^1$ | $R^6$ | $R^8$ | $R^9$ | $R^{10}$ |
|---|---|---|---|---|
| Me | Me | H | $SCF_3$ | H |
| Et | Me | H | $SCF_3$ | H |
| Pr | Me | H | $SCF_3$ | H |
| $CH_2CF_3$ | Me | H | $SCF_3$ | H |
| $CH_2C\equiv H$ | Me | H | $SCF_3$ | H |
| CycPr | Me | H | $SCF_3$ | H |
| $CH_2$CycPr | Me | H | $SCF_3$ | H |

(continued)

| $R^1$ | $R^6$ | $R^8$ | $R^9$ | $R^{10}$ |
|---|---|---|---|---|
| Me | Et | H | $SCF_3$ | H |
| Et | Et | H | $SCF_3$ | H |
| Pr | Et | H | $SCF_3$ | H |
| $CH_2CF_3$ | Et | H | $SCF_3$ | H |
| $CH_2C\equiv CH$ | Et | H | $SCF_3$ | H |
| CycPr | Et | H | $SCF_3$ | H |
| $CH_2$CycPr | Et | H | $SCF_3$ | H |
| Me | CycPr * | H | $SCF_3$ | H |
| Et | CycPr * | H | $SCF_3$ | H |
| Pr | CycPr * | H | $SCF_3$ | H |
| $CH_2CF_3$ | CycPr * | H | $SCF_3$ | H |
| $CH_2C\equiv CH$ | CycPr * | H | $SCF_3$ | H |
| CycPr | CycPr * | H | $SCF_3$ | H |
| $CH_2$CycPr | CycPr * | H | $SCF_3$ | H |

[Table 14]

| $R^1$ | $R^6$ | $R^8$ | $R^9$ | $R^{10}$ |
|---|---|---|---|---|
| Me | $CH_2$CycPr | H | $SCF_3$ | H |
| Et | $CH_2$CycPr | H | $SCF_3$ | H |
| Pr | $CH_2$CycPr | H | $SCF_3$ | H |
| $CH_2CF_3$ | $CH_2$CycPr | H | $SCF_3$ | H |
| $CH_2C\equiv CH$ | $CH_2$CycPr | H | $SCF_3$ | H |
| CycPr | $CH_2$CycPr | H | $SCF_3$ | H |
| $CH_2$CycPr | $CH_2$CycPr | H | $SCF_3$ | H |
| Me | $CH_2C\equiv CH$ | H | $SCF_3$ | H |
| Et | $CH_2C\equiv CH$ | H | $SCF_3$ | H |
| Pr | $CH_2C\equiv CH$ | H | $SCF_3$ | H |
| $CH_2CF_3$ | $CH_2C\equiv CH$ | H | $SCF_3$ | H |
| $CH_2C\equiv CH$ | $CH_2C\equiv CH$ | H | $SCF_3$ | H |
| CycPr | $CH_2C\equiv CH$ | H | $SCF_3$ | H |
| $CH_2$CycPr | $CH_2C\equiv CH$ | H | $SCF_3$ | H |
| Me | $CH_2OCH_3$ | H | $SCF_3$ | H |
| Et | $CH_2OCH_3$ | H | $SCF_3$ | H |
| Pr | $CH_2OCH_3$ | H | $SCF_3$ | H |
| $CH_2CF_3$ | $CH_2OCH_3$ | H | $SCF_3$ | H |
| $CH_2C\equiv CH$ | $CH_2OCH_3$ | H | $SCF_3$ | H |
| CycPr | $CH_2OCH_3$ | H | $SCF_3$ | H |

(continued)

| $R^1$ | $R^6$ | $R^8$ | $R^9$ | $R^{10}$ |
|---|---|---|---|---|
| $CH_2$CycPr | $CH_2OCH_3$ | H | $SCF_3$ | H |

[Table 15]

| $R^1$ | $R^6$ | $R^8$ | $R^9$ | $R^{10}$ |
|---|---|---|---|---|
| Me | $CO_2CH_3$ | H | $SCF_3$ | H |
| Et | $CO_2CH_3$ | H | $SCF_3$ | H |
| Pr | $CO_2CH_3$ | H | $SCF_3$ | H |
| $CH_2CF_3$ | $CO_2CH_3$ | H | $SCF_3$ | H |
| $CH_2C{\equiv}CH$ | $CO_2CH_3$ | H | $SCF_3$ | H |
| CycPr | $CO_2CH_3$ | H | $SCF_3$ | H |
| $CH_2$CycPr | $CO_2CH_3$ | H | $SCF_3$ | H |
| Me | 6-chloro-pyridin-3-ylmethyl | H | $SCF_3$ | H |
| Et | 6-chloro-pyridin-3-ylmethyl | H | $SCF_3$ | H |
| Pr | 6-chloro-pyridin-3-ylmethyl | H | $SCF_3$ | H |
| $CH_2CF_3$ | 6-chloro-pyridin-3-ylmethyl | H | $SCF_3$ | H |
| $CH_2C{\equiv}CH$ | 6-chloro-pyridin-3-ylmethyl | H | $SCF_3$ | H |
| CycPr | 6-chloro-pyridin-3-ylmethyl | H | $SCF_3$ | H |
| $CH_2$CycPr | 6-chloro-pyridin-3-ylmethyl | H | $SCF_3$ | H |
| Me | 2-chloro-thiazol-5-ylmethyl | H | $SCF_3$ | H |
| Et | 2-chloro-thiazol-5-ylmethyl | H | $SCF_3$ | H |
| Pr | 2-chloro-thiazol-5-ylmethyl | H | $SCF_3$ | H |
| $CH_2CF_3$ | 2-chloro-thiazol-5-ylmethyl | H | $SCF_3$ | H |
| $CH_2C{\equiv}CH$ | 2-chloro-thiazol-5-ylmethyl | H | $SCF_3$ | H |
| CycPr | 2-chloro-thiazol-5-ylmethyl | H | $SCF_3$ | H |
| $CH_2$CycPr | 2-chloro-thiazol-5-ylmethyl | H | $SCF_3$ | H |

[Table 16]

| $R^1$ | $R^6$ | $R^8$ | $R^9$ | $R^{10}$ |
|---|---|---|---|---|
| Me | Me | H | $S(O)CF_3$ | H |
| Et | Me | H | $S(O)CF_3$ | H |
| Pr | Me | H | $S(O)CF_3$ | H |
| $CH_2CF_3$ | Me | H | $S(O)CF_3$ | H |
| $CH_2C{\equiv}CH$ | Me | H | $S(O)CF_3$ | H |
| Cyc Pr | Me | H | $S(O)CF_3$ | H |
| $CH_2$CycPr | Me | H | $S(O)CF_3$ | H |
| Me | Et | H | $S(O)CF_3$ | H |
| Et | Et | H | $S(O)CF_3$ | H |

(continued)

| $R^1$ | $R^6$ | $R^8$ | $R^9$ | $R^{10}$ |
|---|---|---|---|---|
| Pr | Et | H | $S(O)CF_3$ | H |
| $CH_2CF_3$ | Et | H | $S(O)CF_3$ | H |
| $CH_2C\equiv CH$ | Et | H | $S(O)CF_3$ | H |
| CycPr | Et | H | $S(O)CF_3$ | H |
| $CH_2$CycPr | Et | H | $S(O)CF_3$ | H |
| Me | CycPr * | H | $S(O)CF_2$ | H |
| Et | CycPr * | H | $S(O)CF_3$ | H |
| Pr | CycPr * | H | $S(O)CF_3$ | H |
| $CH_2CF_3$ | CycPr * | H | $S(O)CF_3$ | H |
| $CH_2C\equiv CH$ | CycPr * | H | $S(O)CF_3$ | H |
| CycPr | CycPr * | H | $S(O)CF_3$ | H |
| $CH_2$CycPr | CycPr * | H | $S(O)CF_3$ | H |

[Table 17]

| $R^1$ | $R^6$ | $R^8$ | $R^9$ | $R^{10}$ |
|---|---|---|---|---|
| Me | $CH_2$CycPr | H | $S(O)CF_3$ | H |
| Et | $CH_2$CycPr | H | $S(O)CF_3$ | H |
| Pr | $CH_2$CycPr | H | $S(O)CF_3$ | H |
| $CH_2CF_3$ | $CH_2$CycPr | H | $S(O)CF_3$ | H |
| $CH_2C\equiv CH$ | $CH_2$CycPr | H | $S(O)CF_3$ | H |
| CycPr | $CH_2$CycPr | H | $S(O)CF_3$ | H |
| $CH_2$CycPr | $CH_2$CycPr | H | $S(O)CF_3$ | H |
| Me | $CH_2C\equiv CH$ | H | $S(O)CF_3$ | H |
| Et | $CH_2C\equiv CH$ | H | $S(O)CF_3$ | H |
| Pr | $CH_2C\equiv CH$ | H | $S(O)CF_3$ | H |
| $CH_2CF_3$ | $CH_2C\equiv CH$ | H | $S(O)CF_3$ | H |
| $CH_2C\equiv CH$ | $CH_2C\equiv CH$ | H | $S(O)CF_3$ | H |
| CycPr | $CH_2C\equiv CH$ | H | $S(O)CF_3$ | H |
| $CH_2$CycPr | $CH_2C\equiv CH$ | H | $S(O)CF_3$ | H |
| Me | $CH_2OCH_3$ | H | $S(O)CF_3$ | H |
| Et | $CH_2OCH_3$ | H | $S(O)CF_3$ | H |
| Pr | $CH_2OCH_3$ | H | $S(O)CF_3$ | H |
| $CH_2CF_3$ | $CH_2OCH_3$ | H | $S(O)CF_3$ | H |
| $CH_2C\equiv CH$ | $CH_2OCH_3$ | H | $S(O)CF_3$ | H |
| CycPr | $CH_2OCH_3$ | H | $S(O)CF_3$ | H |
| $CH_2$CycPr | $CH_2OCH_3$ | H | $S(O)CF_3$ | H |

[Table 18]

| $R^1$ | $R^6$ | $R^8$ | $R^9$ | $R^{10}$ |
|---|---|---|---|---|
| Me | $CO_2CH_3$ | H | $S(O)CF_3$ | H |
| Et | $CO_2CH_3$ | H | $S(O)CF_3$ | H |
| Pr | $CO_2CH_3$ | H | $S(O)CF_3$ | H |
| $CH_2CF_3$ | $CO_2CH_3$ | H | $S(O)CF_3$ | H |
| $CH_2C\equiv CH$ | $CO_2CH_3$ | H | $S(O)CF_3$ | H |
| CycPr | $CO_2CH_3$ | H | $S(O)CF_3$ | H |
| $CH_2$CycPr | $CO_2CH_3$ | H | $S(O)CF_3$ | H |
| Me | 6-chloro-pyridin-3-ylmethyl | H | $S(O)CF_3$ | H |
| Et | 6-chloro-pyridin-3-ylmethyl | H | $S(O)CF_3$ | H |
| Pr | 6-chloro-pyridin-3-ylmethyl | H | $S(O)CF_3$ | H |
| $CH_2CF_3$ | 6-chloro-pyridin-3-ylmethyl | H | $S(O)CF_3$ | H |
| $CH_2C\equiv CH$ | 6-chloro-pyridin-3-ylmethyl | H | $S(O)CF_3$ | H |
| CycPr | 6-chloro-pyridin-3-ylmethyl | H | $S(O)CF_3$ | H |
| $CH_2$CycPr | 6-chloro-pyridin-3-ylmethyl | H | $S(O)CF_3$ | H |
| Me | 2-chloro-thiazol-5-ylmethyl | H | $S(O)CF_3$ | H |
| Et | 2-chloro-thiazol-5-ylmethyl | H | $S(O)CF_3$ | H |
| Pr | 2-chloro-thiazol-5-ylmethyl | H | $S(O)CF_3$ | H |
| $CH_2CF_3$ | 2-chloro-thiazol-5-ylmethyl | H | $S(O)CF_3$ | H |
| $CH_2C\equiv H$ | 2-chloro-thiazol-5-ylmethyl | H | $S(O)CF_3$ | H |
| CycPr | 2-chloro-thiazol-5-ylmethyl | H | $S(O)CF_3$ | H |
| $CH_2$CycPr | 2-chloro-thiazol-5-ylmethyl | H | $S(O)CF_3$ | H |

[Table 19]

| $R^1$ | $R^6$ | $R^8$ | $R^9$ | $R^{10}$ |
|---|---|---|---|---|
| Me | Me | H | $S(O)_2CF_3$ | H |
| Et | Me | H | $S(O)_2CF_3$ | H |
| Pr | Me | H | $S(O)_2CF_3$ | H |
| $CH_2CF_3$ | Me | H | $S(O)_2CF_3$ | H |
| $CH_2C\equiv CH$ | Me | H | $S(O)_2CF_3$ | H |
| CycPr | Me | H | $S(O)_2CF_3$ | H |
| $CH_2$CycPr | Me | H | $S(O)_2CF_3$ | H |
| Me | Et | H | $S(O)_2CF_3$ | H |
| Et | Et | H | $S(O)_2CF_3$ | H |
| Pr | Et | H | $S(O)_2CF_3$ | H |
| $CH_2CF_3$ | Et | H | $S (O)_2CF_3$ | H |
| $CH_2C\equiv CH$ | Et | H | $S(O)_2CF_3$ | H |
| CycPr | Et | H | $S(O)_2CF_3$ | H |

(continued)

| $R^1$ | $R^6$ | $R^8$ | $R^9$ | $R^{10}$ |
|---|---|---|---|---|
| $CH_2$CycPr | Et | H | $S(O)_2CF_3$ | H |
| Me | CycPr * | H | $S(C)\ _2CF_3$ | H |
| Et | CycPr * | H | $S(O)_2CF_3$ | H |
| Pr | CycPr * | H | $S(O)_2CF_3$ | H |
| $CH_2CF_3$ | CycPr * | H | $S(O)_2CF_3$ | H |
| $CH_2C{\equiv}CH$ | CycPr * | H | $S(O)_2CF_3$ | H |
| CycPr | CycPr * | H | $S(O)_2CF_3$ | H |
| $CH_2$CyrPr | CycPr * | H | $S(O)_2CD_3$ | H |

[Table 20]

| $R^1$ | $R^6$ | $R^8$ | $R^9$ | $R^{10}$ |
|---|---|---|---|---|
| Me | $CH_2$CycPr | H | $S(O)_2CF_3$ | H |
| Et | $CH_2$CycPr | H | $S(O)_2CF_3$ | H |
| Pr | $CH_2$CycPr | H | $S\ (O)_2CF_3$ | H |
| $CH_2CF_3$ | $CH_2$CycPr | H | $S(O)_2CF_3$ | H |
| $CH_2C{\equiv}CH$ | $CH_2$CycPr | H | $S(O)_2CF_3$ | H |
| CycPr | $CH_2$CycPr | H | $S(O)_2CF_3$ | H |
| $CH_2$CycPr | $CH_2$CycPr | H | $S(O)_2CF_3$ | H |
| Me | $CH_2C{\equiv}CH$ | H | $S(O)_2CF_3$ | H |
| Et | $CH_2C{\equiv}CH$ | H | $S\ (O)_2CF_3$ | H |
| Pr | $CH_2C{\equiv}CH$ | H | $S(O)_2CF_3$ | H |
| $CH_2CF_3$ | $CH_2C{\equiv}CH$ | H | $S(O)_2CF_3$ | H |
| $CH_2C{\equiv}CH$ | $CH_2C{\equiv}CH$ | H | $S(O)_2CF_3$ | H |
| CycPr | $CH_3C{\equiv}CH$ | H | $S(O)_2CF_3$ | H |
| $CH_2$CycPr | $CH_2C{\equiv}CH$ | H | $S(O)_2CF_3$ | H |
| Me | $CH_2OCH_3$ | H | $S(O)_2CF_3$ | H |
| Et | $CH_2OCH_3$ | H | $S(O)_2CF_3$ | H |
| Pr | $CH_2OCH_3$ | H | $S(O)_2CF_3$ | H |
| $CH_2CF_3$ | $CH_2OCH_3$ | H | $S(O)_2CF_3$ | H |
| $CH_2C{\equiv}CH$ | $CH_2OCH_3$ | H | $S(O)_2CF_3$ | H |
| CycPr | $CH_2OCH_3$ | H | $S(O)_2CF_3$ | H |
| $CH_2$CycPr | $CH_2OCH_3$ | H | $S(O)_2CF_3$ | H |

[Table 21]

| $R^1$ | $R^6$ | $R^8$ | $R^9$ | $R^{10}$ |
|---|---|---|---|---|
| Me | $CO_2CH_3$ | H | $S\ (O)_2\ CF_3$ | H |
| Et | $CO_2CH_3$ | H | $S(O)_2CF_3$ | H |

(continued)

| $R^1$ | $R^6$ | $R^8$ | $R^9$ | $R^{10}$ |
|---|---|---|---|---|
| Pr | $CO_2CH_3$ | H | $S(O)_2CF_3$ | H |
| $CH_2CF_3$ | $CO_2CH_3$ | H | $S(O)_2CF_3$ | H |
| $CH_2C{\equiv}CH$ | $CO_2CH_3$ | H | $S(O)_2CF_3$ | H |
| CycPr | $CO_2CH_3$ | H | $S(O)_2CF_3$ | H |
| $CH_2$CycPr | $CO_2CH_3$ | H | $S(O)_2CF_3$ | H |
| Me | 6-chloro-pyridin-3-ylmethyl | H | $S(O)_2CF_3$ | H |
| Et | 6-chloro-pyridin-3-ylmethyl | H | $S(O)\ _2CF_3$ | H |
| Pr | 6-chloro-pyridin-3-ylmethyl | H | $S(O)\ _2CF_3$ | H |
| $CH_2CF_3$ | 6-chloro-pyridin-3-ylmethyl | H | $S(O)_2CF_3$ | H |
| $CH_2C{\equiv}CH$ | 6-chloro-pyridin-3-ylmethyl | H | $S(O)_2CF_3$ | H |
| CycPr | 6-chloro-pyridin-3-ylmethyl | H | $S(O)_2CF_3$ | H |
| $CH_2$CycPr | 6-chloro-pyridin-3-ylmethyl | H | $S(O)_2CF_2$ | H |
| Me | 2-chloro-thiazol-5-ylmethyl | H | $S(O)_2CF_3$ | H |
| Et | 2-chloro-thiazol-5-ylmethyl | H | $S(O)_2CF_3$ | H |
| Pr | 2-chloro-thiazol-5-ylmethyl | H | $S(O)_2CF_3$ | H |
| $CH_2CF_3$ | 2-chloro-thiazol-5-ylmethyl | H | $S(O)_2CF_3$ | H |
| $CH_2C{\equiv}CH$ | 2-chloro-thiazol-5-ylmethyl | H | $S\ (O)_2CF_3$ | H |
| CycPr | 2-chloro-thiazol-5-ylmethyl | H | $S(O)_2CF_3$ | H |
| $CH_2$CycPr | 2$^-$chloro-thiazol-5-ylmethyl | H | $S\ (O)_2CF_3$ | H |

[Table 22]

| $R^1$ | $R^6$ | $R^8$ | $R^9$ | $R^{10}$ |
|---|---|---|---|---|
| Me | Me | $CF_3$ | H | $CF_3$ |
| Et | Me | $CF_3$ | H | $CF_3$ |
| Pr | Me | $CF_3$ | H | $CF_3$ |
| $CH_2CF_3$ | Me | $CF_3$ | H | $CF_3$ |
| $CH_2C{\equiv}CH$ | Me | $CF_3$ | H | $CF_3$ |
| CycPr | Me | $CF_3$ | H | $CF_3$ |
| $CH_2$CycPr | Me | $CF_3$ | H | $CF_3$ |
| Me | Et | $CF_3$ | H | $CF_3$ |
| Et | Et | $CF_3$ | H | $CF_3$ |
| Fr | Et | $CF_3$ | H | $CF_3$ |
| $CH_2CF_3$ | Et |  | H | $CF_3$ |
| $CF_2C{\equiv}CH$ | Et | $CF_3$ | H | $CF_3$ |
| CycPr | Et | $CF_3$ | H | $CF_3$ |
| $CH_2$CycPr | Et | $CF_3$ | H | $CF_3$ |
| Me | CycPr * | $CF_3$ | H | $CF_3$ |

(continued)

| $R^1$ | $R^6$ | $R^8$ | $R^9$ | $R^{10}$ |
|---|---|---|---|---|
| Et | CycPr * | $CF_3$ | H | $CF_3$ |
| Pr | CycPr * | $CF_3$ | H | $CF_3$ |
| $CH_2CF_3$ | CycPr * | $CF_3$ | H | $CF_3$ |
| $CH_2C{\equiv}CH$ | CycPr * | $CF_3$ | H | $CF_3$ |
| CycPr | CycPr * | $CF_3$ | H | $CF_3$ |
| $CH_2$CycPr | CycPr * | $CF_3$ | H | $CF_3$ |

[Table 23]

| $R^1$ | $R^6$ | $R^8$ | $R^9$ | $R^{10}$ |
|---|---|---|---|---|
| Me | $CH_2$CycPr | $CF_3$ | H | $CF_3$ |
| Et | $CH_2$CycPr | $CF_3$ | H | $CF_3$ |
| Pr | $CH_2$CycPr | $CF_3$ | H | $CF_3$ |
| $CH_2CF_3$ | $CH_2$CycPr | $CF_3$ | H | $CF_3$ |
| $CH_2C{\equiv}CH$ | $CH_2$CycPr | $CF_3$ | H | $CF_3$ |
| CycPr | $CH_2$CycPr | $CF_3$ | H | $CF_3$ |
| $CH_2$CycPr | $CH_2$CycPr | $CF_3$ | H | $CF_3$ |
| Me | $CH_2C{\equiv}CH$ | $CF_3$ |  | $CF_3$ |
| Et | $CH_2C{\equiv}CH$ | $CF_3$ | H | $CF_3$ |
| Pr | $CH_2C{\equiv}CH$ | $CF_3$ | H | $CF_3$ |
| $CH_2CF_3$ | $CH_2C{\equiv}CH$ | $CF_3$ | H | $CF_3$ |
| $CH_2C{\equiv}CH$ | $CH_2C{\equiv}CH$ | $CF_3$ | H | $CF_3$ |
| CycPr | $CH_2C{\equiv}CH$ | $CF_3$ | H | $CF_3$ |
| $CH_2$CycPr | $CH_2C{\equiv}CH$ | $CF_3$ | H | $CF_3$ |
| Me | $CH_2OCH_3$ | $CF_3$ | H | $CF_3$ |
| Et | $CH_2OCH_3$ | $CF_3$ | H | $CF_3$ |
| Pr | $CH_2OCH_3$ | $CF_3$ | H | $CF_3$ |
| $CH_2CF_3$ | $CH_2OCH_3$ | $CF_3$ | H | $CF_3$ |
| $CH_2C{\equiv}CH$ | $CH_2OCH_3$ | $CF_3$ | H | $CF_3$ |
| CycPr | $CH_2OCH_3$ | $CF_3$ | H | $CF_3$ |
| $CH_2$CycPr | $CH_2OCH_3$ | $CF_3$ | H | $CF_3$ |

[Table 24]

| $R^1$ | $R^6$ | $R^8$ | $R^9$ | $R^{10}$ |
|---|---|---|---|---|
| Me | $CO_2CH_3$ | $CF_3$ | H | $CF_3$ |
| Et | $CO_2CH_3$ | $CF_3$ | H | $CF_3$ |
| Pr | $CO_2CH_3$ | $CF_3$ | H | $CF_3$ |
| $CH_2CF_3$ | $CO_2CH_3$ | $CF_3$ | H | $CF_3$ |

(continued)

| $R^1$ | $R^6$ | $R^8$ | $R^9$ | $R^{10}$ |
|---|---|---|---|---|
| $CH_2C{\equiv}CH$ | $CO_2CH_3$ | $CF_3$ | H | $CF_3$ |
| CycPr | $CO_2CH_3$ | $CF_3$ | H | $CF_3$ |
| $CH_2$CycPr | $CO_2CH_3$ | $CF_3$ | H | $CF_3$ |
| Me | 6-chloro-pyridin-3-ylmethyl | $CF_3$ | H | $CF_3$ |
| Et | 6-chloro-pyridin-3-ylmethyl | $CF_3$ | H | $CF_3$ |
| Pr | 6-chloro-pyridin-3-ylmethyl | $CF_3$ | H | $CF_3$ |
| $CH_2CF_3$ | 6-chloro-pyridin-3-ylmethyl | $CF_3$ | H | $CF_3$ |
| $CH_2C{\equiv}CH$ | 6-chloro-pyridin-3-ylmethyl | $CF_3$ | H | $CF_3$ |
| CycPr | 6-chloro-pyridin-3-ylmethyl | $CF_3$ | H | $CF_3$ |
| $CH_2$CycPr | 6-chloro-pyridin-3-ylmethyl | $CF_3$ | H | $CF_3$ |
| Me | 2-chloro-thiazol-5-ylmethyl | $CF_3$ | H | $CF_3$ |
| Et | 2-chloro-thiazol-5-ylmethyl | $CF_3$ | H | $CF_3$ |
| Pr | 2-chloro-thiazol-5-ylmethyl | $CF_3$ | H | $CF_3$ |
| $CH_2CF_3$ | 2-chloro-thiazol-5-ylmethyl | $CF_3$ | H | $CF_3$ |
| $CH_2C{\equiv}CH$ | 2-chloro-thiazol-5-ylmethyl | $CF_3$ | H | $CF_3$ |
| CycPr | 2-chloro-thiazol-5-ylmethyl | $CF_3$ | H | $CF_3$ |
| $CH_2$CycPr | 2-chloro-thiazol-5-ylmethyl | $CF_3$ | H | $CF_3$ |

(In [Table 1] to [Table 24] above, Me represents a methyl group, Et represents an ethyl group, Pr represents a n-propyl group, and CycPr represents a cyclopropyl group.)

In the formula (1), compounds of the present invention wherein n is 1, $R^2$, $R^3$, $R^4$, $R^5$, $R^7$ and $R^{11}$ are a hydrogen atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^3$, $R^4$, $R^5$, $R^7$ and $R^{11}$ are a hydrogen atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^3$, $R^4$, $R^5$, $R^7$ and $R^{11}$ are a hydrogen atom, Q is a sulfur atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^3$, $R^4$, $R^5$, $R^7$ and $R^{11}$ are a hydrogen atom, Q is a sulfur atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^3$, $R^4$, $R^5$, $R^7$ and $R^{11}$ are a hydrogen atom, Q is a sulfur atom, and $R^1$, $R^6$, $R^8$, $R^9$ and $R^{10}$ are the combinations shown in [Table 1] to [Table 24].

In the formula (1), compounds of the present invention wherein n is 0, $R^1$, $R^4$, $R^{11}$, $R^7$ and $R^{11}$ are a hydrogen atom, $R^3$ is a fluorine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a fluorine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a fluorine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is a chlorine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a chlorine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a chlorine atom, Q is an oxygen atom, and $R^1$, $R^6$, $R^8$, $R^9$ and $R^{10}$ are the combinations shown in [Table 1]

to [Table 24].

In the formula (1), compounds of the present invention wherein n is 0, $R^2$, $R^4$, $R^5$, $R^7$ and $R^{11}$ are a hydrogen atom, $R^3$ is a bromine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a bromine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a bromine atom, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is a methyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a methyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^3$, $R^7$ and $R^{11}$ are a hydrogen atom, $R^3$ is a methyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is a trifluoromethyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a trifluoromethyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 2, $R^2$, $R^4$, $R^5$, $R^7$ and $R^{11}$ are a hydrogen atom, $R^3$ is a trifluoromethyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 0, $R^2$, $R^4$, $R^5$, $R^7$ and $R^{11}$ are a hydrogen atom, $R^3$ is a trifluoromethoxy group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is a trifluoromethoxy group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 2, $R^2$, $R^4$, $R^5$, $R^7$ and $R^{11}$ are a hydrogen atom, $R^3$ is a trifluoromethoxy group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 0, $R^2$, $R^4$, $R^5$, $R^7$ and $R^{11}$ are a hydrogen atom, $R^3$ is a 2-pyridyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a 2-pyridyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a 2-pyridyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 0, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is a 2-pyrimidinyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is a 2-pyrimidinyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a 2-pyrimidinyl group, Q is an oxygen atom, and $R^1$, $R^6$, $R^6$, $R^9$ and $R^{10}$ are the combinations shown in [Table 1] to [Table 24] .

In the formula (1), compounds of the present invention wherein n is 0, $R^2$, $R^4$, $R^5$, $R^7$ and $R^{11}$ are a hydrogen atom, $R^3$ is a methoxycarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is a methoxycarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 2, $R^2$, $R^4$, $R^5$, $R^7$ and $R^{11}$ are a hydrogen atom, $R^3$ is a methoxycarbonyl group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is an ethoxycarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is an ethoxycarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 2, $R^2$, $R^4$, $R^5$, $R^7$ and $R^{11}$ are a hydrogen atom, $R^3$ is an ethoxycarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 0, $R^2$, $R^4$, $R^5$, $R^7$ and $R^{11}$ are a hydrogen atom, $R^3$ is an aminocarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is an aminocarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 2, $R^2$, $R^4$, $R^5$, $R^7$ and $R^{11}$ are a hydrogen atom, $R^3$ is an aminocarbonyl group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 0, $R^2$, $R^4$, $R^8$, $R^7$ and $R^{11}$ are a hydrogen atom, In the formula (1), n is 0, $R^2$, $R^4$, $R^5$, $R^7$ and $R^{11}$ are a hydrogen atom, $R^3$ is an amino group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is an amino group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is an amino group, Q is an oxygen atom, and $R^1$, $R^6$, $R^8$, $R^9$ and $R^{10}$ are the combinations shown in [Table 1] to [Table 24]. Compounds of the present invention wherein $R^3$ is a methoxycarbonylamino group, Q is an oxygen atom, and $R^1$, $R^6$, $R^8$, $R^9$ and $R^{10}$ are the combinations shown in [Table 1] to [Table 24] .

In the formula (1), compounds of the present invention wherein n is 1, $R^2$, $R^4$, $R^5$, $R^7$ and $R^{11}$ are a hydrogen atom, $R^3$ is a methoxycarbonylamino group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a methoxycarbonylamino group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 0, $R^2$, $R^4$, $R^5$, $R^7$ and $R^{11}$ are a hydrogen atom, $R^3$ is a tert-butoxycarbonylamino group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 1, $R^2$, $R^4$, $R^5$, $R^7$ and $R^{11}$ are a hydrogen atom, $R^3$ is a tert-butoxycarbonylamino group, Q is an oxygen atom, and $R^1$, $R^6$, $R^8$, $R^9$ and $R^{10}$ are the combinations shown in [Table 1] to [Table 24].

In the formula (1), compounds of the present invention wherein n is 2, $R^2$, $R^4$, $R^5$, $R^7$ and $R^{11}$ are a hydrogen atom, $R^3$ is a tert-butoxycarbonylamino group, Q is an oxygen atom, and $R^1$, $R^6$, $R^8$, $R^9$ and $R^{10}$ are the combinations shown in [Table 1] to [Table 24].

Next, specific examples of the intermediate compound (M6) are shown below.

In the formula (M6), n is 0, $R^2$, $R^3$, $R^4$, $R^5$, $R^7$ and $R^{11}$ are a hydrogen atom, Q is an oxygen atom, and $R^1$, $R^8$, $R^9$ and $R^{10}$ are the combinations shown in [Table 25] to [Table 27].

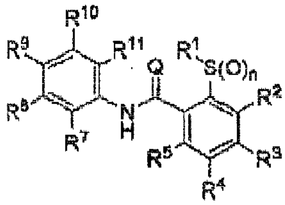

(M 6)

[Table 25]

| $R^1$ | $R^8$ | $R^9$ | $R^{10}$ |
|---|---|---|---|
| Me | H | $CF_3$ | H |
| Et | H | $CF_3$ | H |
| Pr | H | $CF_3$ | H |
| $CH_2CF_3$ | H | $CF_3$ | H |
| $CH_2C{\equiv}CH$ | H | $CF_3$ | H |
| CycPr | H | $CF_3$ | H |
| $CH_2$CycPr | H | $CF_3$ | H |
| Me | H | $CF_2CF_3$ | H |
| Et | H | $CF_2CF_3$ | H |
| Pr | H | $CF_2CF_3$ | H |
| $CH_2CF_3$ | H | $CF_2CF_3$ | H |
| $CH_2C{\equiv}CH$ | H | $CF_2CF_3$ | H |
| CycPr | H | $CF_2CF_3$ | H |
| $CH_2$CycPr | H | $CF_2CF_3$ | H |
| Me | H | $CF(CF_3)_2$ | H |
| Et | H | $CF(CF_3)_2$ | H |
| Pr | H | $CF(CF_3)_2$ | H |
| $CH_2CF_3$ | H | $CF(CF_3)_2$ | H |
| $CH_2C{\equiv}CH$ | H | $CF(CF_3)_2$ | H |
| CycPr | H | $CF(CF_3)_2$ | H |
| $CH_2$CycPr | H | $CF(CF_3)_2$ | H |

[Table 26]

| $R^1$ | $R^8$ | $R^9$ | $R^{10}$ |
|---|---|---|---|
| Me | H | $OCF_3$ | H |
| Et | H | $OCF_3$ | H |
| Pr | H | $OCF_3$ | H |
| $CH_2CF_3$ | H | $OCF_3$ | H |
| $CH_2C{\equiv}CH$ | H | $OCF_3$ | H |

(continued)

| $R^1$ | $R^8$ | $R^9$ | $R^{10}$ |
|---|---|---|---|
| CycPr | H | $OCF_3$ | H |
| $CH_2$CycPr | H | $OCF_3$ | H |
| Me | H | $SCF_3$ | H |
| Et | H | $SCF_3$ | H |
| Pr | H | $SCF_3$ | H |
| $CH_2CF_3$ | H | $SCF_3$ | H |
| $CH_2C{\equiv}CH$ | H | $SCF_3$ | H |
| CycPr | H | $SCF_3$ | H |
| $CH_2$CycPr | H | $SCF_3$ | H |
| Me | H | $S(O)CF_3$ | H |
| Et | H | $S(O)CF_3$ | H |
| Pr | H | $S(O)CF_3$ | H |
| $CH_2CF_3$ | H | $S(O)CF_3$ | H |
| $CH_2C{\equiv}CH$ | H | $S(O)CF_3$ | H |
| CycPr | H | $S(O)CF_3$ | H |
| $CH_2$CycPr | H | $S(O)CF_3$ | H |

[Table 27]

| $R^1$ | $R^8$ | $R^9$ | $R^{10}$ |
|---|---|---|---|
| Me | H | $S(O)_2CF_3$ | H |
| Et | H | $S(O)_2CF_3$ | H |
| Pr | H | $S(O)_2CF_3$ | H |
| $CH_2CF_3$ | H | $S(O)_2CF_3$ | H |
| $CH_2C{\equiv}CH$ | H | $S(O)_2CF_3$ | H |
| CycPr | H | $S(O)_2CF_3$ | H |
| $CH_2$CycPr | H | $S(O)_2CF_3$ | H |
| Me | $CF_3$ | H | $CF_3$ |
| Et | $CF_3$ | H | $CF_3$ |
| Pr | $CF_3$ | H | $CF_3$ |
| $CH_2CF_3$ | $CF_3$ | H | $CF_3$ |
| $CH_2C{\equiv}CH$ | $CF_3$ | H | $CF_3$ |
| CycPr | $CF_3$ | H | $CF_3$ |
| $CH_2$CyrPr | $CF_3$ | H | $CF_3$ |

(In [Table 25] to [Table 27] above, Me represents a methyl group, Et represents an ethyl group, Pr represents a n-propyl group, and CyoPr represents a cyclopropyl group.)

In the formula (M6), compounds of the present invention wherein n is 1, $R^2$, $R^3$, $R^4$, $R^5$, $R^7$ and $R^{11}$ are a hydrogen atom, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^3$, $R^4$, $R^5$, $R^7$ and $R^{11}$ are a

hydrogen atom, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^3$, $R^4$, $R^5$, $R^7$ and $R^{11}$ are a hydrogen atom, Q is a sulfur atom, and $R^1$, $R^8$, $R^9$ and $R^{10}$ are the combinations shown in [Table 25] to [Table 27].

In the formula (M6), compounds of the present invention wherein n is 1, $R^2$, $R^3$, $R^4$, $R^5$, $R^7$ and $R^{11}$ are a hydrogen atom, Q is a sulfur atom, and $R^1$, $R^8$, $R^9$ and $R^{10}$ are the combinations shown in [Table 25] to [Table 27].

In the formula (M6), compounds of the present invention wherein n is 2, $R^2$, $R^3$, $R^4$, $R^5$, $R^7$ and $R^{11}$ are a hydrogen atom, Q is a sulfur atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a fluorine atom, Q is an oxygen atom, and $R^1$, $R^2$, $R^9$ and $R^{10}$ are the combinations shown in [Table 25] to [Table 27].

In the formula (M6), compounds of the present invention wherein n ia 1, $R^2$, $R^4$, $R^5$, $R^7$ and $R^{11}$ are a hydrogen atom, $R^3$ is a fluorine atom, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a fluorine atom, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ ie a chlorine atom, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a chlorine atom, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a chlorine atom, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a bromine atom, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a bromine atom, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a bromine atom, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^3$, $R^7$ and $R^{11}$ are a hydrogen atom, $R^3$ is a methyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a methyl group, Q is an oxygen atom, and $R^1$, $R^6$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a methyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a trifluoromethyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a trifluoromethyl group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a trifluoromethyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a trifluoromethoxy group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a trifluoromethoxy group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a trifluoromethoxy group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a 2-pyridyl group, Q is an oxygen atom, and $R^1$, $R^8$, $R^9$ and $R^{10}$ are the combinations shown in [Table 25 to [Table 27].

In the formula (M6), compounds of the present invention wherein n is 1, $R^2$, $R^4$, $R^5$, $R^7$ and $R^{11}$ are a hydrogen atom, $R^3$ is a 2-pyridyl group, Q is an oxygen atom, and $R^1$, $R^8$, $R^9$ and $R^{10}$ are the combinations shown in [Table 25 to [Table 27].

In the formula (M6), compounds of the present invention wherein n is 2, $R^2$, $R^4$, $R^5$, $R^7$ and $R^{11}$ are a hydrogen atom, $R^3$ is a 2-pyridyl group, Q is an oxygen atom, and $R^1$, $R^8$, $R^9$ and $R^{10}$ are the combinations shown in [Table 25 to [Table 27].

In the formula (M6), compounds of the present invention wherein n is 0, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^8$, $R^9$ and $R^{10}$ are the combinations shown in [Table 25] to [Table 27] .

In the formula (M6), compounds of the present invention wherein n is 1, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a 5-trifluoromethyl-2-pyridyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is a 2-pyrimidinyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a 2-pyrimidinyl group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a 2-pyrimidinyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^8$, $R^5$, $R^7$ and $R^{11}$ are a hydrogen atom, $R^3$ is a methoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a methoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n ia 2, $R^2$, $R^4$, $R^5$, $R^7$ and $R^{11}$ are a hydrogen atom, $R^3$ is a methoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is an ethoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is an ethoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is an ethoxycarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen atom, $R^3$ is an aminocarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is an aminocarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is an aminocarbonyl group, Q is an oxygen atom, and $R^1$, $R^8$, $R^9$ and $R^{10}$ are the combinations shown in [Table 25] to [Table 27].

In the formula (M6), compounds of the present invention wherein n is 0, $R^2$, $R^4$, $R^5$, $R^7$ and $R^{11}$ are a hydrogen

atom, $R^3$ is a methoxycarbonylamino group, Q is an oxygen atom, and $R^1$, $R^8$, $R^9$ and $R^{10}$ are the combinations shown in [Table 25] to [Table 27] .

In the formula (M6), compounds of the present invention wherein n is 1, $R^2$, $R^4$, $R^5$, $R^7$ and $R^{11}$ are a hydrogen atom, $R^3$ is a methoxycarbonylamino group, Q is an oxygen atom, and $R^1$, $R^8$, $R^9$ and $R^{10}$ are the combinations shown in [Table 25] to [Table 27].

In the formula (M6), compounds of the present invention wherein n is 2, $R^2$, $R^4$, $R^5$, $R^7$ and $R^{11}$ are a hydrogen atom, $R^3$ is a methoxycarbonylamino group, Q is an oxygen atom, and $R^1$, $R^8$, $R^9$ and $R^{10}$ are the combinations shown in [Table 25] to [Table 27] .

In the formula (M6), compounds of the present invention wherein n is 0, $R^2$, $R^4$, $R^5$, $R^7$ and $R^{11}$ are a hydrogen atom, $R^3$ is a tert-butoxycarbonylamino group, Q is an oxygen atom, and $R^1$, $R^8$, $R^9$ and $R^{10}$ are the combinations shown in [Table 25] to [Table 27].

In the formula (M6), compounds of the present invention wherein n is 1, $R^2$, $R^4$, $R^5$, $R^7$ and $R^{11}$ are a hydrogen atom, $R^3$ is a tert-butoxycarbonylamino group, Q is an oxygen atom, and $R^1$, $R^8$, $R^9$ and $R^{10}$ are the combinations shown in [Table 25] to [Table 27] .

In the formula (M6), compounds of the present invention wherein n is 2, $R^2$, $R^4$, $R^5$, $R^7$ and $R^{11}$ are a hydrogen atom, $R^3$ is a tert-butoxycarbonylamino group, Q is an oxygen atom, and $R^1$, $R^8$, $R^9$ and $R^{10}$ are the combinations shown in [Table 25] to [Table 27] .

Examples of the pest on which the composition of the present invention has an effect include arthropod pests such as pest insects and pest mites and nematoda. Specifically, examples of the pests include those shown below.

Hemiptera: Delphacidae such as Laodelphax striatellus, Nilaparvata lugens, and Sogatella furcifera, Deltocephalidae such as Nephotettix cincticeps, Nephotettix virescens, and Empoasca onukii, Aphididae such as Aphis gossypii, Myzus persicae, Brevicoryne brassicae, Aphis spiraecola, Macrosiphum euphorbiae, Aulacorthum solani, Rhopalosiphum padi, Toxoptera citricidus, and Hyalopterus pruni, Pentatomidae such as Nezara antennata, Riptortus clavetus, Leptocorisa chinensis, Eysarcoris parvus, and Halyomorpha mista, Aleyrodidae such as Trialeurodes vaporariorum, Bemisia tabaci, Dialeurodes citri, and Aleurocanthus spiniferus, Coccidae such as Acnidiella aurantii, Comstockaspis perniciosa, Unaspis citri, Ceroplastes rubens, Icerya purchasi, Planococcus kraunhiae, Pseudococcus longispinis, and Pseudaulacaspis pentagona, Tingidae, Cimicoidea such as Cimex lectularius, and Psyliidae.

Lepidoptera: Pyralidae such as Chilo suppressalis, Tryporyza incertulas, Cnaphalocrocis medinalis, Notarcha derogata, Plodia interpunctella, Ostrinia furnacalis, Hellula undalis, and Pediasia teterrellus, Noctuidae such as Spodoptera litura, Spodoptera exigua, Pseudaletia separata, Mamestra brassicae, Agrotis ipsilon, Plusia nigrisigna, Trichoplusia spp., Heliothis spp., and Helicoverpa spp., Pieridae such as Pieris rapae, Adoxophyes spp., Tortricidae such as Grapholita molesta, Leguminivora glycinivorella, Matsumuraeses azukivora, Adoxophyes orana fasciata, Adoxophyes honmai., Homona magnanima, Archips fuscocupreanus, and Cydia pomonella, Gracillariidae such as Caloptilia theivora and Phyllonorycter ringoneella, Carposinidae such as Carposina niponensis, Lyonetiidae such as Lyonetia spp., Lymantriidae such as Lymantria spp. and Euproctis spp., Yponomeutidae such as Plutella xylostella, Gelechiidae such as Pectinophora gossypiella and Phthorimaea operculella, Arctiidae such as Hyphantria cunea, and Tineidae such as Tinea translucens and Tineola bisselliella.

Thysanoptera: Thripidae such as Frankliniella occidentalis, Thrips parmi, Scirtothrips dorsalis, Thrips tabaci, and Frankliniella intonsa.

Diptera: Culex such as Culex pipiens pallens, Culex tritaeniorhynchus, and Culex quinquefasciatus, Aedes spp. such as Aedes aegypti and Aedes albopictus, Anopheles spp. such as Anopheles sinensis, Chironomidae, Muscidae such as Musca domestica and Muscina stabulans, Calliphoridae, Sarcophagidae, Fanniidae, Anthomyiidae such as Delia platura and Delia antiqua, Agromyzidae such as Agromyza oryzae, Hydrellia griseola, Liriomyza sativae, Liriomyza trifolii, and Chromatomyia horticola, Chloropidae such as Chlorops oryzae, Tephritidae such as Dacus cucurbitae and Ceratitis capitata, Drosophilidae, Phoridae such as Megaselia spiracularis, Psychodidae such as Clogmia albipunctata, Sciaridae, Simuliidae, Tabanidae such as Tabanus trigonus, Stomoxys, and Stomoxyidae.

Coleoptera: Corn rootworm such as Diabrotica virgifera virgifera and Diabrotica undecimpunctata howardi, Scarabaeidae such as Anomala cuprea, Anomala rufocuprea, and Popillia japonica, Curculionidae such as Sitophilus zeamais, Lissorhoptrus oryzophilus, Callosobruchuys chienensis, Echinocnemus squameus, Anthonomus grandis, and Sphenophorus venatus, Tenebrionidae such as Tenebrio molitor and Tribolium castaneum, Chrysomelidae such as Oulema oryzae, Aulacophora femoralis, Phyllotreta striolata, and Leptinotarsa decemlineata, Dermestidae such as Anthrenus verbasci and Dermestes maculates, Anobiidae such as Lasioderma serricorne, Epilachna such as Epilachna vigintioctopunctata, Lyctidae such as Lyctus brunneus and Tomicus piniperda, Bostrychidae, Ptinidae, Cerambycidae such as Anoplophora malasiaca, Agriotes spp., and Paederus fuscipes.

Orthoptera: Locusta migratoria, Gryllotalpa africana, Oxya yezoensis, Oxya japonica, and Grylloidea.

Siphonaptera: Ctenocephalides felis, Ctenocephalides canis, Pulex irritans, Xenopsylla cheopis, and the like.

Anoplura: Pediculus humanus corporis, Pediculus humanus humanus, Phthirus pubis, Haematopinus eurysternus, Dalmalinia ovis, Haematopinus suis, Linognathus setosus, and the like. Mallophaga: Dalmalinia ovis, Dalmalinia

bovis, Menopon gallinae, Trichodectes canis, Felicola subrostrata, and the like.

Hymenoptera: Formicidae such as Monomorium pharaosis, Formica fusoa japonica, Ochetellus glaber, Pristomyrmex pungens, Pheidole noda, Acromyrmex spp., Solenopsis spp., and Linepithema humile, Vespidae, Bethylidae, and Tenthredinidae such as Athalia rosaa and Athalia japonica.

Nematoda: Aphelenchoides besseyi, Nothotylenchus acris, Meloidogyne incognita, Meloidogyne hapla, Meloidogyne javanica, Heterodera glycines, Globodera rostochiensis, Pratylenchus coffeae, and Pratylenchus neglectus.

Blattodea: Blattella germanica, Periplaneta fuliginosa, Periplaneta americana, Periplaneta brunnea, and Blatta orientalis.

Isoptera: Reticulitermes speratus, Coptotermes formosanus, Incisitermes minor, Cryptotermes domesticus, Odontotermes formosanus, Neotermes koshunensis, Glyptotermes satsumensis, Glyptotermes nakajimai, Glyptotermes fuscus, Glyptotermes kodamai, Glyptotermes kushimensis, Hodotermopsis japonica, Coptotermes guangzhoensis, Reticulitermes miyatakei, Reticulitermes flaviceps amamianus, Reticulitermes sp., Nasutitermes takasagoensis, Pericapritermes nitobei, Sinocapritermes mushae, and the like.

Acarina: Tetranychidae such as Tetranychus urticae, Tetranychus kanzawai, Panonychus citri, Panonychus ulmi, and Oligonychus spp., Eriophyidae such as Aculops pelekassi, Phyllocoptruta citri, Aculops lycopersici, Calacarus carinatus, Acaphylla theavagrans, Eriophyes chibaensis, and Aculus schlechtendali, Tarsonemidae such as Polyphagotarsonemus latus, Tenuipalpidae such as Brevipalpus phoenicis, Tuckerellidae, Metastigmata such as Haemaphysalis longicornis, Haemaphysalis flava, Dermacentor taiwanicus, Dermacentor variabilis, Ixodes ovatus, Ixodes persulcatus, Ixodes scapularis, Amblyomma americanum, Boophilus microplus, and Rhipicephalus sanguineus, Acaridae such as Tyrophagus putrescentiae and Tyrophagus similis, Pyroglyphidae such as Dermatophagoides farinae and Dermatophagoides ptrenyssnus, Cheyletidae such as Cheyletus eruditus, Cheyletus malaccensis, Cheyletus moorei, and Cheyletiella yasguri, Sarcoptidae such as Octodectes cynotis and Sacroptes scabiei, Demodicidae such as Demodex canis, Listrophoridae, Oribatei, Dermanyssidae such as Ornithonyssus bacoti, Ornithonyssus sylvairum, and Dermanyssus gallinae, Trombiculidae such as Leptotrombidium akamushi, Arachnida such as Chiracanthium japonicum and Latrodectus hasseltii, and the like.

Chilopoda: Thereuonema hilgendorfi, Scolopendra subspinipes, and the like.

Diplopoda: Oxidus gracilis, Nedyopus tambanus, and the like.

Isopoda: Armadillidium vulgare, and the like.

Gastropoda: Limax marginatus, Limax flavus, and the like.

The pest control agent of the present invention contains the compound of the present invention and an inert carrier. The pest control agent of the present invention is usually obtained by mixing the compound of the present invention and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, flowables, microcapsule formulations, aerosols, smoking agents, poisonous baits, resin formulations, shampoo agents, paste formulations, foam agents, carbon dioxide preparations, tablets, and the like. These formulations may be processed into mosquito repellent coils, electric mosquito repellent mats, mosquito repellent liquid formulations, smoking agents, fumigants, sheet formulations, spot-on agents, or oral treatment agents, and used.

The pest control agent of the present invention usually contains the compound of the present invention in an amount of 0.01 to 95% by weight.

Examples of the solid carrier which is used in the formulation include fine powder and granules of clays (kaolin clay, diatomaceous earth, bentonite, Fubasami clay, acid clay, etc.), synthetic hydrated silicon oxide, talc, ceramics, other inorganic minerals (sericite, quartz, sulfur, activated carbon, calcium carbonate, hydrated silica, etc.), fine powder and granulated substances of chemical fertilizers (ammonium sulfate, ammonium phosphate, ammonium nitrate, urea, ammonium chloride, etc.) and the like, and synthetic resins (polyester resins such as polypropylene, polyacrylonitrile, polymethylmethacrylate and polyethylene terephthalate, nylon resins such as nylon-6, nylon-11 and nylon-66, polyamide resin, polyvinyl chloride, polyvinylidene chloride, vinyl chloride-propylene copolymer, and the like).

Examples of the liquid carrier include water, alcohols (methanol, ethanol, isopropyl alcohol, butanol, hexanol, benzyl alcohol, ethylene glycol, propylene glycol, phenoxyethanol, etc.), ketones (acetone, methyl ethyl ketone, cyclohexanone, etc.), aromatic hydrocarbons (toluene, xylene, ethylbenzene, dodecylbenzene, phenylxylylethane, methylnaphthalene, etc.), aliphatic hydrocarbons (hexane, cyclohexane, kerosene, light oil, etc.), esters (ethyl acetate, butyl acetate, isopropyl myristate, ethyl oleate, diisopropyl adipate, diisobutyl adipate, propylene glycol monomethyl ether acetate, etc.), nitriles (acetonitrile, isobutyronitrile, etc.), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, etc.), acid amides (DMF, N,N-dimethylacetamide, etc.), halogenated hydrocarbons (dichloromethane, trichloroethane, carbon tetrachloride, etc.), sulfoxides (DMSO, etc.), and propylene carbonate and vegetable oils (soybean oil, cottonseed oil, etc.). Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkyl sulfonates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, etc.), lignin derivatives, bentonite, synthetic water-soluble polymers (polyvinyl alcohol, polyvinyl pyrrolidone, polyacrylic acid, etc.), PAP (isopropyl acid phosphate), BHT (2,6-di-tert-butyl-4-methylphenol) and BHA (mixtures of 2-tert-butyl-4-methoxyphenol and 3-tert-butyl-4-methoxyphenol).

Examples of a base material of the resin formulation include vinyl chloride polymer, polyurethane and the like, and a plasticizer such as ester phthalates (dimethyl phthalate, dioctyl phthalate, etc.), ester adipates or stearic acid may be added to these base materials as necessary. The resin formulation is obtained by kneading a compound into the base material using an ordinary kneading apparatus, and then molding it by injection molding, extrusion molding, press molding or the like, and can be processed into a plate, film, taped, reticular or string resin formulation by further undergoing molding or cutting step as necessary. These resin formulation is processed into, for example, a collar for animal, an ear tag for animal, a sheet formulation, an induction cord, and a gardening pole.

Examples of a base material of the poisonous bait include grain powder, vegetable oil, sugar, crystalline cellulose and the like, and further, an antioxidant such as dibutylhydroxytoluene and nordihydroguaiaretic acid, a preservative such as dehydroacetic acid, a substance for preventing accidental ingestion by children and pets such as red pepper powder, a pest attractant such as cheese flavor, onion flavor and peanut oil or the like are added as necessary.

The method for controlling pests of the present invention is carried out by applying an effective amount of the compound of the present invention to a pest directly and/or a pest-infested area (plants, soil, in-house, animal body, etc.). In the method for controlling pests of the present invention, the compound is usually used in the form of the pest control agent of the present invention.

When the pest control agent of the present invention is used in pest controlling in the agricultural field, the application amount is usually 1 to 10000 g per the amount of the compound of the present invention per 10000 $m^2$. When the pest control agent of the present invention is formulated into an emulsifiable concentrate, a wettable powder, a flowable or the like, the pest control agent is usually diluted with water for an application so as to have a concentration of the active ingredient of 0.01 to 10000 ppm, and dust formulations, granules and the like are usually applied as they are.

These formulations and formulation solutions diluted with water may be directly applied by being sprayed on a pest or a plant such as crops which should be protected from pests, and also may be applied on a soil in order to control a pest that infests in the soil of cultivated land.

Also, the resin formulation processed into a sheet or string can be also applied by a method such as winding it around crops, spreading it in the vicinity of crops, or spreading it to the soil around crop roots.

When the peat control agent of the present invention is used in controlling the pest that inhabits in the house, the application amount is usually 0.01 to 1000 mg in an amount of the compound of the present invention per 1 $m^2$ of an area to be treated, in the case of using it on a planar area, and is usually 0.01 to 500 mg in an amount of the compound of the present invention per 1 $m^3$ of a space to be treated, in the case of using it in a space. When the pest control agent of the present invention is formulated into an emulsifiable concentrate, a wettable powder, a flowable or the like, the pest control agent is usually diluted with water for an application so as to have a concentration of the active ingredient of 0.1 to 10000 ppm, and oil formulations, aerosols, smoking agents, poisonous baits and the like are applied as they are.

When the arthropod pest control agent of the present invention is used in the control of external parasites on livestock such as cows, horses, pigs, sheep, goats and chickens, and small animals such as dogs, cats, rats and mice, veterinary known methods can be applied to the animals. As specific methods, the formulation is administered, for example, by way of a tablet, mixing in feed, a suppository and injection (intramuscular, subcutaneous, intravenous, intraperitoneal injections, etc.), when systemic control is intended, and the formulation is used, for example, by way of spraying an oil solution or aqueous solution, pour-on or spot-on treatment, washing an animal with a shampoo formulation, or putting a collar or ear tag made of a resin formulation on to an animal, when non-systemic control is intended. The amount of the compound of the present invention when administered to an animal body is usually in the range from 0.1 to 1000 mg per 1 kg of the weight of an animal.

The pest control agent of the present invention can be used in the farmland where the following "crops" are grown.

Crops: corn, rice, wheat, barley, rye, oat, sorghum, cotton, soybean, peanut, sarrazin, sugar beet, rapeseed, sunflower, sugar cane, tobacco, etc.

Vegetables: Solanaceae vegetables (eggplant, tomato, green pepper, hot pepper, potato, etc.), Cucurbitaceae vegetables (cucumber, pumpkin, zucchini, watermelon, melon, etc.), Cruciferae vegetables (Japanese radish, turnip, horseradish, kohlrabi, Chinese cabbage, cabbage, brown mustard, broccoli, cauliflower, etc.), Compositae vegetables (burdock, garland chrysanthemum, artichoke, lettuce, etc.), Liliaceae vegetables (Welsh onion, onion, garlic, asparagus, etc.), Umbelliferae vegetables (carrot, parsley, celery, parsnip, etc.), Chenopodiaceae vegetables (spinach, Swiss chard, etc.), Labiatae vegetables (Japanese mint, mint, basil, etc.), strawberry, sweat potato, yam, aroid, etc.

Fruit trees: pomaceous fruits (apple, common pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruits, etc.), nuts (chestnut, walnut, hazel nut, almond, pistachio, cashew nut, macadamia nut, etc.), berry fruits (blueberry, cranberry, blackberry, raspberry, etc.), grape, persimmon, olive, loquat, banana, coffee, date, coconut, oil palm, etc.

Trees other than fruit trees: tea, mulberry, flowering trees and shrubs (azalea, camellia, hydrangea, sasanqua, Illicium religiosum, cherry tree, tulip tree, crape myrtle, fragrant olive, etc.), street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew, elm, horse-chestnut, etc.), sweet viburnum, Podocarpus macrophyllus, Japanese cedar, Japanese cypress, croton, spindle tree, Chainese howthorn, etc.

Lawn: zoysia (Japanese lawn grass, mascarene grass, etc.), Bermuda grass (Cynodon dactylon, etc.), bent grass (creeping bent grass, Agrostis stolonifera, Agrostis tenuis, etc.), bluegrass (Kentucky bluegrass, rough bluegrass, etc.), fescue (tall fescue, chewing fescue, creeping fescue, etc.), ryegrass (darnel, perennial ryegrass, etc.), cocksfoot, timothy grass, etc.

Others: flowers (rose, carnation, chrysanthemum, Eustoma grandiflorum Shinners (prairie gentian), gypsophila, gerbera, pot marigold, salvia, petunia, verbena, tulip, aster, gentian, lily, pansy, cyclamen, orchid, lily of the valley, lavender, stock, ornamental kale, primula, poinsttia, gladiolus, cattleya, daisy, cymbidium, begonia, etc.), bio-fuel plants (Jatropha, curcas, safflower, Camelina alyssum, switchgrass, miscanthus, reed canary grass, Arundo donax, kenaf, cassava, willow, algae, etc.), foliage plants, etc.

The "crops" also contains genetically modified crops.

The pest control agent of the present invention can be used as a mixture with or in combination with other insecticide, miticide, nematicide, fungicide, plant growth regulator, herbicide or synergist. Examples of the active ingredient of said insecticide, miticide, nematicide, fungicide, herbicide and synergist are shown below.

Active Ingredients of Insecticide

(1) Organic Phosphorus Compounds

acephate, Aluminium phosphide, butathiofos, cadusafos, chlorethoxyfos, chlorfenvinphos, chlorpyrifos, chlorpyrifos-methyl, cyanophos: CYAP, diazinon, DCIP (dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, Hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos, tetrachlorvinphos, terbufos, thiometon, trichlorphon: DEP, vamidothion, phorate, and cadusafos.

(2) Carbamate Compounds

alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, and aldicarb.

(3) Pyrethroid Compounds

acrinathrin, allethrin, benfluthrin, beta-cyfluthrin, bifenthrin, cycloprothrin, oyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, 2,3,5,6-tetrafluoro-4-(methoxymethyl)benzyl (EZ)-(1RS, 3RS; 1RS, 3SR)-2,2-dimethyl-3-prop-1-enylcyclopropanecarboxylate, 2,3,5,6-tetrafluoro-4-methylbenzyl (EZ)-(1RS, 3RS; 1RS, 3SR)-2,2-dimethyl-3-prop-1-enylcyclopropanecarboxylate, and 2,3,5,6-tetrafluoro-4-(methoxymethyl)benzyl (1RS, 3RS; 1RS, 3SR)-2,2-dimethyl-3-(2-methyl-1-propenyl)cyclopropanecarbox ylate, 2,3,5,6-tetrafluoro-4-(methoxymethyl)benzyl(EZ)-(1RS, 3RS; 1RS, 3SR)-2,2-dimethyl-3-(2-cyano-1-propenyl)cyclopropanecarboxy late.

(4) Nereistoxin Compounds

cartap, bensultap, thiocyclam, monosultap, and bisultap.

(5) Neonicotinoid Compounds

imidacloprid, nitenpyram, acetamiprid, thiamethoxam, thiacloprid, dinotefuran, and clothianidin.

(6) Benzoyl Urea Compounds

chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron.

(7) Phenylpyrazole-Based Compounds

acetoprole, ethiprole, fipronil, vaniliprole, pyriprole, and pyrafluprole.

(8) Bt Toxins

Living spores derived from Bacillus thuringiensis and produced crystalline toxins and mixtures thereof.

(9) Hydrazine Compounds

chromafenozide, halofenozide, methoxyfenozide, and tebufenozide.

(10) Organic Chlorine Compounds

aldrin, dieldrin, dienochlor, endosulfan, and methoxychlor.

(11) Other Active Ingredients of Insecticide

machine oil, nicotine-sulfate; avermectin-B, bromopropylate, buprofezin, chlorphenapyr, cyantraniliprole, cyromazine, D-D(1, 3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, Potassium oleate, protrifenbute, spiromesifen, sulfoxaflor, Sulfur, metaflumizone, spirotetramat, pyrifluquinazone, spinetoram, chlorantraniliprole, tralopyril, cyantraniliprole, compounds represented by the following formula (K):

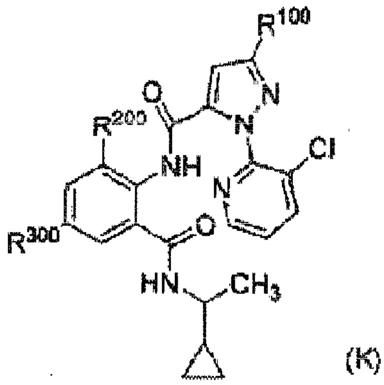

(K)

wherein $R^{100}$ represents chlorine, bromine or a trifluoromethyl group,
$R^{200}$ represents chlorine, bromine or a methyl group, and
$R^{300}$ represents chlorine, bromine or a cyano group, and compounds represented by the following formula (L):

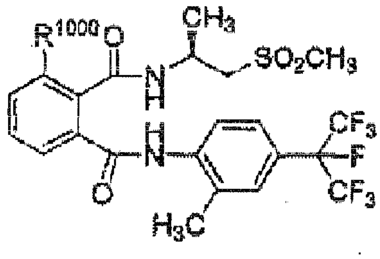

(L)

wherein $R^{1000}$ represents chlorine, bromine or iodine.

Active ingredients of Miticide

acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fencthiocarb, fenpyroximate, fluacrypyrim, fluproxyfen, hexythiazox, propargite: BPPS, polynactins, pyridaben, Pyrimidifen, tebufenpyrad, tetradifon, spirodiclofen, spiromesifen, spirotetramat, amidoflumet, and cyenopyrafen.

Active ingredients of Nematicide

DCIP, fosthiazate, levamisol, methyisothiocyanate, morantel tartarate, and imicyafos.

Active ingredients of Fungicide

azole fungicidal compounds such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, and flutriafol;
Cyclic amine fungicidal compounds such as fenpropimorph, tridemorph, and fenpropidin;
Benzimidazole fungicidal compounds such as carbendezim, benomyl, thiabendazole, and thiophanate-methyl; procymidone; cyprodinil; pyrimethanil; diethofencarb; thiuram; fluazinam; mancozeb; iprodione; vinolozolin; chlorothalonil; captan; mepanipyrim; fenpiclonil; fludioxonil; dichlofluanid; folpet; kresoxim-methyl; azoxystrobin; trifloxystrobin; fluoxastrobin; picoxyetrobin; pyraclostrobin; dimoxystrobin; pyribencarb; spiroxamine; quinoxyfen; fenhexamid; famoxadone; fenamidone; zoxamide; ethaboxam; amisulbrom; iprovalicarb; benthiavalicarb; cyazofamid; mandipropamid; boscalid; penthiopyrad; metrafenone; fluopiran; bixafen; cyflufenamid; proquinazid; isotianil and tiadinil;

Active ingredients of Herbicide

(1) Phenoxy fatty acid herbicidal compounds
2,4-PA, MCP, MCPB, phenothiol, mecoprop, fluroxypyr, triclopyr, clomeprop, and naproanilide.
(2) Benzoate herbicidal compounds
2,3,6-TBA, dicamba, clopyralid, picloram, aminopyralid, quinclorac, and quinmerac.
(3) Urea herbicidal compounds
diuron, linuron, chlortoluron, isoproturon, fluometuron, isouron, tebuthiuron, methabenzthiazuron, cumyluron, daimuron, and methyl-daimuron.
(4) Triazine herbicidal compounds
atrazine, ametoryn, cyanazine, simazine, propazine, simetryn, dimethametryn, prometryn, metribuzin, triaziflam, and indaziflam.
(5) Bipyridinium herbicidal compounds
paraquat, and diquat.
(6) Hydroxybenzonitrile herbicidal compounds
bromoxynil, and ioxynil.
(7) Dinitroaniline herbicidal compounds
pendimethalin, prodiamine, and trifluralin.
(8) Organophosphorus herbicidal compounds

amiprofos-methyl, butamifos, bensulide, piperophos, anilofos, glyphosate, glufosinate, glufosinate-P, and bialaphos.
(9) Carbamate herbicidal compounds
di-allate, tri-allate, EPTC, butylate, benthiocarb, esprocarb, molinate, dimepiperate, swep, chlorpropham, phenmedipham, phenisopham, pyributicarb, and asulam.
(10) Acid amide herbicidal compounds
propanil, propyzamide, bromobutide, and etobenzanid.
(11) Chloroacetanilide herbicidal compounds
acetochlor, alachlor, butachlor, dimethenamid, propachlor, metazachlor, metolachlor, pretilachlor, thenylchlor, and pethoxamid.
(12) Diphenyl ether herbicidal compounds
acifluorfen-sodium, bifenox, oxyfluorfen, lactofen, fomesafen, chlomethoxynil, and aclonifen.
(13) Cyclic imide herbicidal compounds
oxadiazon, cinidon-ethyl, carfentrazone-ethyl, surfentrazone, flumiclorac-pentyl, flumioxazin, pyraflufen-ethyl, oxadiargyl, pentoxazone, fluthiaoet-methyl, butafenacil, benzfendizone, bencarbazone, and saflufenacil.
(14) Pyrazole herbicidal compounds
benzofenap, pyrazolate, pyrazoxyfen, topramezone, and pyrasulfotole.
(15) Triketone herbicidal compounds
isoxaflutole, benzobicyclon, sulcotrione, mesotrione, tembotrione, and tefuryltrione.
(16) Aryloxyphenoxypropionate herbicidal compounds clodinafop-propargyl, cyhalofop-butyl, diclofop-methyl, fenoxaprop-ethyl, fluazifop-butyl, haloxyfop-methyl, and quizalofop-ethyl, metamifop.
(17) Trione oxime herbicidal compounds
alloxydim-sodium, sethoxydim, butroxydim, clethodim, cloproxydim, cycloxydim, tepraloxydim, tralkoxydim, and profoxydim.
(18) Sulfonyl urea herbicidal compounds
chlorsulfuron, sulfometuron-methyl, metsulfuron-methyl, chlorimuron-ethyl, tribenuron-methyl, triasulfuron, metsulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.
(19) Imidazolinone herbicidal compounds
imazamethabenz-methyl, imazamethapyr, imazamox, imazapyr, imazaquin, and imazethapyr.
(20) Sulfonamide herbicidal compounds
flumetsulam, metosulam, diclosulam, florasulam, cloransulam-methyl, penoxsulam, and pyroxsulam.
(21) Pyrimidinyloxybenzoate herbicidal compounds pyrithiobac-sodium, bispyribac-sodium, pyriminobac-methyl, pyribenzoxim, pyriftalid, and pyrimisulfan.
(22) Other herbicidal compounds
bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium, mefenacet, flufenacet, fentrazamide, cafenstrole, indanofan, oxaziclomefone, benfuresate, ACN, pyridate, chloridazon, norflurazon, flurtamone, diflufenican, picolinafen, beflubutamid, clomazone, amicarbazone, pinoxaden, pyraclonil, pyroxasulfone, thiencarbazone-methyl, aminocyclopyrachlor, ipfencarbazone, and methlozolin.

Active ingredients of Synergist

piperonyl butoxide, sesamex, sulfoxide, N-(2-ethylhexyl)-8,9,10-trinorborn-5-ene-2,3-dioarboximide (MGK 264), N-declyimidazole), WARF-antiresistant, TBPT, TPP, IBP, PSCP, $CH_3I$, t-phenylbutenone, diethylmaleate, DMC, FDMC, ETP, and ETN.

EXAMPLES

Hereinbelow, the present invention will be further described in detail by production examples, reference examples, formulation examples, test examples, and the like. However, the present invention is not limited to these examples.
First, the production examples for the production of the compounds of the present invention are shown below.
Production Example 1 (1)
1.5 mL of ethyl mercaptan was added to a mixture of 3.83 g of 2-fluoro-4-trifluoromethylbenzoic acid, 10 mL of NMP and 15 g of cesium carbonate, and the mixture was stirred at 60°C for 4 hours. A saturated aqueous sodium bicarbonate solution was poured to the cooled reaction mixture, and the mixture was extracted with ethyl acetate.

Hydrochloric acid was added to the aqueous layer, and the precipitated solid was filtered and dried under reduced pressure to obtain 4.6 g of 2-ethylsulfanyl-4-trifluoromethylbenzoic acid. 2-Ethylsulfanyl-4-trifluoromethylbenzoic acid

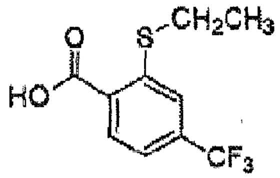

$^1$H-NMR($CDCl_3$) δ: 8.22(1H, d), 7.55(1H, s), 7.43(1H, d), 3.02(2H, q), 1.43(3H, t).

Production Example 1 (2)

A mixture of 0.32 g of 4-trlfluoromethylaniline, 0.50 g of 2-ethylsulfanyl-4-trifluoromethylbenzoic acid, 0.57 g of EDCI hydrochloride, 0.02 g of HOBt and 5 ml of pyridine was stirred at 60°C for 2 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting solid was washed with hexane to obtain 0.72 g of 2-ethylsulfanyl-4-trifluoromethyl-N-(4-trifluoromethyl-phen yl)-benzamide.

2-Ethylsulfanyl-4-trifluoromethyl-N-(4-trifluoromethyl-phen yl)-benzamide

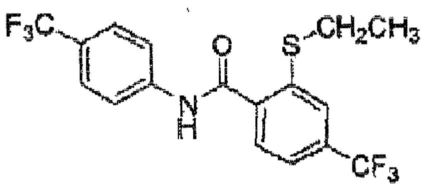

$^1$H-NMR($CDCl_3$)δ: 8.75(1H, s), 7.92(1H, d), 7.79(2H, d), 7.70(1H, s), 7.65(2H, d), 7,58(1H, d), 3.01(2H, q), 1.33(3H, t).
Production Example 1 (3)

0.23 g of potassium carbonate and 0.35 mL of methyl iodide were added to a mixture of 0.45 g of 2-ethylsulfanyl-4-trifluoromethyl-N-(4-trifluoromethyl-phen yl) -benzamide and 6 mL of acetone, and the mixture was stirred at 60°C for 5 hours. 0.35 mL of methyl iodide and 2 mL of DMF were added to the cooled reaction mixture, and the mixture was stirred at 60°C for 5 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 0.38 g of 2-ethylaulfanyl-N-methyl-4-trifluoromethyl-N-(4-trifluorome thyl-phenyl)-benzamide (hereinafter, referred to as Compound of Present Invention 1).

Compound of Present Invention 1

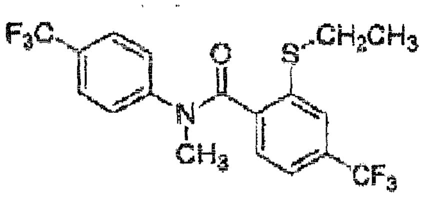

$^1$H-NMR ($CDCl_3$)δ: 7.75-6.92(7H, m), 3.50(3H, brs), 3.09-2.90(2H, m), 1.40-1.30(3H, m).

Production Example 2 (1)

A mixture of 0.35 g of 4-trifluoromethoxyaniline, 0.50 g of 2-ethylsulfanyl-4-trifluoromethylbenzoic acid, 0.57 g of EDCI hydrochloride, 0.02 g of HOBt and 5 ml of pyridine was stirred at 60°C for 2 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting solid was washed with hexane to obtain 0.75 g of 2-ethylaulfanyl-N-(4-trifluoromethoxyl-phenyl)-4-trifluorom ethyl-benzamide.

2-Ethylsulfanyl-N-(4-trifluoromethoxyl-phenyl)-4-trifluorom ethyl-benzamide

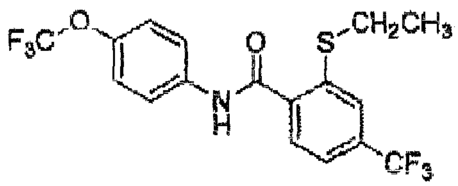

$^1$H-NMR($CDCl_3$)$\delta$: 8.60(1H, s), 7.90(1H, d), 7.74-7.64(3H, m), 7.57(1H, d), 7.25(2H, d), 3.01(2H, q), 1.33(3H, t). Production Example 2 (2)

0.22 g of potassium carbonate and 0.34 mL of methyl iodide were added to a mixture of 0.45 g of 2-ethylsulfanyl-N-(4-trifluoramethaxyl-phenyl)-4-trifluorom ethyl-benzamide and 6 mL of acetone, and the mixture was stirred at 60°C for 5 hours. 0.34 mL of methyl iodide and 2 mL of DMF were added to the cooled reaction mixture, and the mixture was stirred at 60°C for 5 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 0.42 g of 2-ethylsulfanyl-N-methyl-N-(4-trifluoromethoxyl-phenyl)-4-t rifluoromethyl-benzamide (hereinafter, referred to as Compound of Present Invention 2).

Compound of Present Invention 2

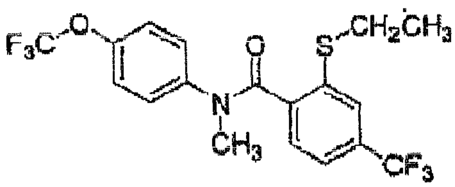

$^1$H-NMR($CDCl_3$)$\delta$: 7.70-6.92(7H, m), 3.47(3H, brs), 2.99(2H, q), 1.33(3H, t).

Production Example 3 (1)

A mixture of 0.42 g of 4-pentafluoroethylaniline, 0.50 g of 2-ethylsulfanyl-4-trifluoromethylbenzoic acid, 0.57 g of EDCI hydrochloride, 0.02 g of HOBt and 5 ml of pyridine was stirred at 60°C for 2 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting solid was washed with hexane to obtain 0.75 g of 2-ethylsulfanyl-N-(4-pentafluoroethyl-phenyl)-4-trifluorome thyl-benzamide.

2-Ethylsulfanyl-N-(4-pentafluoroethyl-phenyl)-4-trifluorome thyl-benzamide

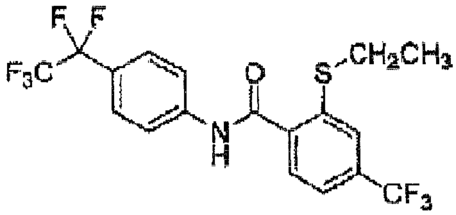

$^1H$-NMR($CDCl_3$)δ: 8.75(1H, s), 7.92(1H, d), 7.81(2H, d), 7.69(1H, s), 7.62(2H, d), 7.59(1H, d), 3.02(2H, q), 1.34(3H, t).
Production Example 3 (2)

0.25 g or potassium carbonate and 0.38 mL of methyl iodide were added to a mixture of 0.50 g of 2-ethylsulfanyl-N-(4-pentafluoroethyl-phenyl)-4-trifluorome thyl-benzamide and 6 mL of acetone, and the mixture was stirred at 60°C for 5 hours. 0.38 mL of methyl iodide and 2 mL of DMF were added to the cooled reaction mixture, and the mixture was stirred at 60°C for 5 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 0.52 g of 2-ethylsulfanyl-N-methyl-N-(4-pentafluoroethyl-phenyl)-4-tr ifluoromethyl-benzamide (hereinafter, referred to as Compound of Present Invention 3).

Compound of Present Invention 3

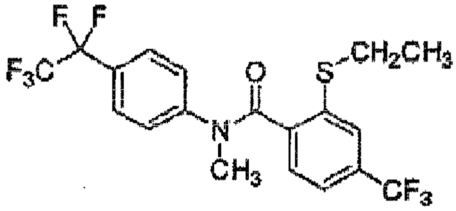

$^1H$-NMR($CDCl_3$) δ: 7.78-7.09(7H, m), 3.47(3H, bra), 2.97(2H, q), 1.32(3H, t).

Production Example 4 (1)

A mixture of 0.38 g of 4-trifluoromethylsulfanylaniline, 0.50 g of 2-ethylsulfanyl-4-trifluoromethylbenzoic acid, 0.57 g of EDCI hydrochloride, 0.02 g of HOBt and 5 ml of pyridine was stirred at 60°C for 2 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting solid was washed with hexane to obtain 0.74 g of 2-ethylsulfanyl-N-(4-trifluoromethylsulfanyl-phenyl)-4-trif luoromethyl-benzamide.

2-Ethylsulfanyl-N-(4-trifluoromethylsulfanyl-phenyl)-4-trif luoromethyl-benzamide

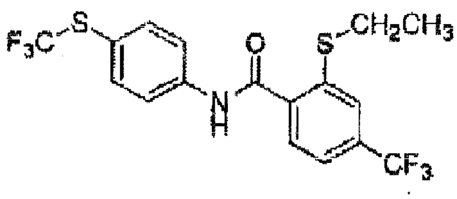

$^1H$-NMR($CDCl_3$)δ: 8.71(1H, s), 7.91(1H, d), 7.75(2H, d), 7.71-7.65(3H, m), 7.58(1H, d), 3.01(2H, q), 1.34(3H, t). Production Example 4 (2)

0.25 g of potassium carbonate and 0.38 mL of methyl iodide were added to a mixture of 0.50 g of 2-ethylsulfanyl-N-(4-trifluoromethylsulfanyl-phenyl)-4-trif luoromethyl-benzamide and 6 mL of acetone, and the mixture was stirred at

60°C for 5 hours. 0.38 mL of methyl iodide and 2 mL of DMF were added to the cooled reaction mixture, and the mixture was stirred at 60°C for 5 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 0.53 g of 2-ethylsulfanyl-N-methyl-4-trifluoromethyl-N-(4-trifluorome thylsulfanyl-phenyl)-benzamide (hereinafter, referred to as Compound of Present Invention 4).

Compound of Present Invention 4

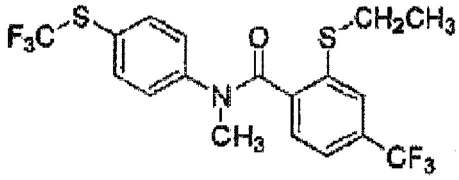

$^1$H-NMR(CDCl$_3$)δ: 7.69-7.08(7H, m), 3.48(3H, bra), 2.98(2H, q), 1.32(3H, t).

Production Example 5 (1)

A mixture of 0.45 g of 3,5-bis(trifluoromethyl)-aniline, 0.50 g of 2-ethylaulfanyl-4-trifluoromethylbenzoic acid, 0.57 g of EDCI hydrochloride, 0.02 g of HOBt and 5 ml of pyridine was stirred at 60°C for 2 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting solid was washed with hexane to obtain 0.80 g of N-(3,5-bis-trifluoromethyl-phenyl)-2-ethylsulfanyl-4-triflu oromethyl-benzamide.

N-(3,5-Bis-trifluoromethyl-phenyl)-2-ethylaulfanyl-4-triflu oromethyl-benzamide

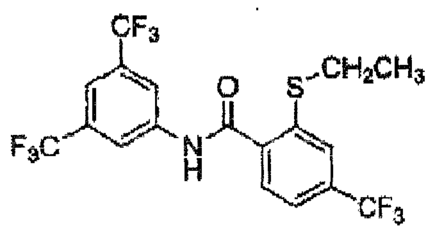

$^1$H-NMR(CDCl$_3$)δ: 9.00(1H, s), 8.17(2H, s), 7.95(1H, d), 7.72(1H, s), 7.69(1H, s), 7.61(1H, d), 3.04(2H, q), 1.35(3H, t).

Production Example 5 (2)

0.25 g of potassium carbonate and 0.38 mL of methyl iodide were added to a mixture of 0.50 g of N-(3,5-bis-trifluoromethyl-phenyl)-2-ethylsulfanyl-4-triflu oromethyl-benzamide and 6 mL of acetone, and the mixture was stirred at 60°C for 5 hours. 0.38 mL of methyl iodide and 2 mL of DMF were added to the cooled reaction mixture and the mixture was stirred at 60°C for 5 hours. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 0.37 g of N-(3,5-bis-trifluoromethyl-phenyl)-2-ethylsulfanyl-N-methyl -4-trifluoromethyl-benzamide (hereinafter, referred to as Compound of Present Invention 5).

Compound of Present Invention 5

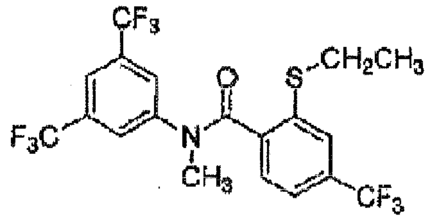

$^1$H-NMR($CDCl_3$) δ: 7.77-7.19(6H, m), 3.50(3H, brs), 3.05-2.85(2H, m), 1.32(3H, t).

Production Example 6 (1)

7.9 g of thionyl chloride was added to a mixture of 7.23 g of 4-bromo-2-fluorobenzoic acid, 30 mL of toluene and 0.1 ml of DMF, and the mixture was stirred at 80 °C for 1 hour. The reaction mixture was concentrated under reduced pressure, and 30 ml of toluene, 6.22 g of N-methyl-4-trifluoromethylsulfanylaniline and 7.74 g of diisopropylethylamine were added, and the mixture was stirred at 80°C for 1 hour. A saturated aqueous sodium bicarbonate solution was poured to the cooled reaction mixture, and the mixture was extracted with ethyl acetate. The organic layer was dried over anhydrous sodium sulfate and then concentrated under reduced pressure, and the resulting residue was applied to a silica gel column chromatography to obtain 12.84 g of N-(4-trlfluoromethylsulfanyl-phenyl)-4-bromo-2-fluoro-N-met hyl-benzamide.

N-(4-Trifluoromethylsulfanyl-phenyl)-4-bromo-2-fluoro-N-met hyl-benzamide

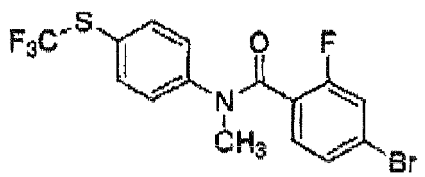

$^1$H-NMR($CDCl_3$) δ: 7.53(2H, d), 7.26-7.22(2H, m), 7.16-7.08(2H, m), 7.07-7.00(1H, m), 3.49(3H, s).

Production Example 6 (2)

1.05 g of sodium ethanethiolate was added to a mixture of 4.08 g of N-(4-trifluoromethylsulfanyl-phenyl)-4-bromo-2-fluoro-N-met hyl-benzamide and 30 ml of DMF under ice cooling, and the mixture was stirred at room temperature for 2 hours. A saturated aqueous sodium chloride solution was poured to the reaction mixture, and the mixture was extracted with ethyl acetate. The organic layer was dried over anhydrous sodium sulfate and then concentrated under reduced pressure, and the resulting residue was applied to a silica gel column chromatography to obtain 3.80 g of a mixture of N-(4-trifluoromethylsulfanyl-phenyl)-4-bromo-2-fluoro-N-met hyl-benzamide, N-(4-trifluoromethylsulfanyl-phenyl)-4-bromo-2-ethylsulfony 1-N-methyl-benzamide, and N-(4-trlfluoromethylsulfanyl-phenyl)-2-fluoro-4-ethylsulfan yl-N-methyl-benzamide.

3.22 g of m-chloroperbenzoic acid (purity of 68%) was added to a mixture of the orude product obtained above and 20 ml of chloroform under ice cooling, and the mixture was stirred at room temperature for 2 hours. A saturated aqueous sodium bicarbonate solution and a saturated aqueous sodium thiosulfate solution were added to the reaction mixture, and the mixture was extracted with ethyl acetate. The organic layer was dried over anhydrous sodium sulfate and then concentrated under reduced pressure, and the resulting residue was applied to a silica gel column chromatography to obtain 2.00 g of N-(4-trifluoromethylsulfanyl-phenyl)-4-bromo-2-ethylsulfony 1-N-methyl-benzamide (hereinafter, referred to as Compound of Present Invention 88).

Compound of Present Invention 88

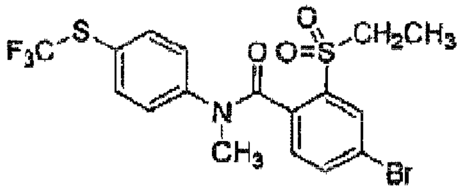

$^1$H-NMR($CDCl_3$)$\delta$: 8.24-6.81(7H, m), 3.66-3.15(5H, m), 1.39-1.29(3H, m).

Production Example 7

Carbon monoxide at 10 atmospheric pressure was introduced to a mixture of 1.77 g of Compound of Present Invention 88, 25 mg of dichlorobis(triphenylphosphine)palladium(II), 60 mg of 1,1'-bis(diphenylphosphino)ferrocene, 0.55 g of potassium carbonate, 1 ml of ethanol and 10 ml of toluene, and the mixture was stirred at 105°C for 6 hours. The cooled reaction mixture was released into water, and the mixture was extracted with ethyl acetate. The organic layer was dried over anhydrous sodium sulfate and then concentrated under reduced pressure, and the resulting residue was applied to a silica gel column chromatography to obtain 0.27 g of 3-ethylsulfonyl-N-methyl-N-(4-trifluoromethylsulfanyl-pheny 1)-ethyl terephthalate (hereinafter, referred to as Compound of Present Invention 87).

Compound of Present Invention 87

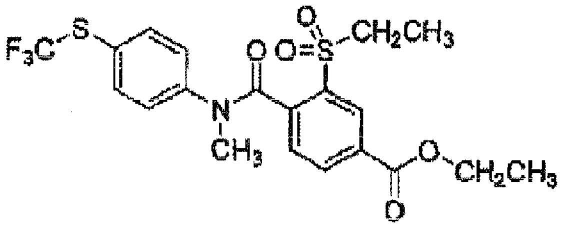

$^1$H-NMR($CDCl_3$)$\delta$: 8.73-7.00(7H, m), 4.54-4.27(2H, m), 3.68-3.15(5H, m), 1.50-1.23(3H, m).

Production Example 8

A mixture of 1.42 g of Compound of Present Invention 87, 0.14 g of lithium hydroxide, 6 ml of THF, 3 ml of methanol and 3 ml of water was stirred at room temperature for 2 hours. A saturated aqueous ammonium chloride solution and a 2 N hydrochloric acid were added to the reaction mixture, and the mixture was extracted with chloroform. The organic layer was dried over anhydrous sodium sulfate and then concentrated under reduced pressure to obtain 1.34 g of 3-ethylsulfonyl-N-methyl-N-(4-trifluoromethylsulfanyl-pheny 1)-terephthallc acid (hereinafter, referred to as Compound of Present Invention 91).

Compound of Present Invention 91

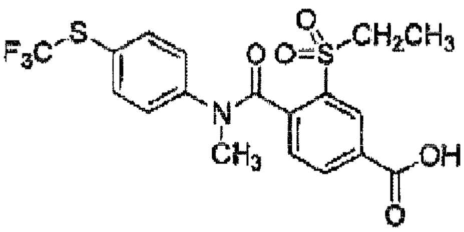

$^1$H-NMR($CDCl_3$)$\delta$: 8.77-8.61(1H, m), 8.48-8.00(1H, m), 7.78-7.09(5H, m), 3.81-3.18(5H, m), 1.91-1.85(1H, m),

1.38-1.29(3H, m).

Production Method 9

0.66 g of oxalyl chloride was added to a mixture of 1.16 g of Compound of Present Invention 91, 15 ml of chloroform and 0.1 mL of DMF under ice cooling, and the mixture was stirred at room temperature for 2 hours. The reaction mixture was concentrated under reduced pressure, and 9 mL of chloroform was added. 3 ml of the reaction mixture was transferred to another reaction vessel, 0.52 g of aqueous ammonia (28%) was added, and the mixture was stirred at room temperature for 1 hour. A saturated aqueous sodium bicarbonate solution was poured to the reaction mixture, and the mixture was extracted with ethyl acetate. The organic layer was dried over anhydrous sodium sulfate and then concentrated under reduced pressure, and the resulting residue was applied to a silica gel column chromatography to obtain 0.26 g of 3-ethylsulfonyl-$N^1$-methyl-$N^1$-(4-trifluoromethylsulfanyl-phe nyl)-terephthalamide (hereinafter, referred to as Compound of Present Invention 94).

Compound of Present Invention 94

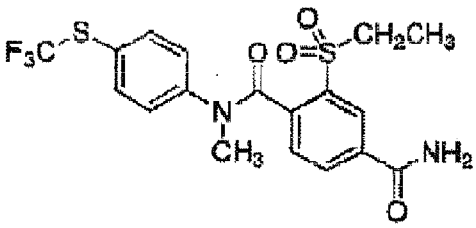

$^1$H-NMR($CDCl_3$)$\delta$: 8.4$\delta$-8.29(1H, m), 7.88-7.09(6H, m), 6.52-5.75(2H, m), 3.71-3.18(5H, m), 1.37-1.29(3H, m).

The compounds described in the production examples described above and the compounds manufactured by the manufacturing method according to the method described in the production examples described above are shown in the tables. The compounds of the present invention represented by the formula (1):

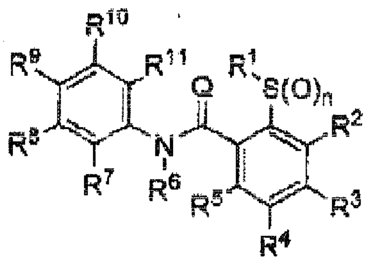

(1)

wherein $R^1$, $R^2$, $R^3$, $R^4$, $R^5$, $R^6$, $R^7$, $R^8$, $R^9$, $R^{10}$, $R^{11}$, n and Q represent the combinations shown in [Table 28] to [Table 32] shown below.

[Table 28]

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 1 | Et | H | $CF_3$ | H | H | $CH_3$ | H | H | $CF_3$ | H | H | 0 | 0 |
| 2 | Et | H | $CF_3$ | H | H | $CH_3$ | H | H | $OCF_3$ | H | H | 0 | 0 |
| 3 | Et | H | $CF_3$ | H | H | $CH_3$ | H | H | $CF_2CF_3$ | H | H | 0 | 0 |
| 4 | Et | H | $CF_3$ | H | H | $CH_3$ | H | H | $SCF_2$ | H | H | 0 | 0 |
| 5 | Et | H | $CF_3$ | H | H | $CH_3$ | H | $CF_3$ | H | $CF_3$ | H | 0 | 0 |
| 6 | Et | H | $CF_3$ | H | H | $CH_3$ | H | Cl | H | Cl | H | 0 | 0 |
| 7 | Et | H | $CF_3$ | H | H | $CH_3$ | H | F | H | F | H | 0 | 0 |

(continued)

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 8 | Et | H | $CF_3$ | H | H | $CH_3$ | H | H | $CF_3$ | H | H | 2 | 0 |
| 9 | Et | H | $CF_3$ | H | H | $CH_3$ | H | H | $OCF_3$ | H | H | 2 | 0 |
| 10 | Et | H | $CF_3$ | H | H | $CH_3$ | H | H | $CF_2CF_3$ | H | H | 2 | 0 |
| 11 | Et | H | $CF_3$ | H | H | $CH_3$ | H | H | $SCF_3$ | H | H | 2 | 0 |
| 12 | Et | H | $CF_3$ | H | H | $CH_3$ | H | H | $SOCF_3$ | H | H | 2 | 0 |
| 13 | Et | H | $CF_3$ | H | H | $CH_3$ | H | $CF_3$ | H | $CF_3$ | H | 2 | 0 |
| 14 | Et | H | $CF_3$ | H | H | $CH_3$ | H | C1 | H | Cl | H | 2 | 0 |
| 15 | Et | H | $CF_3$ | H | H | $CH_3$ | H | F | H | F | H | 2 | 0 |
| 16 | Et | H | H | H | H | $CH_3$ | H | H | $CF_3$ | H | H | 0 | 0 |
| 17 | Et | H | H | H | H | $CH_3$ | H | H | $OCF_3$ | H | H | 0 | 0 |
| 18 | Et | H | H | H | H | $CH_3$ | H | H | $CF_2CF_3$ | H | H | 0 | 0 |
| 19 | Et | H | H | H | H | $CH_3$ | H | H | $SCF_3$ | H | H | 0 | 0 |
| 20 | Et | H | H | H | H | $CH_3$ | H | $CF_3$ | H | $CF_3$ | H | 0 | 0 |
| 21 | Et | H | H | H | H | $CH_3$ | H | Cl | H | Cl | H | 0 | 0 |
| 22 | Et | H | H | H | H | $CH_3$ | H | F | H | F | H | 0 | 0 |

[Table 29]

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 23 | Et | H | H | H | H | $CH_3$ | H | H | $CF_3$ | H | H | 2 | 0 |
| 24 | Et | H | H | H | H | $CH_3$ | H | H | $OCF_3$ | H | H | 2 | 0 |
| 25 | Et | H | H | H | H | $CH_3$ | H | H | $CF_2CF_3$ | H | H | 2 | 0 |
| 26 | Et | H | H | H | H | $CH_3$ | H | H | $SCF_2$ | H | H | 2 | 0 |
| 27 | Et | H | H | H | H | $CH_3$ | H | $CF_3$ | H | $CF_3$ | H | 2 | 0 |
| 28 | Et | H | H | H | H | $CH_3$ | H | Cl | H | Cl | H | 2 | 0 |
| 29 | Et | H | H | H | H | $CH_3$ | H | F | H | F | H | 2 | 0 |
| 30 | Et | H | F | H | H | $CH_3$ | H | H | $CF_2CF_2$ | H | H | 2 | 0 |
| 31 | Et | H | Cl | H | H | $CH_3$ | H | H | $CF_2CF_3$ | H | H | 2 | 0 |
| 32 | Et | H | Br | H | H | $CH_3$ | H | H | $CF_2CF_3$ | H | H | 2 | 0 |
| 33 | Et | H | I | H | H | $CH_3$ | H | H | $CF_2CF_3$ | H | H | 2 | 0 |
| 34 | Et | H | 2-pyridyl | H | H | $CH_3$ | H | H | $CF_2CF_3$ | H | H | 2 | 0 |
| 35 | Et | H | 2-pyrimidyl | H | H | $CH_3$ | H | H | $CF_2CF_3$ | H | H | 2 | 0 |
| 36 | Et | H | $CH_3$ | H | H | $CH_3$ | H | H | $CH_2CF_3$ | H | H | 2 | 0 |
| 37 | Et | H | $OCH_3$ | H | H | $CH_3$ | H | H | $CF_2CF_3$ | H | H | 2 | 0 |
| 38 | Et | H | $CF_3$ | H | H | $CH_2CH_3$ | H | H | $CH_2CF_3$ | H | H | 2 | 0 |
| 39* | Et | H | $CF_3$ | H | H | CycPr* | H | H | $CH_2CF_3$ | H | H | 2 | 0 |
| 40* | Et | H | $CF_3$ | H | H | $CH_2$CycPr* | H | H | $CF_2CF_3$ | H | H | 2 | 0 |
| 41* | Et | H | $CF_3$ | H | H | $CH_2CCH$* | H | H | $CF_2CF_3$ | H | H | 2 | 0 |
| 42* | Et | H | $CF_3$ | H | H | $COCH_3$* | H | H | $CF_2CF_3$ | H | H | 2 | 0 |
| 43* | Et | H | $CF_3$ | H | H | $CH_3OCH_3$ * | H | H | $CF_2CF_3$ | H | H | 2 | 0 |
| 44 | Et | H | $CF_3$ | H | H | $CH_3$ | H | H | Pr | H | H | 0 | 0 |
| * reference example | | | | | | | | | | | | | |

[Table 30]

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 45 | Et | H | $CF_3$ | H | H | $CH_3$ | H | H | Br | H | H | 2 | 0 |
| 46 | Et | H | $CF_3$ | H | H | $CH_3$ | H | H | $CH_3$ | H | H | 0 | 0 |
| 47 | Et | H | $CF_3$ | H | H | $CH_3$ | H | H | $CH_3$ | H | H | 2 | 0 |
| 48 | Et | H | $CF_3$ | H | H | $CH_3$ | H | H | $CH_2CH_3$ | H | H | 0 | 0 |
| 49 | Et | H | $CF_3$ | H | H | $CH_3$ | H | H | $CH_2CH_3$ | H | H | 2 | 0 |
| 50 | Et | H | $CF_3$ | H | H | $CH_3$ | H | H | $CH(CH_3)_2$ | H | H | 0 | 0 |
| 51 | Et | H | $CF_3$ | H | H | $CH_3$ | H | F | $CH(CH_3)_2$ | H | H | 2 | 0 |
| 52 | Et | H | $CF_3$ | H | H | $CH_3$ | H | H | $CF_3$ | H | F | 0 | 0 |
| 53 | Et | H | $CF_3$ | H | H | $CH_3$ | H | $CF_3$ | F | H | H | 0 | 0 |
| 54 | Et | H | $CF_3$ | H | H | $CH_3$ | H | $CF_3$ | Cl | H | H | 0 | 0 |
| 55 | Et | H | $CF_3$ | H | H | $CH_3$ | H | $CF_3$ | Br | H | H | 0 | 0 |
| 56 | Et | H | $CF_3$ | H | H | $CH_3$ | H | $CF_3$ | $CH_3$ | H | H | 0 | 0 |
| 57 | Et | H | $CF_3$ | H | H | $CH_3$ | H | $CF_3$ | $OCH_3$ | H | H | 0 | 0 |
| 58 | Et | H | $CF_3$ | H | H | $CH_3$ | H | $OCF_3$ | H | H | H | 0 | 0 |
| 59 | Et | H | $CF_3$ | H | H | $CH_3$ | H | $OCF_2$ $CF_2H$ | H | H | H | 0 | 0 |
| 60 | Et | H | $CF_3$ | H | H | $CH_3$ | H | Br | $OCF_3$ | H | H | 0 | 0 |
| 61 | Et | H | $CF_3$ | H | H | $CH_3$ | H | H | $SCH_3$ | H | H | 0 | 0 |
| 62 | Et | H | $CF_3$ | H | H | $CH_3$ | H | H | $OCH_3$ | H | H | 0 | 0 |
| 63 | Et | H | $CF_3$ | H | H | $CH_3$ | H | $CF_3$ | $CH_3$ | H | H | 2 | 0 |
| 64 | Et | H | $CF_3$ | H | H | $CH_3$ | H | H | F | H | H | 0 | 0 |
| 65 | Et | H | $CF_3$ | H | H | $CH_3$ | H | H | F | H | H | 2 | 0 |
| 66 | Et | H | $CF_3$ | H | H | $CH_3$ | H | H | CN | H | H | 0 | 0 |

[Table 31]

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 67 | Et | H | $CF_3$ | H | H | $CH_3$ | H | H | CycHex | H | H | 0 | 0 |
| 68 | Et | H | $CF_3$ | H | H | $CH_3$ | H | Cl | $CF_3$ | H | H | 0 | 0 |
| 69 | Et | H | $CF_3$ | H | H | $CH_3$ | H | Cl | $CF_3$ | H | H | 2 | 0 |
| 70 | Et | H | $CF_3$ | H | H | $CH_3$ | H | H | $CF_3$ | H | F | 2 | 0 |
| 71 | Et | H | $CF_3$ | H | H | $CH_3$ | H | F | $CF_3$ | H | F | 2 | 0 |
| 72 | Et | H | $CF_3$ | H | H | $CH_3$ | H | $CF_3$ | F | H | H | 2 | 0 |
| 73 | Et | H | $CF_3$ | H | H | $CH_3$ | H | $CF_3$ | Cl | H | H | 2 | 0 |
| 74 | Et | H | $CF_3$ | H | H | $CH_3$ | H | $CF_3$ | Br | H | H | 2 | 0 |
| 75 | Et | H | $CF_3$ | H | H | $CH_3$ | H | $CF_3$ | $OCH_3$ | H | H | 2 | 0 |
| 76 | Et | H | $CF_3$ | H | H | $CH_3$ | H | $OCF_3$ | H | H | H | 2 | 0 |

(continued)

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 77 | Et | H | $CF_3$ | H | H | $CH_3$ | H | $CCF_2CF_2H$ | H | H | H | 2 | 0 |
| 78 | Et | H | $CF_3$ | H | H | $CH_3$ | H | Br | $OCF_3$ | H | H | 2 | 0 |
| 79 | Et | H | $CF_3$ | H | H | $CH_3$ | H | H | $SO_2CH_3$ | H | H | 2 | 0 |
| 80 | Et | H | $CF_3$ | H | H | $CH_3$ | H | H | $OCH_3$ | H | H | 2 | 0 |
| 81 | Et | H | $CF_3$ | H | H | $CH_3$ | H | H | $OCH_2CH_3$ | H | H | 2 | 0 |
| 82 | Et | H | $CF_3$ | H | H | $CH_3$ | H | H | OPh | H | H | 2 | 0 |
| 83 | Et | H | $CF_3$ | H | H | $CH_3$ | H | H | CN | H | H | 2 | 0 |
| 84 | Et | H | $CF_3$ | H | H | $CH_3$ | H | H | CycHex | H | H | 2 | 0 |
| 85 | Et | H | CF, | H | H | $CH_3$ | H | $CF_3$ | $CF_3$ | H | H | 2 | 0 |
| 86 | Et | H | $CF_3$ | H | H | $CH_3$ | H | F | $CF_3$ | F | H | 2 | 0 |
| 87 | Et | H | $CO_2Et$ | H | H | $CH_3$ | H | H | $SCF_3$ | H | H | 2 | 0 |
| 88 | Et | H | Br | H | H | $CH_3$ | H | H | $SCF_3$ | H | H | 2 | 0 |

[Table 32]

| Compound of Present Invention | $R^1$ | $R^2$ | $R^3$ | $R^4$ | $R^5$ | $R^6$ | $R^7$ | $R^8$ | $R^9$ | $R^{10}$ | $R^{11}$ | n | Q |
|---|---|---|---|---|---|---|---|---|---|---|---|---|---|
| 89 | Et | H | $CF_3$ | H | H | $CH_3$ | H | $CF_3$ | H | H | H | 2 | 0 |
| 90 | Et | H | CN | H | H | $CH_3$ | H | H | $SCF_3$ | H | H | 2 | 0 |
| 91 | Et | H | $CO_2H$ | H | H | $CH_3$ | H | H | $SCF_3$ | H | H | 2 | 0 |
| 92 | Et | H | $CON(CH_3)_2$ | H | H | $CH_3$ | H | H | $SCF_3$ | H | H | 2 | 0 |
| 93 | Et | H | $CONH(CH_3)$ | H | H | $CH_3$ | H | H | $SCF_3$ | H | H | 2 | |
| 94 | Et | H | $CONH_2$ | H | H | $CH_3$ | H | H | $SCF_3$ | H | H | 2 | 0 |
| 95 | Et | H | $NHCO_2CH_3$ | H | H | $CH_3$ | H | H | $SCF_3$ | H | H | 2 | 0 |
| 96 | Et | H | $NHCO_2C(CH_3)_3$ | H | H | $CH_3$ | H | H | $SCF_3$ | H | H | 2 | 0 |
| 97 | Et | H | $NH_2$ | H | H | $CH_3$ | H | H | $SCF_3$ | H | H | 2 | 0 |
| 98 | Et | H | $CF_3$ | H | H | $CH_3$ | H | F | $CF_3$ | H | F | 0 | 0 |
| 99 | Et | H | $CF_3$ | H | H | $CH_3$ | H | $CF_3$ | $CF_3$ | H | H | 0 | 0 |
| 100 | Et | H | $CF_3$ | H | H | $CH_3$ | H | F | $CF_3$ | F | H | 0 | 0 |
| 101 | Et | H | $CF_3$ | H | H | $CH_3$ | H | H | $OCH_2CH_3$ | H | H | 0 | 0 |
| 102 | Et | H | $CF_3$ | H | H | $CH_3$ | H | H | OPh | H | H | 0 | 0 |
| 103 | Et | H | $CO_2Et$ | H | H | $CH_3$ | H | H | $SCF_3$ | H | H | 0 | 0 |

(In [Table 28] to [Table 32] above, Et represents an ethyl group, Pr represents a n-propyl group, CycPr represents a cyclopropyl group, CycHex represents a cyclohexyl group, and Ph represents a phenyl group.)

$^1$H-NMR data of the compound of the present invention shown in [Table 28] to [Table 32] are shown below.

Compound of Present Invention 6

$^1$H-NMR($CDCl_3$)δ: 7.65-6.94(6H, m), 3.42(3H, brs), 3.01(2H, q), 1.35(3H, t).

Compound of Present Invention 7

$^1$H-NMR($CDCl_3$)δ: 7.54-7.44(1H, m), 7.40-7.11(2H, m), 6.91-6.72(2H, m), 6.69-6.57(1H, m), 3.42(3H, brs), 3.01(2H, q), 1.34(3H, t).

Compound of Present Invention 8

$^1$H-NMR($CDCl_3$)δ: 8.38-7.09(7H, m), 3.70-3.18(5H, m), 1.39-1.28(3H, m).

Compound of Present Invention 9

$^1$H-NMR($CDCl_3$)δ: 8.38-7.03(7H, m), 3.72-3.15(5H, m), 1.38-1.29(3H, m).

Compound of Present Invention 10

$^1$H-NMR($CDCl_3$)δ: 8.37-7.10(7H, m), 3.72-3.17(5H, m), 1.39-1.29(3H, m).

Compound of Present Invention 11

$^1$H-NMR($CDCl_3$)δ: 8.48-6.95(7H, m), 3.76-3.10(5H, m), 1.47-1.30(3H, m).

Compound of Present Invention 12

$^1$H-NMR($CDCl_3$)δ: 8.39-7.08(7H, m), 3.71-3.19(5H, m), 1.39-1.29(3H, m).

Compound of Present Invention 13

$^1$H-NMR($CDCl_3$)δ: 8.38-6.59(6H, m), 3.67-3.10(5H, m), 1.40-1.25(3H, m).

Compound of Present Invention 14

$^1$H-NMR($CDCl_3$)δ: 8.38-7.13(6H, m), 3.68-3.11(5H, m), 1.40-1.29(3H, m).

Compound of Present Invention 15

$^1$H-NMR($CDCl_3$)δ: 8.38-7.13(6H, m), 3.68-3.11(5H, m), 1.40-1.29(3H, m).
Compounds represented by the formula (M6).

Compound of Present Invention 18

$^1$H-NMR($CDCl_3$)δ: B.69-6.79(8H, m), 3.66-2.41(5H, m), 1.45-1.03(3H, m).

Compound of Present Invention 19

$^1$H-NMR($CDCl_3$)δ: 7.80-6.98(8H, m), 3.54-2.48(5H, m), 1.34-1.22(3H, m).

Compound of Present Invention 25

$^1$H-NMR($CDCl_3$)δ: 8.48-6.95(8H, m), 3.75-3.16(5H, m), 1.38-1.26(3H, m).

Compound of Present Invention 26

$^1$H-NMR($CDCl_3$)δ: 8.15-6.94(8H, m), 3.71-3.17(5H, m), 1.39-1.29(3H, m).

Compound of Present Invention 44

$^1$H-NMR($CDCl_3$)δ: 7.94-6.93(7H, m), 3.48(3H, s), 3.04-2.93(2H, m), 1.36-1.29(3H, m).

Compound of Present Invention 45

$^1$H-NMR($CDCl_3$)δ: 8.35-7.14(7H, m), 3.72-3.14(5H, m), 1.38-1.26(3H, m).

Compound of Present Invention 46

$^1$H-NMR($CDCl_3$)δ: 7.47-6.89(7H, m), 3.53-3.11(3H, m), 2.98(2H, q), 2.41-2.19(3H, m), 1.34(3H, t).

Compound of Present Invention 47

$^1$H-NMR($CDCl_3$)δ: B.39-6.90(7H, m), 3.71-3.10(5H, m), 2.46-2.17(3H, m), 1.34(3H, t).

Compound of Present Invention 48

$^1$H-NMR($CDCl_3$)δ: 8.09-6.86(7H, m), 4.42-3.40(3H, m), 3.26-2.86(2H, m), 2.77-2.43(2H, m), 1.40-1.26(3H, m), 1.26-1.06(3H, m).

Compound of Present Invention 49

$^1$H-NMR($CDCl_3$)δ: 8.37-8.14(1H, m), 8.08-6.92(6H, m), 4.45-3.14(5H, m), 2.73-2.48(2H, m), 1.40-1.08(6H, m).

Compound of Present Invention 50

$^1$H-NMR($CDCl_3$)δ: 7.67-6.94(7H, m), 4.42-2.72(6H, m), 1.37-1.07(9H, m).

Compound of Present Invention 51

$^1$H-NMR($CDCl_3$)δ: 8.35-8.16(1H, m), 8.02-7.02(6H, m), 4.40-3.16(5H, m), 2.99-2.73(1H, m), 1.37-1.11(9H, m).

Compound of Present Invention 52

$^1$H-NMR($CDCl_3$)δ: 7.81-7.06(6H, m), 3.62-2.86(5H, m), 1.34(3H, t).

Compound of Present Invention 53

$^1$H-NMR($CDCl_3$)δ: 7.83-6.82(6H, m), 3.69-2.81(5H, m), 1.33(3H, t).

Compound of Present Invention 54

$^1$H-NMR($CDCl_3$)δ: 7.74-7.00(6H, m), 3.46(3H, s), 2.98(2H, d), 1.33(3H, t).

Compound of Present Invention 55

$^1$H-NMR($CDCl_3$)δ: 7.71-6.97(6H, m), 3.46(3H, s), 2.97(2H, q), 1.32(3H, t).

Compound of Present Invention 56

$^1$H-NMR($CDCl_3$)δ: 7.77-6.92(6H, m), 3.60-2.88(5H, m), 2.38(3H, s), 1.33(3H, t).

Compound of Present Invention 57

$^1$H-NMR($CDCl_3$)δ: 7.71-6.72(6H, m), 3.98-2.89(8H, m), 1.33(3H, t).

Compound of Present Invention 58

$^1$H-NMR($CDCl_3$)δ: 7.48-6.92(7H, m), 3.50(3H, s), 2.98(2H, q), 1.33(3H, t).

Compound of Present Invention 59

$^1$H-NMR($CDCl_3$)δ: 7.64-6.89(7H, m), 5.83(1H, t), 3.50(3H, s), 2.97(2H, q), 1.33(3H, t).

Compound of Present Invention 60

$^1$H-NMR($CDCl_3$)δ: 7.73-6.96(6H, m), 3.44(3H, a), 2.99(2H, q), 1.34(3H, t).

Compound of Present Invention 61

$^1$H-NMR($CDCl_3$)δ: 7.47-6.95(7H, m), 3.47(3H, s), 2.98(2H, q), 2.40(3H, s), 1.33(3H, t).

Compound of Present Invention 62

$^1$H-NMR($CDCl_3$)δ: 7.40(1H, s), 7.20(1H, d), 7.13(1H, d), 7.07(2H, d), 6.68(2H, d), 3.71(3H, s), 3.46(3H, s), 2.98(2H, q), 1.33(3H, t).

Compound of Present Invention 63

$^1$H-NMR(DMSO-$D_6$)δ: 8.31-7.26(6H, m), 3.61-3.35(5H, m), 2.30(3H, s), 1.22-1.16(3H, m).

Compound of Present Invention 64

$^1$H-NMR($CDCl_3$)δ: 7.71-6.74(7H, m), 3.55-2.89(5H, m), 1.33(3H, t).

Compound of Present Invention 65

$^1$H-NMR($CDCl_3$)δ: 8.36-6.85(7H, m), 3.69-3.13(5H, m), 1.38-1.29(3H, m).

Compound of Present Invention 66

$^1$H-NMR($CDCl_3$)δ: 7.55(2H, d), 7.47(1H, s), 7.37-7.19(4H, m), 3.46(3H, s), 2.98(2H, q), 1.33(3H, t).

Compound of Present Invention 67

$^1$H-NMR($CDCl_3$)δ: 7.67-6.84(7H, m), 3.55-2.89(5H, m), 2.44-2.32(1H, m), 1.90-1.08(8H, m).

Compound of Present Invention 68

$^1$H-NMR($CDCl_3$)δ: 7.56(1H, d), 7.50(1H, s), 7.43(1H, s), 7.37(1H, d), 7.30-7.24(1H, m), 7.22-7.15(1H, m), 3.43(3H, s), 3.00(2H, q), 1.34(3H, t).

Compound of Present Invention 69

$^1$H-NMR($CDCl_3$)δ: 8.37-7.16(6H, m), 3.67-3.16(5H, m), 1.39-1.29(3H, m).

Compound of Present Invention 70

$^1$H-NMR($CDCl_3$)δ: 8.40-7.19(6H, m), 3.70-3.13(5H, m), 1.38-1.29(3H, m).

Compound of Present Invention 71

$^1$H-NMR($CDCl_3$)δ; 8.37-8.19(1H, m), 8.08-7.30(4H, m), 3.61-3.12(5H, m), 1.42-1.29(3H, m).

Compound of Present Invention 72

$^1$H-NMR($CDCl_3$)δ: 8.37-6.99(6H, m), 3.64-3.16(5H, m), 1.40-1.29(3H, m).

Compound of Present Invention 73

$^1$H-NMR($CDCl_3$)δ: 8.36-8.20(1H, m), 8.05-7.15(5H, m), 3.64-3.17(5H, m), 1.38-1.29(3H, m).

Compound of Present Invention 74

$^1$H-NMR($CDCl_3$)δ: 8.36-8.20(1H, m), 8.06-7.15(5H, m), 3.64-3.17(5H, m), 1.39-1.29(3H, m).

Compound of Present Invention 75

$^1$H-NMR(DMSO-$D_6$)δ: 8.25-7.09(6H, m), 3.96-3.36(8H, m), 1.21-1.14(3H, m).

Compound of Present Invention 76

$^1$H-NMR($CDCl_3$)δ: 8.38-8.18(1H, m), 8.06-6.95(6H, m), 3.69-3.17(5H, m), 1.38-1.29(3H, m).

Compound of Present Invention 77

$^1$H-NMR($CDCl_3$)δ: 8.37-8.16(1H, m), 8.12-6.92(6H, m), 6.09-5.67(1H, m), 3.67-3.15(5H, m), 1.38-1.29(3H, m).

Compound of Present Invention 78

$^1$H-NMR($CDCl_3$)δ: 8.38-8.19(1H, m), 8.11-7.06(5H, m), 3.65-3.14(5H, m), 1.38-1.29(3H, m).

Compound of Present Invention 80

$^1$H-NMR($CDCl_3$)δ: 8.35-6.54(7H, m), 3.86-3.12(8H, m), 1.38-1.28(3H, m).

Compound of Present Invention 81

$^1$H-NMR($CDCl_3$)δ: 8.36-6.53(7H, m), 4.07-3.13(7H, m), 1.44-1.28(6H, m).

Compound of Present Invention 82

$^1$H-NMR($CDCl_3$)δ: 8.35-6.78(12H, m), 3.67-3.15(5H, m), 1.37-1.28(3H, m).

Compound of Present Invention 83

$^1$H-NMR($CDCl_3$)δ: 8.37-7.09(7H, m), 3.67-3.19(5H, m), 1.39-1.29(3H, m).

Compound of Present Invention 84

$^1$H-NMR($CDCl_3$)δ: 8.36-7.00(7H, m), 3.68-3.14(5H, m), 2.56-2.33(1H, m), 1.93-1.14(8H, m).

Compound of Present Invention 85

$^1$H-NMR($CDCl_3$)δ: 8.37-7.12(6H, m), 4.03-3.21(5H, m), 1.43-1.28(3H, m).

Compound of Present Invention 86

$^1$H-NMR($CDCl_3$)δ: 8.36-7.05(5H, m), 4.03-3.17(5H, m), 1.41-1.30(3H, m).

Compound of Present Invention 89

$^1$H-NMR($CDCl_3$)δ: 8.38-7.10(7H, m), 3.68-3.18(5H, m), 1.38-1.28(3H, m).

Compound of Present Invention 90

$^1$H-NMR($CDCl_3$)δ: 8.41-7.04(7H, m), 3.74-3.14(5H, m), 1.37-1.28(3H, m).

Compound of Present Invention 92

$^1$H-NMR($CDCl_3$)δ: 8.12-7.97(1H, m), 7.87-7.02(6H, m), 3.72-2.73(11H, m), 1.36-1.29(3H, m).

Compound of Present Invention 93

$^1$H-NMR($CDCl_3$)δ: 8.40-7.83(2H, m), 7.77-7.08(5H, m), 6.70-6.42(1H, m), 3.71-3.17(5H, m), 3.09-2.96(3H, m), 1.35-1.28(3H, m).

Compound of Present Invention 95

$^1$H-NMR($CDCl_3$)δ: 7.90-6.79(7H, m), 3.86-3.16(8H, m), 1.37-1.28(3H, m).

Compound of Present Invention 96

$^1$H-NMR($CDCl_3$)δ: 7.89-6.61(7H, m), 3.69-3.17(5H, m), 1.56-1.46(9H, m), 1.36-1.28(3H, m).

Compound of Present Invention 98

$^1$H-NMR($CDCl_3$)δ: 7.64-7.14(5H, m), 3.38(3H, s), 3.02(2H, q), 1.35(3H, t).

Compound of Present Invention 99

$^1$H-NMR($CDCl_3$)δ: 8.06-7.27(6H, m), 3.47(3H, s), 2.98(2H, q), 1.32(3H, t).

Compound of Present Invention 100

$^1$H-NMR($CDCl_3$)δ: 8.08-6.92(5H, m), 3.39(3H, a), 3.02(3H, q), 1.34(3H, t).

Compound of Present Invention 101

$^1$H-NMR($CDCl_3$)δ: 7.39(1H, a), 7.20(1H, d), 7.13(1H, d), 7.05(2H, d), 6.67(2H, d), 3.92(2H, q), 3.46(3H, s), 2.97(2H, q), 1.38-1.30(6H, m).

Compound of Present Invention 102

$^1$H-NMR($CDCl_3$)δ: 7.52-6.64(12H, m), 3.55-3.13(3H, m), 2.98(2H, q), 1.32(3H, t).

Compound of Present Invention 103

$^1$H-NMR($CDCl_3$)δ: 7.94-7.09(7H, m), 4.35(2H, q), 3.55-3.42(3H, m), 2.98(2H, q), 1.37(3H, t), 1.31(3H, t).

Compounds represented by the formula (M6) (reference):
(All compounds M6 are reference compounds due to $R^6$ being H)

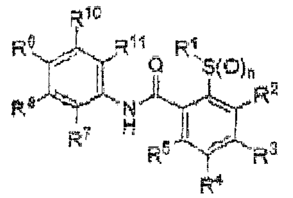

(M 6)

wherein $R^1$, $R^2$, $R^3$, $R^4$, $R^5$, $R^7$, $R^8$, $R^9$, $R^{10}$, $R^{11}$, n and Q represent the combinations shown in [Table 33] to [Table 35] shown below.

[Table 33]

| Intermediate Compound | $R^1$ | $R^2$ | $R^3$ | $R^4$ | $R^5$ | $R^7$ | $R^8$ | $R^9$ | $R^{10}$ | $R^{11}$ | n | Q |
|---|---|---|---|---|---|---|---|---|---|---|---|---|
| M6-1 | Et | H | $CF_3$ | H | H | H | H | $CF_3$ | H | H | 0 | 0 |
| M6-2 | Et | H | $CF_3$ | H | H | H | H | $OCF_2$ | H | H | 0 | 0 |
| M6-3 | Et | H | $CF_3$ | H | H | H | H | $CF_2CF_3$ | H | H | 0 | 0 |
| M6-4 | Et | H | $CF_3$ | H | H | H | H | $SCF_3$ | H | H | 0 | 0 |
| M6-5 | Et | H | $CF_3$ | H | H | H | $CF_3$ | H | $CF_3$ | H | 0 | 0 |
| M6-6 | Et | H | $CF_3$ | H | H | H | Cl | H | Cl | H | 0 | 0 |
| M6-7 | Et | H | $CF_3$ | H | H | H | F | H | F | H | 0 | 0 |
| M6-8 | Et | H | $C F_3$ | H | H | H | H | $CF_3$ | H | H | 2 | 0 |
| M6-9 | Et | H | $CF_3$ | H | H | H | H | $OCF_3$ | H | H | 2 | 0 |
| M6-10 | Et | H | $CF_3$ | H | H | H | H | $CF_2CF_3$ | H | H | 2 | 0 |
| M6-11 | Et | H | $CF_3$ | H | H | H | H | $SCF_3$ | H | H | 2 | 0 |
| M6-12 | Et | H | $CF_3$ | H | H | H | $CF_3$ | H | $CF_3$ | H | 2 | 0 |
| M6-13 | Et | H | $CF_3$ | H | H | H | Cl | H | Cl | H | 2 | 0 |
| M6-14 | ET | H | $CF_3$ | H | H | H | F | H | F | H | 2 | 0 |
| M6-15 | Et | H | F | H | H | H | H | $CF_2CF_3$ | H | H | 2 | 0 |
| M6-16 | Et | H | Cl | H | H | H | H | $CF_2CF_3$ | H | H | 2 | 0 |
| M6-17 | Et | H | Br | H | H | H | H | $CF_2CF_3$ | H | H | 2 | 0 |
| M6-18 | Et | H | 1 | H | H | H | H | $CF_2CF_3$ | H | H | 2 | 0 |
| M6-19 | Et | H | 2-pyridyl | H | H | H | H | $CF_2CF_3$ | H | H | 2 | 0 |
| M6-20 | Et | H | 2-pyrimidyl | H | H | H | H | $CF_2CF_3$ | H | H | 2 | 0 |
| M6-21 | Et | H | $CH_3$ | H | H | H | H | $CF_2CF_3$ | H | H | 2 | 0 |
| M6-22 | Et | H | $OCH_3$ | H | H | H | H | $CF_2CF_3$ | H | H | 2 | 0 |

[Table 34]

| Intermediate Compound | $R^1$ | $R^2$ | $R^3$ | $R^4$ | $R^5$ | $R^7$ | $R^6$ | $R^9$ | $R^{10}$ | $R^{11}$ | n | Q |
|---|---|---|---|---|---|---|---|---|---|---|---|---|
| M6-23 | Et | H | H | H | H | H | H | $SCF_3$ | H | H | 0 | 0 |
| M6-24 | Et | H | H | H | H | H | H | $CF_2CF_3$ | H | H | 0 | 0 |
| M6-25 | Et | H | $CF_3$ | H | H | H | H | Br | H | H | 0 | 0 |
| M6-26 | Et | H | $CF_3$ | H | H | H | H | $CH_3$ | H | H | 0 | 0 |
| M6-27 | Et | H | $CF_3$ | H | H | H | H | $CH_2CH_3$ | H | H | 0 | 0 |

(continued)

| Intermediate Compound | $R^1$ | $R^2$ | $R^3$ | $R^4$ | $R^5$ | $R^7$ | $R^6$ | $R^9$ | $R^{10}$ | $R^{11}$ | n | Q |
|---|---|---|---|---|---|---|---|---|---|---|---|---|
| M6-28 | Et | H | $CF_3$ | H | H | H | H | $CH(CH_3)_2$ | H | H | 0 | 0 |
| M6-29 | Et | H | $CF_3$ | H | H | H | H | F | H | H | 0 | 0 |
| M6-30 | Et | H | $CF_3$ | H | H | H | $CF_3$ | H | H | H | 2 | 0 |
| M6-31 | Et | H | $CF_3$ | H | H | H | Cl | $CF_2$ | H | H | 0 | 0 |
| M6-32 | Et | H | $CF_3$ | H | H | H | H | $CF_3$ | H | F | 0 | 0 |
| M6-33 | Et | H | $CF_3$ | H | H | H | F | $CF_3$ | H | F | 0 | 0 |
| M6-34 | Et | H | $CF_3$ | H | H | H | $CF_3$ | F | H | H | 0 | 0 |
| M6-35 | Et | H | $CF_3$ | H | H | H | $CF_3$ | Cl | H | H | 0 | 0 |
| M6-36 | Et | H | $CF_3$ | H | H | H | $CF_3$ | Br | H | H | 0 | 0 |
| M6-37 | Et | H | $CF_3$ | H | H | H | $CF_3$ | $CH_3$ | H | H | 0 | 0 |
| M6-38 | Et | H | $CF_3$ | H | H | H | $CF_3$ | $OCH_3$ | H | H | 0 | 0 |
| M6-39 | Et | H | $CF_3$ | H | H | H | $OCF_3$ | H | H | H | 0 | 0 |
| M6-40 | Et | H | $CF_3$ | H | H | H | $OCF_3CF_2H$ | H | H | H | 0 | 0 |
| M6-41 | Et | H | $CF_3$ | H | H | H | Br | $OCF_3$ | H | H | 0 | 0 |
| M6-42 | Et | H | $CF_3$ | H | H | H | $CF_3$ | $CF_3$ | H | H | 0 | 0 |
| M6-43 | Et | H | $CF_3$ | H | H | H | F | $CF_3$ | F | H | 0 | 0 |
| M6-44 | Et | H | $CF_3$ | H | H | H | H | $SCH_3$ | H | H | 0 | 0 |

[Table 35]

| Intermediate compound | $R^1$ | $R^1$ | $R^3$ | $R^4$ | $R^5$ | $R^7$ | $R^8$ | $R^9$ | $R^{10}$ | $R^{11}$ | n | Q |
|---|---|---|---|---|---|---|---|---|---|---|---|---|
| M6-45 | Et | H | $CF_3$ | H | H | H | H | $OCH_3$ | H | H | 0 | 0 |
| M6-46 | Et | H | $CF_3$ | H | H | H | H | $OCH_2CH_3$ | H | H | 0 | 0 |
| M6-47 | Et | H | $CF_3$ | H | H | H | H | OPh | H | H | 0 | 0 |
| M6-48 | Et | H | $CF_3$ | H | H | H | H | CN | H | H | 0 | 0 |
| M6-49 | Et | H | $CF_3$ | H | H | H | H | CycHex | H | H | 0 | 0 |

(In [Table 33] to [Table 35] above, Et represents an ethyl group, CycHex represents a cyclohexyl group, and Ph represents a phenyl group.)

$^1$H-NMR data of the intermediate compound of the present invention shown in [Table 33] to [Table 35] are shown below.

Intermediate Compound M6-6

$^1$H-NMR($CDCl_3$)δ: 8.70(1H, s), 7.91(1H, d), 7.70(1H, s), 7.64(2H, d), 7.59(1H, d), 7.20-7.17(1H, m), 3.01(2H, q), 1.34(3H, t).

Intermediate Compound M6-7

$^1$H-NMR($CDCl_3$)δ: 8.73(1H, s), 7.91(1H, d), 7.69(1H, s), 7.58(1H, d), 7.33-7.22(2H, m), 6.69-6.58(1H, m), 3.01(2H, q), 1.34(3H, t).

Intermediate Compound M6-8

$^1$H-NMR($CDCl_3$)δ: 9.04(1H, s), 8.01(1H, d), 7.92(1H, s), 7.76(1H, d), 7.58(2H, d), 7.38(2H, d), 3.54(2H, q), 1.29(3H, t).

Intermediate Compound M6-9

$^1$H-NMR($CDCl_3$)δ: 8.68(1H, brs), 8.06-7.97(2H, m), 7.77(1H, d), 7.53(2H, d), 7.06(2H, d), 3.52(2H, q), 1.29(3H, t).

Intermediate Compound M6-10

$^1$H-NMR($CDCl_3$)δ: 9.09(1H, brs), 8.01(1H, d), 7.91(1H, s), 7.75(1H, d), 7.62(2H, d), 7.37(2H, d), 3.54(2H, q), 1.29(3H, t).

Intermediate Compound M6-11

$^1$H-NMR($CDCl_3$)δ: 9.09(1H, a), 8.01(1H, d), 7.91(1H, s), 7.75(1H, d), 7.62(2H, d), 7.37(2H, d), 3.54(2H, q), 1.29(3H, t).

Intermediate Compound M6-12

$^1$H-NMR($CDCl_3$)δ: 9.51(1H, s), 8.02(1H, d), 7.97(2H, s), 7.85(1H, s), 7.74(1H, d), 7.43(1H, s), 3.60(2H, q), 1.32(3H, t).

Intermediate Compound M6-13

$^1$H-NMR($CDCl_3$)δ: 9.17(1H, s), 8.00(1H, d), 7.79(1H, a), 7.68(1H, d), 7.43(2H, d), 6.97(1H, dd), 3.55(2H, q), 1.28(3H, q).

Intermediate Compound M6-14

$^1$H-NMR($CDCl_3$)δ; 9.16(1H, s), 8.00(1H, d), 7.80(1H, s), 7.70(1H, d), 7.08(2H, dd), 6.48-6.36(1H, m), 3.53(2H, q), 1.27(3H, t).

Intermediate Compound M6-23

$^1$H-NMR($CDCl_3$)δ: 9.11(1H, s), H, dd), 7.76(2H, dd), 7.66(2H, d), 7.51(1H, dd), 7.45(1H, td), 7.37(1H, td), 2.95(2H, q), 1.29(3H, t).

Intermediate Compound M6-24

$^1$H-NMR($CDCl_3$)δ: 9.15(1H, s), 7.89(1H, dd), 7.83(2H, d), 7.60(2H, d), 7.51(1H, dd), 7.45(1H, td), 7.37(1H, td), 2.95(2H, q), 1.29(3H, t).

Intermediate Compound M6-25

$^1$H-NMR($CDCl_3$)δ: 8.56(1H, s), 7.90(1H, d), 7.68(1H, s), 7.60-7.55(3H, m), 7.50(2H, d), 3.00(2H, q), 1.33(3H, t).

Intermediate Compound M6-26

$^1$H-NMR($CDCl_3$)δ: 8.43(1H, a), 7.88(1H, d), 7.66(1H, s), 7.58-7.51(3H, m), 7.19(2H, d), 3.00(2H, q), 2.35(3H, s), 1.32(3H, t).

Intermediate Compound M6-27

$^1$H-NMR($CDCl_3$)δ; 8.44(1H, a), 7.86(1H, d), 7.66(1H, s), 7.59-7.51(3H, m), 7.21(2H, d), 2.99(2H, q), 2.65(2H, q), 1.32(3H, t), 1.24(3H, t).

Intermediate Compound M6-28

$^1$H-NMR($CDCl_3$)δ: 8.41(1H, s), 7.88(1H, d), 7.66(1H, s), 7.59-7.53(3H, m), 7.26-7.22(2H, m), 3.00(2H, q), 2.96-2.88(1H, m), 1.32(3H, t), 1.26(6H, d).

Intermediate Compound M6-29

$^1$H-NMR($CDCl_3$)δ: 8.49(1H, s), 7.89(1H, d), 7.67(1H, a), 7.63(2H, dd), 7.56(1H, d), 7.09(2H, dd), 3.01(2H, q), 1.33(3H, t).

Intermediate Compound M6-30

$^1$H-NMR($CDCl_3$)δ: 8.83(1H, s), 8.03-7.94(3H, m), 7.76(1H, d), 7.50(1H, d), 7.27-7.19(2H, m), 3.55(2H, q), 1.30(3H, t).

Intermediate Compound M6-31

$^1$H-NMR($CDCl_3$)δ: 8.89(1H, s), 7.95(1H, s), 7.89(1H, d), 7.70-7.66(2H, m), 7.61(1H, d), 7.56(1H, d), 3.01(2H, q), 1.33(3H, t).

Intermediate Compound M6-32

$^1$H-NMR($CDCl_3$)δ: 9.16(1H, s), B.70(1H, dd), 7.97(1H, d), 7.72(1H, a), 7.60(1H, d), 7.49(1H, d), 7.42(1H, d), 3.03(2H, q), 1.35(3H, t).

Intermediate Compound M6-33

$^1$H-NMR($CDCl_3$)δ: 9.36(1H, s), 8.57(1H, dd), 7.99(1H, d), 7.73(1H, a), 7.61(1H, d), 7.39(1H, dd), 3.03(2H, q), 1.35(3H, t).

Intermediate Compound M6-34

$^1$H-NMR($CDCl_3$)δ: 8.70(1H, s), 7.92(2H, d), 7.87(1H, d), 7.69(1H, s), 7.58(1H, d), 7.22(1H, d), 3.02(2H, q), 1.34(3H, t).

Intermediate Compound M6-35

$^1$H-NMR($CDCl_3$)δ: 8.77(1H, s), 7.98(1H, a), 7.92(1H, d), 7.87(1H, d), 7.70(1H, s), 7.59(1H, d), 7.52(1H, d), 3.02(2H, q), 1.34(3H, t).

Intermediate Compound M6-36

$^1$H-NMR($CDCl_3$)δ: 8.78(1H, a), 7.98(1H, a), 7.92(1H, d), 7.80(1H, d), 7.74-7.68(2H, m), 7.59(1H, d), 3.01(2H, q), 1.34(3H, t).

Intermediate Compound M6-37

$^1$H-NMR($CDCl_3$)δ: 8.62(1H, s), 7.89(1H, d), 7.84(1H, s), 7.79(1H, d), 7.68(1H, s), 7.56(1H, d), 7.30(1H, d), 3.01(2H, q), 2.48(3H, brs), 1.33(3H, t).

Intermediate Compound M6-38

$^1$H-NMR($CDCl_3$)δ: 8.54(1H, s), 7.93-7.87(2H, m), 7.76(1H, d), 7.67(1H, s), 7.56(1H, d), 7.03(1H, d), 3.92(3H, s), 3.01(2H, q), 1.34(3H, t).

Intermediate Compound M6-39

$^1$H-NMR($CDCl_3$)δ: 8.68(1H, a), 7.92(1H, d), 7.69(2H, s), 7.58(1H, d), 7.51(1H, d), 7.40(1H, dd), 7.05(1H, d),

3.01(2H, q), 1.34(3H, t).

Intermediate Compound M6-40

$^1$H-NMR($CDCl_3$)δ: 8.67(1H, s), 7.90(1H, d), 7.68(2H, s), 7.57(1H, d), 7.50(1H, d), 7.38(1H, dd), 7.06(1H, d), 5.92(1H, t), 3.01(2H, q), 1.33(3H, t).

Intermediate Compound M6-41

$^1$H-NMR(DMSO-$D_6$)δ: 10.84(1H, a), 8.24(1H, d), 7.76-7.72(3H, m), 7.67(1H, d), 7.56(1H, dd), 3.08(2H, q), 1.22(3H, q).

Intermediate Compound M6-42

$^1$H-NMR($CDCl_3$)δ: 9.01(1H, s), 8.12-8.05(2H, m), 7.94(1H, d), 7.87(1H, d), 7.71(1H, s), 7.60(1H, d), 3.02(2H, q), 1.34(3H, t).

Intermediate Compound M6-43

$^1$H-NMR(DMSO-$D_6$)δ: 11.18(1H, a), 7.78-7.72(2H, m), 7.68-7.60(3H, m), 3.08(2H, q), 1.24(3H, t).

Intermediate Compound M6-44

$^1$H-NMR($CDCl_3$)δ: 8.51(1H, s), 7.89(1H, d), 7.67(1H, s), 7.60(2H, d), 7.55(1H, d), 7.29(2H, d), 3.00(2H, q), 2.49(3H, a), 1.32(3H, t).

Intermediate Compound M6-45

$^1$H-NMR($CDCl_3$)δ: 8.38(1H, a), 7.89(1H, d), 7.67(1H, s), 7.59-7.53(3H, m), 6.92(2H, d), 3.82(3H, s), 3.00(2H, q), 1.33(3H, t).

Intermediate Compound M6-46

$^1$H-NMR($CDCl_3$)δ: 8.37(1H, s), 7.88(1H, d), 7.67(1H, s), 7.58-7.52(3H, m), 6.91(2H, d), 4.04(2H, q), 3.00(2H, q), 1.42(3H, t), 1.33(3H, t).

Intermediate Compound M6-47

$^1$H-NMR($CDCl_3$)δ: 8.47(1H, s), 7.90(1H, d), 7.68(1H, a), 7.62(2H, d), 7.56(1H, d), 7.37-7.31(2H, m), 7.13-7.08(1H, m), 7.07-6.99(4H, m), 3.02(2H, q), 1.34(3H, t).

Intermediate Compound M6-48

$^1$H-NMR($CDCl_3$)δ: 8.87(1H, a), 7.93(1H, d), 7.80(2H, d), 7.71-7.66(3H, m), 7.59(1H, d), 3.01(2H, q), 1.34(3H, t).

Intermediate Compound M6-49

$^1$H-NMR($CDCl_3$)δ: 8.40(1H, s), 7.88(1H, d), 7.67(1H, s), 7.59-7.52(3H, m), 7.23(2H, d), 3.00(2H, q), 2.55-2.45(1H, m), 1.91-1.71(5H, m), 1.48-1.20(8H, m).

Next, formulation examples of the compound of the present invention are shown. These formulation examples are according to the invention in as far as at least one of the Compounds of Present Invention 1 to 38 or 44 to 103 are used. The part means part by weight.

Formulation Example 1

10 parts of any one of Compounds of Present Invention 1 to 103 and Intermediate Compounds M6-1 to M6-49 are dissolved in a mixture of 35 parts of xylene and 35 parts of DMF, and 14 parts of polyoxyethylenestyrylphenyl ether and 6 parts of calcium dodecylbenzenesulfonate are added thereto. The mixture is mixed to obtain each emulsifiable

concentrate. Formulation Example 2

4 parts cf sodium lauryl sulfate, 2 parts of calcium lignosulfonste, 20 parts of synthetic hydrous silicon oxide fine powder and 54 parts of diatomaceous earth are mixed, and 20 parts of any one of Compounds of Present Invention 1 to 103 and Intermediate Compounds M6-1 to M6-49 are further added thereto. The mixture is mixed to obtain each wettable powder. Formulation Example 3

1 part of synthetic hydrous silicon oxide fine powder, 2 parts of calcium lignosulfonate, 30 parts of bentonite and 65 parts of kaolin clay are added to 2 parts of any one of Compounds of Present Invention 1 to 103 and Intermediate Compounds M6-1 to M6-49. Subsequently, an appropriate amount of water is added to this mixture, and the mixture is further stirred, granulated with a granulator, and forced-air dried to obtain each granule.

Formulation Example 4

1 part of any one of Compounds of Present Invention 1 to 103 and Intermediate Compounds M6-1 to M6-49 is dissolved in an appropriate amount of acetone, and 5 parts of synthetic hydrous silicon oxide fine powder, 0.3 parts of PAP and 93.7 parts of Fubasami clay are added thereto. The mixture is sufficiently stirred and mixed to evaporate and eliminate acetone to obtain each dust formulation.

Formulation Example 5

35 parts of a mixture of polyoxyethylene alkyl ether sulfate ammonium salt and white carbon (weight ratio 1 : 1), 10 parts of any one of Compounds of Present Invention 1 to 103 and Intermediate Compounds M6-1 to M6-49 and 55 parts of water are mixed, and finely pulverized by wet grinding method to obtain each flowable.

Formulation Example 6

0.1 parts of any one of Compounds of Present Invention 1 to 103 and Intermediate Compounds M6-1 to M6-49 are dissolved in 5 parts of xylene and 5 parts of trichloroethane, and the mixture is mixed with 89.9 parts of deodorized kerosene to obtain each oil solution.

Formulation Example 7

10 mg of any one of Compounds of Present Invention 1 to 103 and Intermediate Compounds M6-1 to M6-49 is dissolved in 0.5 ml of acetone, and this solution is applied to 5 g of solid feed powder for animal (solid feed powder for breeding CE-2, product of CLEA Japan, Inc.), and the mixture is uniformly mixed. Subsequently, acetone is evaporated to dryness to obtain each poisonous bait.

Formulation Example 8

An aerosol can is filled with 0.1 parts of any one of Compounds of Present Invention 1 to 103 and Intermediate Compounds M6-1 to M6-49 and 49.9 parts of Neothiazol (Chuo Kasei Co., Ltd.), and an aerosol valve is attached, and then the container is filled with 25 parts of dimethyl ether and 25 parts of LPG and shaken, and an actuator is attached to obtain an oil-based aerosol.

Formulation Example 9

0.6 parts of any one of Compounds of Present Invention 1 to 103 and Intermediate Compounds M6-1 to M6-49, 0.01 parts of BHT (2,6-di-tert-butyl-4-methylphenol), 5 parts of xylene, 3.39 parts of deodorized kerosene and 1 part of emulsifier {Atmos 300 (registered trade name for Atmos Chemical Ltd.)} are mixed and dissolved, and an aerosol container is filled with the resulting solution and 50 parts of distilled water. A valve is attached to the container, and then the container is filled with 40 parts of a propellant (LPG) under pressure through the valve to obtain an aqueous aerosol.

Formulation Example 10

0.1 g of any one of Compounds of Present Invention 1 to 103 and Intermediate Compounds M6-1 to M6-49 are dissolved in 2 ml of propylene glycol, and the solution is impregnated in a porous ceramic plate with a size of 4.0 cm × 4.0 cm and 1.2 cm in thickness to obtain a heating type smoking agent.

Formulation Example 11

5 parts of any one of Compounds of Present Invention 1 to 103 and Intermediate Compounds M6-1 to M6-49 and 95 parts of an ethylene-methyl methacrylate copolymer (a ratio of methyl methacrylate in the copolymer: 10% by weight, Acryft WD301, manufactured by SUMITOMO CHEMICAL Co., Ltd.) are melt-kneaded with a closed pressurizing kneader (manufactured by Moriyama Works), and the resulting kneaded matter is extruded from an extrusion molding machine through a molding die to obtain a rod-shaped molded body with a size of 15 cm in length and 3 mm in diameter.

Formulation Example 12

5 parts of any one of Compounds of Present Invention 1 to 103 and Intermediate Compounds M6-1 to M6-49 and 95 parts of a soft vinyl chloride resin are melt-kneaded with a closed pressurizing kneader (manufactured by Moriyama Works), and the resulting kneaded matter is extruded from an extrusion molding machine through a molding die to obtain a rod-shaped molded body with a size of 15 cm in length and 3 mm in diameter.

Formulation Example 13

100 mg of any one of Compounds of Present Invention 1 to 103 and Intermediate Compounds M6-1 to M6-49, 68.75 mg of lactose, 237.5 mg of corn starch, 43.75 mg of microcrystalline cellulose, 18.75 mg of polyvinylpyrrolidone, 28.75 mg of sodium carboxymethyl starch and 2.5 mg of magnesium stearate are mixed, and the resulting mixture is compressed to an appropriate size to obtain a tablet.

Formulation Example 14

25 mg of any one of Compounds of Present Invention 1 to 103 and Intermediate Compounds M6-1 to M6-49, 60 mg of lactose, 25 mg of corn starch, 6 mg of carmellose calcium and an appropriate amount of 5% hydroxypropyl methylcellulose are mixed, and a hard shell gelatin capsule or a hydroxypropyl methylcellulose capsule is filled with the resulting mixture to obtain an encapsulated formulation.

Formulation Example 15

Distilled water is added to 1000 mg of any one of Compounds of Present Invention 1 to 103 and Intermediate Compounds M6-1 to M6-49, 500 mg of fumaric acid, 2000 mg of sodium chloride, 150 mg of methylparaben, 50 mg of propylparaben, 25000 mg of granulated sugar, 13000 mg of sorbitol (70% solution), 100 mg of Veegum K (Vanderbilt Co.), 35 mg of flavor and 500 mg of colorant, such that a final volume is 100 ml, and the mixture is mixed to obtain a suspension for oral administration.

Formulation Example 16

5 parts of any one of Compounds of Present Invention 1 to 103 and Intermediate Compounds M6-1 to M6-49 and 5 parts of polysorbate 85 are dissolved in 3 parts of benzyl alcohol and 30 parts of propylene glycol, and a phosphate buffer is added to this solution so as to have a pH of 6.0 to 6.5, and then water is added until a total amount is 100 parts to obtain a liquid formulation for oral administration.

Formulation Example 17

5 parts of aluminum distearate are dispersed in 57 parts of fractionated palm oil and 3 parts of polysorbate 85 by heating. This dispersion is cooled to room temperature, and 25 parts of saccharin are dispersed in an oily vehicle thereof. 10 parts of any one of Compounds of Present Invention 1 to 103 and Intermediate Compounds M6-1 to M6-49 are added thereto to obtain a paste formulation for oral administration.

Formulation Example 18

5 parts of any one of Compounds of Present Invention 1 to 103 and Intermediate Compounds M6-1 to M6-49 and 95 parts of limestone filler are mixed, and a granule for oral administration is obtained using wet granulation method.

Formulation Example 19

5 parts of any one of Compounds of Present Invention 1 to 103 and Intermediate Compounds M6-1 to M6-49 are dissolved in 80 parts of diethylene glycol monoethyl ether, and 15 parts of propylene carbonate are mixed therewith

to obtain a spot-on solution.

Formulation Example 20

10 parts of any one of Compounds of Present Invention 1 to 103 and Intermediate Compounds M6-1 to M6-49 are dissolved in 70 parts of diethylene glycol monoethyl ether, and 20 parts of 2-octyl dodecanol are mixed therewith to obtain a pour-on solution.

Formulation Example 21

60 parts of NIKKOL TEALS-42 (Nikko Chemicals Co., Ltd., 42% aqueous solution of triethanolamine lauryl sulfate) and 20 parts of propylene glycol are added to 0.5 parts of any one of Compounds of Present Invention 1 to 103 and Intermediate Compounds M6-1 to M6-49, and the mixture is sufficiently stirred and mixed until it becomes a uniform solution, and then 19.5 parts of water are added and further sufficiently stirred and mixed to obtain a shampoo agent as a uniform solution.

Formulation Example 22

0.15 parts of any one of Compounds of Present Invention 1 to 103 and intermediate Compounds M6-1 to M6-49, 95 parts of an animal feed and 4.85 parts of a mixture of secondary calcium phosphate, diatomaceous earth, Aerosil and carbonate (or chalk) are sufficiently stirred and mixed to obtain a feed premix for animal.

Formulation Example 23

7.2 g of any one of Compounds of Present Invention 1 to 103 and Intermediate Compounds M6-1 to M6-49 and 92.8 g of VOSCO S-55 (manufactured by Maruishi Pharmaceutical Co., Ltd.) are dissolved and mixed at 100°C, poured into a suppository mold, and cooled and solidified to obtain a suppository.
Next, the pest control effect of the compound of the present invention is shown as test examples.

Test Example 1

Compounds of Present Invention 1 to 5, 8 to 15, 18, 19, 25, 26, 45, 52 to 55, 57 to 61, 64 to 66, 68 to 74, 78 and 86 to 90 and Intermediate Compounds M6-8, M6-9, M6-11, M6-14, and M6-23 as obtained in Formulation Example 5 were diluted with water, so as to have a concentration of the active ingredient of 500 ppm, to prepare a diluted liquid for test.
On the other hand, on a cucumber seedling (the first true leaf stage) planted in a plastic oup was inoculated with about 30 Aphis gossypii (whole stage), and leaving it for a day. 20 ml of the diluted liquid for test is sprayed on the seedling.
Six days after spraying, the number of surviving Aphis gossypii parasitized on the leaves of the cucumber was examined, and the controlling value was calculated according to the following equation:

$$\text{Controlling value } (\%) = \{1 - (Cb \times Tai)/(Cai \times Tb)\} \times 100$$

wherein the symbols represent as follows:

Cb: the number of insects in a non-treated section before treatment,
Cai: the number of insects in a non-treated section on observation,
Tb: the number of insects in a treated section before treatment,
Tai: the number of insects in a treated section on observation,
wherein the non-treated section refers to a section where the diluted liquid for test prepared by diluting the formulation not containing the compound of the present invention as in Formulation Example 5 with the same amount of water as in the treated section was sprayed.

As a result, in the treated section using a diluted liquid for test of each of Compounds of Present Invention 1 to 5, 8 to 15, 18, 19, 25, 26, 45, 52 to 55, 57 to 61, 64 to 66, 68 to 74, 78, and 86 to 90 and Intermediate Compounds M6-8, M6-9, M6-11, M6-14 and M6-23, the controlling value was 90% or more.

Test Example 2

The formulations of Compounds of Present Invention 4, 8 to 12, 18, 19, 25, 26, 71, 88 and 90 as obtained in Formulation Example 5 were diluted with water, so as to have a concentration of the active ingredient of 500 ppm, to prepare a diluted liquid for test.

On the other hand, a cucumber seedling (the second true leaf stage) planted in a plastic cup was drenched at its foot with 5 ml of the diluted liquid for test, and kept in a greenhouse at 25°C for 7 days. On the cucumber leaf surface was inoculated about 30 Aphis gossypii (whole stage), and further kept in the greenhouse for 6 days, then the number of surviving Aphis gossypii parasitized on the leaves of the cucumber was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment,
Cai: the number of insects in a non-treated section on observation,
Tb: the number of insects in a treated section before treatment,
Tai: the number of insects in a treated section on observation,
wherein the non-treated section refers to a section where the diluted liquid for test prepared by diluting the formulation not containing the compound of the present invention as in Formulation Example 5 with the same amount of water as in the treated section was sprayed.

As a result, in the treated section using a diluted liquid for test of each of Compounds of Present Invention 4, 8 to 12, 18, 19, 25, 26, 71, 88 and 90, the controlling value was 90% or more.

Test Example 3

The formulations of Compounds of Present Invention 1 to 4, 6, 8 to 11, 18, 19, 53 to 55, 58, 68 and 71 and Intermediate Compound M6-23 as obtained in Formulation Example 5 were diluted with water, so as to have a concentration of the active ingredient of 500 ppm, to prepare a diluted liquid for test.

On a rice seedling in the second leaf stage planted in a polyethylene cup was sprayed 10 ml of each diluted liquid for test. After air-drying, 20 third-fourth instar larvae of Nilaparvata lugens were released, and kept in the greenhouse at 25°C. After 6 days, the number of Nilaparvata lugens parasitized on the rice was examined, and the controlling value was calculated according to the following equation:

$$\text{Controlling value (\%)} = \{1 - (Cb \times Tai)/(Cai \times Tb)\} \times 100$$

wherein the symbols represent as follows:

Cb: the number of insects in a non-treated section before treatment,
Cai: the number of insects in a non-treated section on observation,
Tb: the number of insects in a treated section before treatment,
Tai: the number of insects in a treated section on observation,
wherein the non-treated section refers to a section where the diluted liquid for test prepared by diluting the formulation not containing the compound of the present invention as in Formulation Example 5 with the same amount of water as in the treated section was sprayed.

As a result, in the treated section using a diluted liquid for test of each of Compounds of Present Invention 1 to 4, 6, B to 11, 18, 19, 53 to 55, 58, 68 and 71 and Intermediate Compound M6-23, the controlling value was 90% or more.

Test Example 4

The formulations of Compounds of Present Invention 8, 10, 12 and 18 as obtained in Formulation Example 5 were diluted with water, so as to have a concentration of the active ingredient of 500 ppm, to prepare a diluted liquid for test.

On the other hand, a rice seedling (2 weeks after sowing, the second leaf stage) planted in a plastic cup is drenched at its foot with 5 ml of each diluted liquid for test, and kept in a greenhouse of 25°C for 7 days. 20 third-fourth instar larvae of Nilaparvata lugens were released, and further kept in the greenhouse for 6 days, then the number of surviving Nilaparvata lugens parasitized on the rice was examined, and the controlling value was calculated according to the following equation:

$$\text{Controlling value } (\%) = \{1 - (Cb \times Tai)/(Cai \times Tb)\} \times 100$$

wherein the symbols represent as follows:

Cb: the number of insects in a non-treated section before treatment,
Cai: the number of insects in a non-treated section on observation,
Tb: the number of insects in a treated section before treatment,
Tai: the number of insects in a treated section on observation,
wherein the non-treated section refers to a section where the diluted liquid for test prepared by diluting the formulation not containing the compound of the present invention as in Formulation Example 5 with the same amount of water as in the treated section was sprayed.

As a result, in the treated section using a diluted liquid for test containing each of Compounds of Present Invention 8, 10, 12 and 18, the controlling value was 90% or more.

Test Example 5

The formulations of Compounds of Present Invention 1 to 4, 8 to 12, 18, 19, 25, 26, 71, 87, 88 and 90 and Intermediate Compounds M6-11 and M6-23 as obtained in Formulation Example 5 were diluted with water, ao as to have a concentration of the active ingredient of 500 ppm, to prepare a diluted liquid for test.

On the other hand, Bemisia tabaci adult was released on a tomato seedling (the third true leaf stage) planted in a polyethylene cup, and made to lay eggs for about 72 hours. The tomato seedling was kept in a greenhouse for 8 days, and when instar larvae hatched from the eggs, the above diluted liquid for test was sprayed in an amount of 20 ml/cup, and the cup was kept in a greenhouse at 25°C. After 7 days, the number of surviving instar larvae on the tomato leaves was examined, and the controlling value was calculated according to the following equation:

$$\text{Controlling value } (\%) = \{1 - (Cb \times Tai)/(Cai \times Tb)\} \times 100$$

wherein the symbols represent as follows:

Cb: the number of insects in a non-treated section before treatment,
Cai: the number of insects in a non-treated section on observation,
Tb: the number of insects in a treated section before treatment,
Tai: the number of insects in a treated section on observation,
wherein the non-treated section refers to a section where the diluted liquid for test prepared by diluting the formulation not containing the compound of the present invention as in Formulation Example 5 with the same amount of water as in the treated section was sprayed.

As a result, in the treated section using a diluted liquid for test of each of Compounds of Present Invention 1 to 4, 8 to 12, 18, 19, 25, 26, 71, 87, 88 and 90 and Intermediate Compounds M6-11 and M6-23, the controlling value was 90% or more.

Test Example 6

The formulations of Compounds of Present Invention 1 to 5, 8 to 11, 13, 14, 18, 19, 25, 26, 52 to 55, 57 to 61, 66, 68 to 74, 76 to 78, 86, 88 and 90 and Intermediate Compounds M6-8, M6-9, M6-11 to M6-13 and M6-23 to M6-24 as obtained in Formulation Example 5 were diluted with water, so as to have a concentration of the active ingredient of 500 ppm, to prepare a diluted liquid for test.

On the other hand, on cabbage at the third leaf stage planted in a polyethylene cup is sprayed in an amount

of 20 mL/cup of the diluted liquid for test. After the drug solution was dried, the foliage part was cut off, and then placed in a 50 mL volume cup. Five second instar larvae of Plutella xylostella were released into the cup, and the cup was sealed with a lid. After the cup was kept at 25°C for 5 days, the number of surviving insects was counted. The mortality was calculated according to the following equation:

Mortality (%) = (Number of dead insects/Number of tested insects) × 100.

As a result, in the treated section using a diluted liquid for test of each of Compounds of Present Invention 1 to 5, 8 to 11, 13, 14, 18, 19, 25, 26, 52 to 55, 57 to 61, 66, 68 to 74, 76 to 78, 86, 88 and 90 and Intermediate Compounds M6-8, M6-9, M6-11 to M6-13 and M6-23 to M6-24, the death rate was 80% or more.

Test Example 7

The formulations of Compounds of Present Invention 1 to 5, 8 to 14, 18, 19, 25, 26, 54, 55, 60, 68 to 74, 76 to 78, 85, 86, 88 and 90 and Intermediate Compounds M6-8 to M6-13 as obtained in Formulation Example 5 were diluted with water, so as to have a concentration of the active ingredient of 500 ppm, to prepare a diluted liquid for test.

On the other hand, an apple tree was planted in a plastic cup, and grown until the seventh-eighth true leaf was spread. To the apple tree was sprayed in an amount of 20 mL/cup of the diluted liquid for test. After the drug solution was dried, 60 first-instar Adoxophyes orana fasciata were released, and the plastic cup the bottom of which was cut off and on which a filter paper was put was upside-down and covered. After 7 days, the number of surviving insects was counted, and the mortality was calculated according to the following equation:

Mortality (%) = (Number of dead insects/Number of tested insects) × 100.

As a result, in the treated section using a diluted liquid for test of each of Compounds of Present Invention 1 to 5, 8 to 14, 18, 19, 25, 26, 54, 55, 60, 68 to 74, 76 to 78, 85, 86, 88 and 90 and Intermediate Compounds M6-8 to M6-13, the mortality was 90% or more.

As a result, in the treated section using a diluted liquid for test of each of Compounds of Present Invention 1 to 5, 10, 18, 19, 25, 26, 60 and 68 and Intermediate Compound M6-1, the mortality was 100%.

Test Example 8

Each of formulations of Compounds of Present Invention 1 to 5, 10, 18, 19, 25, 26, 60 and 68 and Intermediate Compound M6-1 as obtained in Formulation Example 5 was diluted with water, so as to have a concentration of the active ingredient of 500 ppm, to prepare a diluted liquid for test.

A filter paper having a diameter of 5.5 cm was spread on the bottom of a polyethylene cup having the same diameter and 0.7 ml of the diluted liquid for test was added dropwise onto the filter paper, and 30 mg of sucrose was uniformly placed as bait. Into the polyethylene cup, 10 female imagoes of Musca domestica were released, and the cup was sealed with a lid. After 24 hours, the life and death of Musca domestica was examined, and the mortality was calculated.

As a result, in the treated section using a diluted liquid for test of each of Compounds of Present Invention 1 to 5, 10, 18, 19, 25, 26, 60 and 68 and Intermediate Compound M6-1, the mortality was 100%.

Test Example 9

The formulations of compounds of Present Invention 3, 10 and 11 as obtained in Formulation Example 5 were diluted with water, so as to have a concentration of the active ingredient of 500 ppm, to prepare a diluted liquid for test.

A filter paper having a diameter of 5.5 cm was spread on the bottom of a polyethylene cup having the same diameter and 0.7 ml of the diluted liquid for test was added dropwise onto the filter paper, and 30 mg of sucrose was uniformly placed as bait. Into the polyethylene cup, 2 male imagoes of Blattella germanica were released, and the cup was sealed with a lid. After 6 days, the life and death of Blattella germanica was examined, and the mortality was calculated.

As a result, in the treated section using a diluted liquid for test containing each of Compounds of Present

Invention 3, 10 and 11, the mortality was 100%.

Test Example 10

The formulations of Compounds of Present Invention 1 to 5, 8 to 11, 13, 18, 19, 25, 26, 44, 53 to 55, 59, 60, 68 to 74, 78, 86 to 88 and 90 and Intermediate Compounds M6-3, M6-4, M6-11, M6-12 and M6-23 as obtained in Formulation Example 5 were diluted with water, so as to have a concentration of the active ingredient of 500 ppm, to prepare a diluted liquid for test.

0.7 ml of the diluted liquid for test was added to 100 ml of ion-exchanged water (active ingredient concentration: 3.5 ppm). 20 last-instar larvae of Culex pipiens pallens were released into the solution. One day later, the life and death of the Culex pipiens pallens was examined, and the mortality of the pest was calculated.

As a result, in the treated section using a diluted liquid for test of each of Compounds of Present Invention 1 to 5, 8 to 11, 13, 18, 19, 25, 26, 44, 53 to 55, 59, 60, 68 to 74, 78, 86 to 88 and 90 and Intermediate Compounds M6-3, M6-4, M6-11, M6-12 and M6-23, the mortality was 95% or more.

Test Example 11

2 mg of each of Compounds of Present Invention 15, 18, 19, 46, 54, 58, 62, 64, 69, 72, 77 to 79, 81 and 84 and Intermediate Compounds M6-9 to M6-10 was weighed in a screw tube (Maruemu No. 5; 27 × 55 mm), and 0.2 mL of acetone was added thereto and sealed with a cap to dissolve the compound. The screw tube was rotated and inverted to uniformity coat the drug solution onto the whole inner wall of the tube. After removing the cap, the solution was air-dried for about 2 hours, then non-blood-sucking nymphal ticks, Haemaphysalis longicornis (5 ticks/group) were released, and the tube was sealed with the cap. After 2 days, the number of dead ticks was examined, and the mortality was calculated according to the following equation:

$$\text{Mortality (\%)} = 100 \times (\text{Number of dead ticks/Number of tested ticks}).$$

As a result, in the treated section treated with each of Compounds of Present Invention 15, 18, 19, 46, 54, 58, 62, 64, 69, 72, 77 to 79, 81 and 84 and Intermediate Compounds M6-9 to M6-10, the mortality was 100%.

INDUSTRIAL APPLICABILITY

The compound of the present invention has a controlling effect on pests and is useful as an active ingredient of a pest control agent.

## Claims

1. An amide compound represented by formula (1) or an N-oxide thereof:

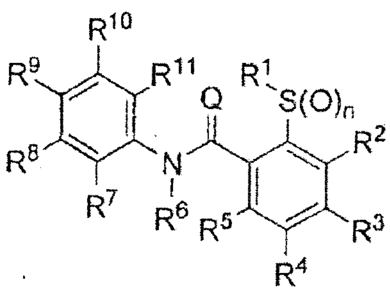

(1)

wherein

$R^1$ represents a C1 to C6 chain hydrocarbon group optionally having one or more atoms or groups selected from group X or a C3 to C6 cycloalkyl group optionally having one or more atoms or groups selected from group Y,

$R^2$, $R^3$, $R^4$ and $R^5$ are the same or different and represent a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom.

$R^6$ represents a methyl group, an ethyl group, a propyl group, an isopropyl group, a butyl group, an isobutyl group, or a hexyl group,

$R^7$ and $R^{11}$ are the same or different and represent a C1 to C6 chain hydrocarbon group optionally having one or more atoms or groups selected from group X, a C3 to C6 cycloalkyl group optionally having one or more atoms or groups selected from group Y, $-OR^{16}$, $-S(O)_mR^{16}$, a fluorine atom or a hydrogen atom,

$R^8$, $R^9$ and $R^{10}$ are the same or different and represent a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{14}$, $-C(O)NR^{15}R^{16}$, $-SF_5$, a cyano group, a nitro group, a halogen atom, or a hydrogen atom,

$R^{12}$ and $R^{13}$ are the same or different and represent a C1 to C6 alkyl group optionally having one or more halogen atoms, a phenyl group optionally having one or more atoms or groups selected from group Z, a 5- or 6-membered heterocyclic group optionally having one or more atoms or groups selected from group Z or a hydrogen atom,

$R^{14}$ represents a C1 to C6 alkyl group optionally having one or more halogen atoms, a phenyl group optionally having one or more atoms or groups selected from group Z or a 5- or 6-membered heterocyclic group optionally having one or more atoms or groups selected from group Z,

$R^{15}$ represents a C1 to C6 alkyl group optionally having one or more halogen atoms or a hydrogen atom,

$R^{16}$ represents a C1 to C6 alkyl group optionally having one or more halogen atoms,

Q represents an oxygen atom or a sulfur atom,

m represents 0, 1 or 2, and

n represents 0, 1 or 2;

Group X: a group consisting of C3 to C6 cycloalkyl groups optionally having one or more atoms or groups selected from the group consisting of halogen atoms and C1 to C3 alkyl groups, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, and halogen atoms;

Group Y: a group consisting of C1 to C6 alkyl groups optionally having one or more halogen atoms, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, and halogen atoms;

Group Z: a group consisting of C1 to C6 alkyl groups optionally having one or more halogen atoms, C1 to C6 alkoxy groups optionally having one or more halogen atoms, C1 to C6 alkylsulfanyl groups optionally having one or more halogen atoms. C1 to C6 alkylsulfinyl groups optionally having one or more halogen atoms, C1 to C6 alkylsulfonyl groups optionally having one or more halogen atoms, C1 to C6 alkylamino groups optionally having one or more halogen atoms, C2 to C8 dialkylamino groups optionally having one or more halogen atoms, C2 to C6 alkylcarbonyl groups optionally having one or more halogen atoms, C2 to C6 alkoxycarbonyl groups optionally having one or more halogen atoms, hydroxy groups, thiol groups, amino groups, cyano groups, nitro groups, and halogen atoms;

Group W: a group consisting of C3 to C6 cycloalkyl groups optionally having one or more atoms or groups selected from the group consisting of halogen atoms and C1 to C3 alkyl groups, C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkoxycarbonyl groups optionally having one or more halogen atoms, hydroxy groups, cyano groups, and halogen atoms;

wherein $R^8$, $R^9$ and $R^{10}$ do not simultaneously represent a hydrogen atom, when $R^7$, $R^8$, $R^9$ and $R^{11}$ represent a hydrogen atom, $R^{10}$ does not represent a methyl group, and when $R^7$, $R^8$, $R^{10}$ and $R^{11}$ represent a hydrogen atom, $R^9$ does not represent a pyrrolidin-1-yl group, and

when m is 1 or 2 in $-S(O)_mR^{12}$, $R^{12}$ does not represent a hydrogen atom.

**2.** The compound according to claim 1,

wherein $R^1$ is a C3 to C6 cycloalkyl group optionally having one or more atoms or groups selected from the group consisting of halogen atoms and C1 to C3 alkyl groups,
a C1 to C6 alkyl group optionally having one or more atoms or groups selected from the group consisting of halogen atoms and a cyclopropyl group (wherein the cyclopropyl group optionally has one or more halogen atoms or one or more C1 to C3 alkyl groups), a C2 to C6 alkenyl group optionally having one or more halogen atoms, or
a C2 to C6 alkynyl group optionally having one or more halogen atoms, $R^2$, $R^4$ and $R^5$ are the same or different and are a C1 to C6 alkyl group optionally having one or more halogen atoms, $-OR^{12}$, $-S(O)_mR^{12}$, a halogen atom, or a hydrogen atom,
$R^3$ is a C1 to C6 chain hydrocarbon group optionally having one or more atoms or groups selected from group X, a C3 to C6 cycloalkyl group optionally having one or more atoms or groups selected from group Y, a phenyl group optionally having one or more atoms or groups selected from group Z, a 5- or 6-membered heterocyclic group optionally having one or more atoms or groups selected from group Z, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, a cyano group, a nitro group, a halogen atom, or a hydrogen atom,
$R^6$ is a methyl group, an ethyl group, a propyl group, an isopropyl group, a butyl group, an isobutyl group, or a hexyl group,
$R^7$ and $R^{11}$ are the same or different and are a C1 to C6 alkyl group optionally having one or more halogen atoms, $-OR^{16}$, $-S(O)_mR^{16}$, a fluorine atom or a hydrogen atom, $R^8$, $R^9$ and $R^{10}$ are the same or different and are a C1 to C6 chain hydrocarbon group optionally having one or more atoms or groups selected from group X, $-OR^{12}$, $-S(O)_mR^{12}$, $-SF_5$, a halogen atom or a hydrogen atom,
$R^{12}$ and $R^{13}$ are the same or different and are a C1 to C6 alkyl group optionally having one or more halogen atoms, a phenyl group (wherein the phenyl group optionally has a C1 to C3 alkyl group optionally having one or more halogen atoms or one or more halogen atoms), a 5- or 6-membered heterocyclic group (wherein the 5- or 6-membered heterocyclic group optionally has a C1 to C3 alkyl group optionally having one or more halogen atoms or one or more halogen atoms) or a hydrogen atom,
$R^{14}$ is a C1 to C6 alkyl group optionally having one or more halogen atoms, a phenyl group (wherein the phenyl group optionally has a C1 to C3 alkyl group optionally having one or more halogen atoms or one or more halogen atoms) or a 5- or 6-membered heterocyclic group (wherein the 5- or 6-membered heterocyclic group optionally has a C1 to C3 alkyl group optionally having one or more halogen atoms or one or more halogen atoms), and
Q is an oxygen atom.

**3.** The compound according to claim 1,
wherein $R^1$ is a C3 to C6 cycloalkyl group optionally having one or more atoms or groups selected from the group consisting of halogen atoms and C1 to C3 alkyl groups,
a C2 to C6 alkyl group optionally having one or more halogen atoms or
a (C3 to C6 cycloalkyl)C1 to C3 alkyl group optionally having one or more halogen atoms,
$R^2$, $R^4$ and $R^5$ are the same or different and are a halogen atom or a hydrogen atom,
$R^3$ is a C1 to C6 alkyl group optionally having one or more halogen atoms, a C2 to C6 alkenyl group optionally having one or more halogen atoms, a C2 to C6 alkynyl group optionally having one or more halogen atoms, a 5- or 6-membered heterocyclic group (wherein the 5- or 6-membered heterocyclic group optionally has a C1 to C3 alkyl group optionally having one or more halogen atoms or one or more halogen atoms), $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, a halogen atom, or a hydrogen atom,
$R^6$ is a methyl group, an ethyl group, a propyl group, an isopropyl group, a butyl group, an isobutyl group, or a hexyl group,
$R^7$ and $R^{11}$ are the same or different and are a fluorine atom or a hydrogen atom, $R^8$, $R^9$ and $R^{10}$ are the same or different and are a C1 to C6 alkyl group optionally having one or more halogen atoms, a C2 to C6 alkenyl group optionally having one or more halogen atoms, a C2 to C6 alkynyl group optionally having one or more halogen atoms, $-OR^{12}$, $-S(O)_mR^{12}$, a halogen atom or a hydrogen atom,
$R^{12}$ and $R^{13}$ are the same or different and are a C1 to C6 alkyl group optionally having one or more halogen atoms or a hydrogen atom,
$R^{14}$ is a C1 to C6 alkyl group optionally having one or more halogen atoms, and
Q is an oxygen atom.

**4.** The compound according to any of claims 1 to 3, wherein $R^2$, $R^4$ and $R^5$ are a hydrogen atom.

**5.** The compound according to any of claims 1 to 4, wherein $R^7$ and $R^{11}$ are a hydrogen atom.

6. The compound according to any of claims 1 to 5, wherein $R^3$ is a C1 to C3 alkyl group optionally having one or more halogen atoms, a C1 to C3 alkoxy group optionally having one or more halogen atoms, a C1 to C3 alkylsulfanyl group optionally having one or more halogen atoms, a C1 to C3 alkylsulfinyl group optionally having one or more halogen atoms, a C1 to C3 alkylsulfonyl group optionally having one or more halogen atoms, a C1 to C6 alkylamino group optionally having one or more halogen atoms, a C2 to C8 dialkylamino group optionally having one or more halogen atoms, a C2 to C6 alkylcarbonylamino group optionally having one or more halogen atoms, a C2 to C6 alkoxycarbonylamino group optionally having one or more halogen atoms, a C1 to C3 alkylsulfonylamino group optionally having one or more halogen atoms, an amino group, a C2 to C6 alkylcarbonyl group optionally having one or more halogen atoms, a C2 to C6 alkoxycarbonyl group optionally having one or more halogen atoms, a C2 to C6 alkylaminocarbonyl group optionally having one or more halogen atoms, a C3 to C8 dialkylaminocarbonyl group optionally having one or more halogen atoms, an aminocarbonyl group, a pyridyl group (wherein the pyridyl group optionally has one or more atoms or substituents selected from the group consisting of C1 to C3 alkyl groups optionally having a halogen atom, C1 to C3 alkoxy groups optionally having a halogen atom, and halogen atoms), a pyrimidinyl group (wherein the pyrimidinyl group optionally has one or more atoms or substituents selected from the group consisting of C1 to C3 alkyl groups optionally having a halogen atom, C1 to C3 alkoxy groups optionally having a halogen atom, and halogen atoms), a halogen atom, or a hydrogen atom.

7. The compound according to any of claims 1 to 6, wherein $R^6$ is a methyl or an ethyl group.

8. The compound according to any of claims 1 to 7, wherein $R^1$ is an ethyl group, a cyclopropyl group, or a cyclopropylmethyl group.

9. The compound according to any of claims 1 to 8, wherein $R^8$, $R^9$ and $R^{10}$ are the same or different and are a C1 to C6 haloalkyl group, a C2 to C6 haloalkenyl group, a C1 to C6 haloalkoxy group, a C1 to C6 haloalkylsulfanyl group, a C1 to C6 haloalkylsulfinyl group, a C1 to C6 haloalkylsulfonyl group, a halogen atom, or a hydrogen atom.

10. The compound according to any of claims 1 to 8, wherein $R^8$, $R^9$ and $R^{10}$ are the same or different and are a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

11. The compound according to any of claims 1 to 8, wherein $R^7$, $R^8$, $R^{10}$ and $R^{11}$ are a hydrogen atom, $R^9$ is a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, or a halogen atom.

12. The compound according to any of claims 1 to 8, wherein $R^7$, $R^9$ and $R^{11}$ are a hydrogen atom, $R^8$ and $R^{10}$ are the same or different and are a C1 to C3 perfluoroalkyl group, a C1 to C3 perfluoroalkoxy group, a C1 to C3 perfluoroalkylsulfanyl group, a C1 to C3 perfluoroalkylsulfinyl group, a C1 to C3 perfluoroalkylsulfonyl group, a halogen atom, or a hydrogen atom.

13. A pest control composition comprising the compound as defined in any of claims 1 to 12, and an inert carrier.

14. A method for controlling pests comprising applying an effective amount of the compound as defined in any of claims 1 to 12 to plants, soil or in-house.

15. The compound as defined in any of claims 1 to 12 for use in the control of pests.

**Patentansprüche**

1. Eine Amidverbindung dargestellt durch Formel (1) oder ein N-Oxid davon:

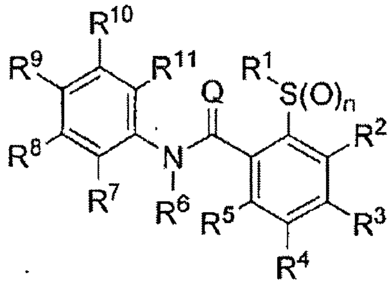

(1)

wobei

$R^1$ eine C1-C6-kettige Kohlenwasserstoffgruppe, die gegebenenfalls ein(e) oder mehrere Atome oder Gruppen ausgewählt aus Gruppe X aufweist, oder eine C3-C6-Cycloalkylgruppe, die gegebenenfalls ein(e) oder mehrere Atome oder Gruppen ausgewählt aus Gruppe Y aufweist, darstellt,

$R^2$, $R^3$, $R^4$ und $R^5$ gleich oder verschieden sind und eine C1- bis C6-kettige Kohlenwasserstoffgruppe, die gegebenenfalls ein(e) oder mehrere Atome oder Gruppen ausgewählt aus Gruppe X aufweist, eine C3- bis C6-Cycloalkylgruppe, die gegebenenfalls ein(e) oder mehrere Atome oder Gruppen ausgewählt aus Gruppe Y aufweist, eine Phenylgruppe, die gegebenenfalls ein(e) oder mehrere Atome oder Gruppen ausgewählt aus Gruppe Z aufweist, eine 5- oder 6-gliedrige heterocyclische Gruppe, die gegebenenfalls ein(e) oder mehrere Atome oder Gruppen ausgewählt aus Gruppe Z aufweist, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, eine Cyanogruppe, eine Nitrogruppe, ein Halogenatom oder ein Wasserstoffatom darstellen;

$R^6$ eine Methylgruppe, eine Ethylgruppe, eine Propylgruppe, eine Isopropylgruppe, eine Butylgruppe, eine Isobutylgruppe oder eine Hexylgruppe darstellt,

$R^7$ und $R^{11}$ gleich oder verschieden sind und eine C1- bis C6-kettige Kohlenwasserstoffgruppe, die gegebenenfalls ein(e) oder mehrere Atome oder Gruppen ausgewählt aus Gruppe X aufweist, eine C3- bis C6-Cycloalkylgruppe, die gegebenenfalls ein(e) oder mehrere Atome oder Gruppen ausgewählt aus Gruppe Y aufweist, $-OR^{16}$, $-S(O)_mR^{16}$, ein Fluoratom oder ein Wasserstoffatom darstellen,

$R^8$, $R^9$ und $R^{10}$ gleich oder verschieden sind und eine C1- bis C6-kettige Kohlenwasserstoffgruppe, die gegebenenfalls ein(e) oder mehrere Atome oder Gruppen ausgewählt aus Gruppe X aufweist, eine C3- bis C6-Cycloalkylgruppe, die gegebenenfalls ein(e) oder mehrere Atome oder Gruppen ausgewählt aus Gruppe Y aufweist, eine Phenylgruppe, die gegebenenfalls ein(e) oder mehrere Atome oder Gruppen ausgewählt aus Gruppe Z aufweist, eine 5- oder 6-gliedrige heterocyclische Gruppe, die gegebenenfalls ein(e) oder mehrere Atome oder Gruppen ausgewählt aus Gruppe Z aufweist, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{14}$, $-C(O)NR^{15}R^{16}$, $-SF_5$, eine Cyanogruppe, eine Nitrogruppe, ein Halogenatom oder ein Wasserstoffatom darstellen, $R^{12}$ und $R^{13}$ gleich oder verschieden sind und eine C1- bis C6-Alkylgruppe mit gegebenenfalls einem oder mehreren Halogenatomen, eine Phenylgruppe, die gegebenenfalls ein(e) oder mehrere Atome oder Gruppen ausgewählt aus Gruppe Z aufweist, eine 5- oder 6-gliedrige heterocyclische Gruppe, die gegebenenfalls ein(e) oder mehrere Atome oder Gruppen ausgewählt aus Gruppe Z aufweist, oder ein Wasserstoffatom darstellen,

$R^{14}$ eine C1- bis C6-Alkylgruppe mit gegebenenfalls einem oder mehreren Halogenatomen, eine Phenylgruppe, die gegebenenfalls ein(e) oder mehrere Atome oder Gruppen ausgewählt aus Gruppe Z aufweist, oder eine 5- oder 6-gliedrige heterocyclische Gruppe, die gegebenenfalls ein(e) oder mehrere Atome oder Gruppen ausgewählt aus der Gruppe Z aufweist, darstellt,

$R^{15}$ eine C1- bis C6-Alkylgruppe mit gegebenenfalls einem oder mehreren Halogenatomen oder ein Wasserstoffatom darstellt,

$R^{16}$ eine C1- bis C6-Alkylgruppe mit gegebenenfalls einem oder mehreren Halogenatomen darstellt,

Q ein Sauerstoffatom oder ein Schwefelatom darstellt,

m für 0, 1 oder 2 steht und

n für 0, 1 oder 2 steht;

Gruppe X: eine Gruppe bestehend aus C3- bis C6-Cycloalkylgruppen, die gegebenenfalls ein(e) oder mehrere Atomen oder Gruppen ausgewählt aus der Gruppe bestehend aus Halogenatomen und C1- bis C3-Alkylgruppen aufweisen, C1- bis C6-Alkoxygruppen mit gegebenenfalls einem oder mehreren Halogenatomen, C1- bis C6-Alkylsulfanylgruppen mit gegebenenfalls einem oder mehreren Halogenatomen, C1- bis C6-Alkylsulfinylgruppen mit gegebenenfalls einem oder mehreren Halogenatomen, C1- bis C6-Alkylsulfonylgruppen mit gegebenenfalls einem oder mehreren Halogenatomen, und Halogenatomen;

Gruppe Y: eine Gruppe bestehend aus C1- bis C6-Alkylgruppen mit gegebenenfalls einem oder mehreren Halogenatomen, C1- bis C6-Alkoxygruppen mit gegebenenfalls einem oder mehreren Halogenatomen, C1- bis C6-Alkylsulfanylgruppen mit gegebenenfalls einem oder mehreren Halogenatomen, C1- bis C6-Alkylsulfinylgruppen mit gegebenenfalls einem oder mehreren Halogenatomen, C1- bis C6-Alkylsulfonylgruppen mit gegebenenfalls einem oder mehreren Halogenatomen, und Halogenatomen;

Gruppe Z: eine Gruppe bestehend aus C1- bis C6-Alkylgruppen mit gegebenenfalls einem oder mehreren Halogenatomen, C1- bis C6-Alkoxygruppen mit gegebenenfalls einem oder mehreren Halogenatomen, C1- bis C6-Alkylsulfanylgruppen mit gegebenenfalls einem oder mehreren Halogenatomen, C1- bis C6-Alkylsulfinylgruppen mit gegebenenfalls einem oder mehreren Halogenatomen, C1- bis C6-Alkylsulfonylgruppen mit gegebenenfalls einem oder mehreren Halogenatomen, C1- bis C6-Alkylaminogruppen mit gegebenenfalls einem oder mehreren Halogenatomen, C2- bis C8-Dialkylaminogruppen mit gegebenenfalls einem oder mehreren Halogenatomen, C2- bis C6-Alkylcarbonylgruppen mit gegebenenfalls einem oder mehreren Halogenatomen, C2- bis C6-Alkoxycarbonylgruppen mit gegebenenfalls einem oder mehreren Halogenatomen, Hydroxygruppen, Thiolgruppen, Aminogruppen, Cyanogruppen, Nitrogruppen und Halogenatomen;

Gruppe W: eine Gruppe bestehend aus C3- bis C6-Cycloalkylgruppen, die gegebenenfalls ein(e) oder mehrere Atome oder Gruppen ausgewählt aus der Gruppe bestehend aus Halogenatomen und C1- bis C3-Alkylgruppen aufweisen, C1- bis C6-Alkoxygruppen mit gegebenenfalls einem oder mehreren Halogenatomen, C1- bis C6-Alkylsulfanylgruppen mit gegebenenfalls einem oder mehreren Halogenatomen, C1- bis C6-Alkylsulfinylgruppen mit gegebenenfalls einem oder mehreren Halogenatomen, C1- bis C6-Alkylsulfonylgruppen mit gegebenenfalls einem oder mehreren Halogenatomen, C2- bis C6-Alkoxycarbonylgruppen mit gegebenenfalls einem oder mehreren Halogenatomen, Hydroxygruppen, Cyanogruppen und Halogenatomen;

wobei $R^8$, $R^9$ und $R^{10}$ nicht gleichzeitig ein Wasserstoffatom darstellen, wenn $R^7$, $R^8$, $R^9$ und $R^{11}$ ein Wasserstoffatom darstellen, $R^{10}$ keine Methylgruppe darstellt, und wenn $R^7$, $R^8$, $R^{10}$ und $R^{11}$ ein Wasserstoffatom darstellen, $R^9$ keine Pyrrolidin-1-ylgruppe darstellt und

wenn m in $-S(O)_mR^{12}$ gleich 1 oder 2 ist, $R^{12}$ kein Wasserstoffatom darstellt.

**2.** Die Verbindung gemäß Anspruch 1,

wobei $R^1$ eine C3- bis C6-Cycloalkylgruppe, die gegebenenfalls ein(e) oder mehrere Atome oder Gruppen ausgewählt aus der Gruppe bestehend aus Halogenatomen und C1- bis C3-Alkylgruppen aufweist,

eine C1- bis C6-Alkylgruppe, die gegebenenfalls ein(e) oder mehrere Atome oder Gruppen ausgewählt aus der Gruppe bestehend aus Halogenatomen und einer Cyclopropylgruppe (wobei die Cyclopropylgruppe gegebenenfalls eine oder mehrere Halogenatome oder eine oder mehrere C1- bis C3-Alkylgruppen aufweist) aufweist, eine C2- bis C6-Alkenylgruppe mit gegebenenfalls einem oder mehreren Halogenatomen, oder

eine C2- bis C6-Alkinylgruppe mit gegebenenfalls einem oder mehreren Halogenatomen darstellt,

$R^2$, $R^4$ und $R^5$ gleich oder verschieden sind und eine C1- bis C6-Alkylgruppe mit gegebenenfalls einem oder mehreren Halogenatomen, $-OR^{12}$, $-S(O)_mR^{12}$, ein Halogenatom oder ein Wasserstoffatom darstellen,

$R^3$ eine C1- bis C6-kettige Kohlenwasserstoffgruppe, die gegebenenfalls ein(e) oder mehrere Atome oder Gruppen ausgewählt aus Gruppe X aufweist, eine C3- bis C6-Cycloalkylgruppe, die gegebenenfalls ein(e) oder mehrere Atome oder Gruppen ausgewählt aus Gruppe Y aufweist, eine Phenylgruppe, die gegebenenfalls ein(e) oder mehrere Atome oder Gruppen ausgewählt aus Gruppe Z aufweist, eine 5- oder 6-gliedrige heterocyclische Gruppe, die gegebenenfalls ein(e) oder mehrere Atome oder Gruppen ausgewählt aus Gruppe Z aufweist, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, eine Cyanogruppe, eine Nitrogruppe, ein Halogenatom oder ein Wasserstoffatom darstellt;

$R^6$ eine Methylgruppe, eine Ethylgruppe, eine Propylgruppe, eine Isopropylgruppe, eine Butylgruppe, eine Isobutylgruppe oder eine Hexylgruppe darstellt,

$R^7$ und $R^{11}$ gleich oder verschieden sind und eine C1- bis C6-Alkylgruppe mit gegebenenfalls einem oder mehreren Halogenatomen, $-OR^{16}$, $-S(O)_mR^{16}$, ein Fluoratom oder ein Wasserstoffatom darstellen,

$R^8$, $R^9$ und $R^{10}$ gleich oder verschieden sind und eine C1- bis C6-kettige Kohlenwasserstoffgruppe, die gegebenenfalls ein(e) oder mehrere Atome oder Gruppen ausgewählt aus Gruppe X aufweist, $-OR^{12}$, $-S(O)_mR^{12}$, $SF_5$, ein Halogenatom oder ein Wasserstoffatom darstellen,

$R^{12}$ und $R^{13}$ gleich oder verschieden sind und eine C1- bis C6-Alkylgruppe mit gegebenenfalls einem oder mehreren Halogenatomen, eine Phenylgruppe (wobei die Phenylgruppe gegebenenfalls eine C1- bis C3-Alkylgruppe mit gegebenenfalls einem oder mehreren Halogenatomen, oder ein oder mehrere Halogenatome aufweist), eine 5- oder 6-gliedrige heterocyclische Gruppe (wobei die 5- oder 6-gliedrige heterocyclische Gruppe gegebenenfalls eine C1- bis C3-Alkylgruppe mit gegebenenfalls einem oder mehreren Halogenatomen, oder ein oder mehrere Halogenatome aufweist) oder ein Wasserstoffatom darstellen,

$R^{14}$ eine C1- bis C6-Alkylgruppe mit gegebenenfalls einem oder mehreren Halogenatomen, eine Phenylgruppe (wobei die Phenylgruppe gegebenenfalls eine C1- bis C3-Alkylgruppe mit gegebenenfalls einem oder mehreren

Halogenatomen, oder ein oder mehrere Halogenatome aufweist) oder eine 5- oder 6-gliedrige heterocyclische Gruppe (wobei die 5- oder 6-gliedrige heterocyclische Gruppe gegebenenfalls eine C1- bis C3-Alkylgruppe mit gegebenenfalls einem oder mehreren Halogenatomen, oder ein oder mehrere Halogenatome aufweist) und
Q ein Sauerstoffatom ist.

**3.** Die Verbindung gemäß Anspruch 1,
wobei $R^1$ eine C3- bis C6-Cycloalkylgruppe, die gegebenenfalls ein(e) oder mehrere Atome oder Gruppen ausgewählt aus der Gruppe bestehend aus Halogenatomen und C1- bis C3-Alkylgruppen aufweist,
eine C2- bis C6-Alkylgruppe mit gegebenenfalls einem oder mehreren Halogenatomen, oder
eine (C3- bis C6-Cycloalkyl)C1- bis C3-Alkylgruppe mit gegebenenfalls einem oder mehreren Halogenatomen dar-darstellt,
$R^2$, $R^4$ und $R^5$ gleich oder verschieden sind und ein Halogenatom oder ein Wasserstoffatom darstellen,
$R^3$ eine C1- bis C6-Alkylgruppe mit gegebenenfalls einem oder mehreren Halogenatomen, eine C2- bis C6-Alkenylgruppe mit gegebenenfalls einem oder mehreren Halogenatomen, eine C2- bis C6-Alkinylgruppe mit gegebenenfalls einem oder mehreren Halogenatomen, eine 5- oder 6-gliedrige heterocyclische Gruppe (wobei die 5- oder 6-gliedrige heterocyclische Gruppe gegebenenfalls eine C1- bis C3-Alkylgruppe mit gegebenenfalls einem oder mehreren Halogenatomen oder ein oder mehrere Halogenatome aufweist), $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, ein Halogenatom oder ein Wasserstoffatom darstellt,
$R^6$ eine Methylgruppe, eine Ethylgruppe, eine Propylgruppe, eine Isopropylgruppe, eine Butylgruppe, eine Isobutylgruppe oder eine Hexylgruppe darstellt,
$R^7$ und $R^{11}$ gleich oder verschieden sind und ein Fluoratom oder ein Wasserstoffatom darstellen,
$R^8$, $R^9$ und $R^{10}$ gleich oder verschieden sind und eine C1- bis C6-Alkylgruppe mit gegebenenfalls einem oder mehreren Halogenatomen, eine C2- bis C6-Alkenylgruppe mit gegebenenfalls einem oder mehreren Halogenatomen, eine C2- bis C6-Alkinylgruppe mit gegebenenfalls einem oder mehreren Halogenatomen, $-OR^{12}$, $-S(O)_mR^{12}$, ein Halogenatom oder ein Wasserstoffatom darstellen,
$R^{12}$ und $R^{13}$ gleich oder verschieden sind und eine C1- bis C6-Alkylgruppe mit gegebenenfalls einem oder mehreren Halogenatomen oder ein Wasserstoffatom darstellen,
$R^{14}$ eine C1- bis C6-Alkylgruppe mit gegebenenfalls einem oder mehreren Halogenatomen darstellt und
Q ein Sauerstoffatom ist.

**4.** Die Verbindung gemäß einem der Ansprüche 1 bis 3, wobei $R^2$, $R^4$ und $R^5$ ein Wasserstoffatom darstellen.

**5.** Die Verbindung gemäß einem der Ansprüche 1 bis 4, wobei $R^7$ und $R^{11}$ ein Wasserstoffatom darstellen.

**6.** Die Verbindung gemäß einem der Ansprüche 1 bis 5, wobei $R^3$ eine C1- bis C3-Alkylgruppe mit gegebenenfalls einem oder mehreren Halogenatomen, eine C1- bis C3-Alkoxygruppe mit gegebenenfalls einem oder mehreren Halogenatomen, eine C1- bis C3-Alkylsulfanylgruppe mit gegebenenfalls einem oder mehreren Halogenatomen, eine C1- bis C3-Alkylsulfinylgruppe mit gegebenenfalls einem oder mehreren Halogenatomen, eine C1- bis C3-Alkylsulfonylgruppe mit gegebenenfalls einem oder mehreren Halogenatomen, eine C1- bis C6-Alkylaminogruppe mit gegebenenfalls einem oder mehreren Halogenatomen, eine C2- bis C8-Dialkylaminogruppe mit gegebenenfalls einem oder mehreren Halogenatomen, eine C2- bis C6-Alkylcarbonylaminogruppe mit gegebenenfalls einem oder mehreren Halogenatomen, eine C2- bis C6-Alkoxycarbonylaminogruppe mit gegebenenfalls einem oder mehreren Halogenatomen, eine C1- bis C3-Alkylsulfonylaminogruppe mit gegebenenfalls einem oder mehreren Halogenatomen, eine Aminogruppe, eine C2- bis C6-Alkylcarbonylgruppe mit gegebenenfalls einem oder mehreren Halogenatomen, eine C2- bis C6-Alkoxycarbonylgruppe mit gegebenenfalls einem oder mehreren Halogenatomen, eine C2- bis C6-Alkylaminocarbonylgruppe mit gegebenenfalls einem oder mehreren Halogenatomen, eine C3- bis C8-Dialkylaminocarbonylgruppe mit gegebenenfalls einem oder mehreren Halogenatomen, eine Aminocarbonylgruppe, eine Pyridylgruppe (wobei die Pyridylgruppe gegebenenfalls ein(en) oder mehrere Atome oder Substituenten ausgewählt aus der Gruppe bestehend aus C1- bis C3-Alkylgruppen mit gegebenenfalls einem Halogenatom, C1- bis C3-Alkoxygruppen mit gegebenenfalls einem Halogenatom und Halogenatomen aufweist), eine Pyrimidinylgruppe (wobei die Pyrimidinylgruppe gegebenenfalls ein(en) oder mehrere Atome oder Substituenten ausgewählt aus der Gruppe bestehend aus C1- bis C3-Alkylgruppenn mit gegebenenfalls einem Halogenatom, C1- bis C3-Alkoxygruppen mit gegebenenfalls einem Halogenatom, und Halogenatomen aufweist), ein Halogenatom oder ein Wasserstoffatom darstellt.

**7.** Die Verbindung gemäß einem der Ansprüche 1 bis 6, wobei $R^6$ eine Methyl- oder eine Ethylgruppe darstellt.

**8.** Die Verbindung gemäß einem der Ansprüche 1 bis 7, wobei $R^1$ eine Ethylgruppe, eine Cyclopropylgruppe oder eine Cyclopropylmethylgruppe darstellt.

**9.** Die Verbindung gemäß einem der Ansprüche 1 bis 8, wobei $R^8$, $R^9$ und $R^{10}$ gleich oder verschieden sind und eine C1- bis C6-Halogenalkylgruppe, eine C2- bis C6-Halogenalkenylgruppe, eine C1- bis C6-Halogenalkoxygruppe, eine C1- bis C6-Halogenalkylsulfanylgruppe, eine C1- bis C6-Halogenalkylsulfinylgruppe, eine C1- bis C6-Halogenalkylsulfonylgruppe, ein Halogenatom oder ein Wasserstoffatom darstellen.

**10.** Die Verbindung gemäß einem der Ansprüche 1 bis 8, wobei $R^8$, $R^9$ und $R^{10}$ gleich oder verschieden sind und eine C1- bis C3-Perfluoralkylgruppe, eine C1- bis C3-Perfluoralkoxygruppe, eine C1- bis C3-Perfluoralkylsulfanylgruppe, eine C1- bis C3-Perfluoralkylsulfinylgruppe, eine C1- bis C3-Perfluoralkylsulfonylgruppe, ein Halogenatom oder ein Wasserstoffatom darstellen.

**11.** Die Verbindung gemäß einem der Ansprüche 1 bis 8, wobei $R^7$, $R^8$, $R^{10}$ und $R^{11}$ ein Wasserstoffatom darstellen, $R^9$ eine C1- bis C3-Perfluoralkylgruppe, eine C1- bis C3-Perfluoralkoxygruppe, eine C1- bis C3-Perfluoralkylsulfanylgruppe, eine C1- bis C3-Perfluoralkylsulfinylgruppe, eine C1- bis C3-Perfluoralkylsulfonylgruppe oder ein Halogenatom darstellt.

**12.** Die Verbindung gemäß einem der Ansprüche 1 bis 8, wobei $R^7$, $R^9$ und $R^{11}$ ein Wasserstoffatom darstellen, $R^8$ und $R^{10}$ gleich oder verschieden sind und eine C1- bis C3-Perfluoralkylgruppe, eine C1- bis C3-Perfluoralkoxygruppe, eine C1- bis C3-Perfluoralkylsulfanylgruppe, eine C1- bis C3-Perfluoralkylsulfinylgruppe, eine C1- bis C3-Perfluoralkylsulfonylgruppe, ein Halogenatom oder ein Wasserstoffatom darstellen.

**13.** Ein Schädlingsbekämpfungsmittel, umfassend die Verbindung wie in einem der Ansprüche 1 bis 12 definiert und einen inerten Träger.

**14.** Ein Verfahren zur Schädlingsbekämpfung, umfassend das Auftragen einer wirksamen Menge der Verbindung wie in einem der Ansprüche 1 bis 12 definiert auf Pflanzen, den Boden oder im Haus.

**15.** Die Verbindung wie in einem der Ansprüche 1 bis 12 definiert zur Verwendung bei der Schädlingsbekämpfung.

**Revendications**

**1.** Composé amide représenté par la formule (1) ou N-oxyde de celui-ci :

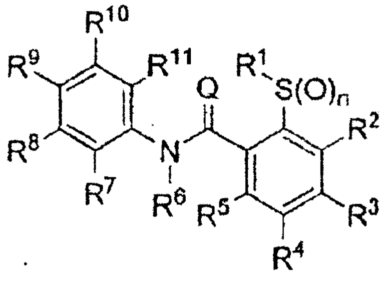

(1)

où

$R^1$ représente un groupe hydrocarboné à chaîne en C1 à C6 ayant éventuellement un ou plusieurs atomes ou groupes choisis dans le groupe X ou un groupe C3 à C6 cycloalkyle ayant éventuellement un ou plusieurs atomes ou groupes choisis dans le groupe Y,

$R^2$, $R^3$, $R^4$ et $R^5$ sont identiques ou différents et représentent un groupe hydrocarboné à chaîne en C1 à C6 ayant éventuellement un ou plusieurs atomes ou groupes choisis dans le groupe X, un groupe C3 à C6 cycloalkyle ayant éventuellement un ou plusieurs atomes ou groupes choisis dans le groupe Y, un groupe phényle ayant éventuellement un ou plusieurs atomes ou groupes choisis dans le groupe Z, un groupe hétérocyclique à 5 ou 6 chaînons ayant éventuellement un ou plusieurs atomes ou groupes choisis dans le groupe Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, un groupe cyano, un groupe nitro, un atome d'halogène, ou un atome d'hydrogène,

$R^6$ représente un groupe méthyle, un groupe éthyle, un groupe propyle, un groupe isopropyle, un groupe butyle, un groupe isobutyle, ou un groupe hexyle,
$R^7$ et $R^{11}$ sont identiques ou différents et représentent un groupe hydrocarboné à chaîne en C1 à C6 ayant éventuellement un ou plusieurs atomes ou groupes choisis dans le groupe X, un groupe C3 à C6 cycloalkyle ayant éventuellement un ou plusieurs atomes ou groupes choisis dans le groupe Y, $-OR^{16}$, $-S(O)_mR^{16}$, un atome de fluor ou un atome d'hydrogène,
$R^8$, $R^9$ et $R^{10}$ sont identiques ou différents et représentent un groupe hydrocarboné à chaîne en C1 à C6 ayant éventuellement un ou plusieurs atomes ou groupes choisis dans le groupe X, un groupe C3 à C6 cycloalkyle ayant éventuellement un ou plusieurs atomes ou groupes choisis dans le groupe Y, un groupe phényle ayant éventuellement un ou plusieurs atomes ou groupes choisis dans le groupe Z, un groupe hétérocyclique à 5 ou 6 chaînons ayant éventuellement un ou plusieurs atomes ou groupes choisis dans le groupe Z, $-OR^{12}$, $-S(O)_mR^{12}$, $-S(O)_2NR^{12}R^{13}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{14}$, $-C(O)NR^{15}R^{16}$, $-SF_5$, un groupe cyano, un groupe nitro, un atome d'halogène, ou un atome d'hydrogène,
$R^{12}$ et $R^{13}$ sont identiques ou différents et représentent un groupe C1 à C6 alkyle ayant éventuellement un ou plusieurs atomes d'halogène, un groupe phényle ayant éventuellement un ou plusieurs atomes ou groupes choisis dans le groupe Z, un groupe hétérocyclique à 5 ou 6 chaînons ayant éventuellement un ou plusieurs atomes ou groupes choisis dans le groupe Z ou un atome d'hydrogène,
$R^{14}$ représente un groupe C1 à C6 alkyle ayant éventuellement un ou plusieurs atomes d'halogène, un groupe phényle ayant éventuellement un ou plusieurs atomes ou groupes choisis dans le groupe Z ou un groupe hétérocyclique à 5 ou 6 chaînons ayant éventuellement un ou plusieurs atomes ou groupes choisis dans le groupe Z,
$R^{15}$ représente un groupe C1 à C6 alkyle ayant éventuellement un ou plusieurs atomes d'halogène ou un atome d'hydrogène,
$R^{16}$ représente un groupe C1 à C6 alkyle ayant éventuellement un ou plusieurs atomes d'halogène,
Q représente un atome d'oxygène ou un atome de soufre,
m représente 0, 1 ou 2, et
n représente 0, 1 ou 2 ;
Groupe X : un groupe consistant en les groupes C3 à C6 cycloalkyle ayant éventuellement un ou plusieurs atomes ou groupes choisis dans le groupe consistant en les atomes d'halogène et les groupes C1 à C3 alkyle, les groupes C1 à C6 alcoxy ayant éventuellement un ou plusieurs atomes d'halogène, les groupes C1 à C6 alkylsulfanyle ayant éventuellement un ou plusieurs atomes d'halogène, les groupes C1 à C6 alkylsulfinyle ayant éventuellement un ou plusieurs atomes d'halogène, les groupes C1 à C6 alkylsulfonyle ayant éventuellement un ou plusieurs atomes d'halogène, et les atomes d'halogène ;
Groupe Y : un groupe consistant en les groupes C1 à C6 alkyle ayant éventuellement un ou plusieurs atomes d'halogène, les groupes C1 à C6 alcoxy ayant éventuellement un ou plusieurs atomes d'halogène, les groupes C1 à C6 alkylsulfanyle ayant éventuellement un ou plusieurs atomes d'halogène, les groupes C1 à C6 alkylsulfinyle ayant éventuellement un ou plusieurs atomes d'halogène, les groupes C1 à C6 alkylsulfonyle ayant éventuellement un ou plusieurs atomes d'halogène, et les atomes d'halogène ;
Groupe Z : un groupe consistant en les groupes C1 à C6 alkyle ayant éventuellement un ou plusieurs atomes d'halogène, les groupes C1 à C6 alcoxy ayant éventuellement un ou plusieurs atomes d'halogène, les groupes C1 à C6 alkylsulfanyle ayant éventuellement un ou plusieurs atomes d'halogène, les groupes C1 à C6 alkylsulfinyle ayant éventuellement un ou plusieurs atomes d'halogène, les groupes C1 à C6 alkylsulfonyle ayant éventuellement un ou plusieurs atomes d'halogène, les groupes C1 à C6 alkylamino ayant éventuellement un ou plusieurs atomes d'halogène, les groupes C2 à C8 dialkylamino ayant éventuellement un ou plusieurs atomes d'halogène, les groupes C2 à C6 alkylcarbonyle ayant éventuellement un ou plusieurs atomes d'halogène, les groupes C2 à C6 alcoxycarbonyle ayant éventuellement un ou plusieurs atomes d'halogène, les groupes hydroxyle, les groupes thiol, les groupes amino, les groupes cyano, les groupes nitro, et les atomes d'halogène ;
Groupe W : un groupe consistant en les groupes C3 à C6 cycloalkyle ayant éventuellement un ou plusieurs atomes ou groupes choisis dans le groupe consistant en les atomes d'halogène et les groupes C1 à C3 alkyle, les groupes C1 à C6 alcoxy ayant éventuellement un ou plusieurs atomes d'halogène, les groupes C1 à C6 alkylsulfanyle ayant éventuellement un ou plusieurs atomes d'halogène, les groupes C1 à C6 alkylsulfinyle ayant éventuellement un ou plusieurs atomes d'halogène, les groupes C1 à C6 alkylsulfonyle ayant éventuellement un ou plusieurs atomes d'halogène, les groupes C2 à C6 alcoxycarbonyle ayant éventuellement un ou plusieurs atomes d'halogène, les groupes hydroxyle, les groupes cyano, et les atomes d'halogène ;
où $R^8$, $R^9$ et $R^{10}$ ne représentent pas simultanément un atome d'hydrogène, quand $R^7$, $R^8$, $R^9$ et $R^{11}$ représentent un atome d'hydrogène, $R^{10}$ ne représente pas un groupe méthyle, et quand $R^7$, $R^8$, $R^{10}$ et $R^{11}$ représentent un atome d'hydrogène, $R^9$ ne représente pas un groupe pyrrolidin-1-yle, et
quand m est 1 ou 2 dans $-S(O)_mR^{12}$, $R^{12}$ ne représente pas un atome d'hydrogène.

**2.** Composé selon la revendication 1,
où $R^1$ est un groupe C3 à C6 cycloalkyle ayant éventuellement un ou plusieurs atomes ou groupes choisis dans le groupe consistant en les atomes d'halogène et les groupes C1 à C3 alkyle,
un groupe C1 à C6 alkyle ayant éventuellement un ou plusieurs atomes ou groupes choisis dans le groupe consistant en les atomes d'halogène et un groupe cyclopropyle (où le groupe cyclopropyle a éventuellement un ou plusieurs atomes d'halogène ou un ou plusieurs groupes C1 à C3 alkyle), un groupe C2 à C6 alcényle ayant éventuellement un ou plusieurs atomes d'halogène, ou
un groupe C2 à C6 alcynyle ayant éventuellement un ou plusieurs atomes d'halogène,
$R^2$, $R^4$ et $R^5$ sont identiques ou différents et sont un groupe C1 à C6 alkyle ayant éventuellement un ou plusieurs atomes d'halogène, $-OR^{12}$, $-S(O)_mR^{12}$, un atome d'halogène, ou un atome d'hydrogène,
$R^3$ est un groupe hydrocarboné à chaîne en C1 à C6 ayant éventuellement un ou plusieurs atomes ou groupes choisis dans le groupe X, un groupe C3 à C6 cycloalkyle ayant éventuellement un ou plusieurs atomes ou groupes choisis dans le groupe Y, un groupe phényle ayant éventuellement un ou plusieurs atomes ou groupes choisis dans le groupe Z, un groupe hétérocyclique à 5 ou 6 chaînons ayant éventuellement un ou plusieurs atomes ou groupes choisis dans le groupe Z, $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, $-SF_5$, un groupe cyano, un groupe nitro, un atome d'halogène, ou un atome d'hydrogène,
$R^6$ est un groupe méthyle, un groupe éthyle, un groupe propyle, un groupe isopropyle, un groupe butyle, un groupe isobutyle, ou un groupe hexyle,
$R^7$ et $R^{11}$ sont identiques ou différents et sont un groupe C1 à C6 alkyle ayant éventuellement un ou plusieurs atomes d'halogène, $-OR^{16}$, $-S(O)_mR^{16}$, un atome de fluor ou un atome d'hydrogène,
$R^8$, $R^9$ et $R^{10}$ sont identiques ou différents et sont un groupe hydrocarboné à chaîne en C1 à C6 ayant éventuellement un ou plusieurs atomes ou groupes choisis dans le groupe X, $-OR^{12}$, $-S(O)_mR^{12}$, $-SF_5$, un atome d'halogène ou un atome d'hydrogène,
$R^{12}$ et $R^{13}$ sont identiques ou différents et sont un groupe C1 à C6 alkyle ayant éventuellement un ou plusieurs atomes d'halogène, un groupe phényle (où le groupe phényle a éventuellement un groupe C1 à C3 alkyle ayant éventuellement un ou plusieurs atomes d'halogène ou un ou plusieurs atomes d'halogène), un groupe hétérocyclique à 5 ou 6 chaînons (où le groupe hétérocyclique à 5 ou 6 chaînons a éventuellement un groupe C1 à C3 alkyle ayant éventuellement un ou plusieurs atomes d'halogène ou un ou plusieurs atomes d'halogène) ou un atome d'hydrogène,
$R^{14}$ est un groupe C1 à C6 alkyle ayant éventuellement un ou plusieurs atomes d'halogène, un groupe phényle (où le groupe phényle a éventuellement un groupe C1 à C3 alkyle ayant éventuellement un ou plusieurs atomes d'halogène ou un ou plusieurs atomes d'halogène), ou un groupe hétérocyclique à 5 ou 6 chaînons (où le groupe hétérocyclique à 5 ou 6 chaînons a éventuellement un groupe C1 à C3 alkyle ayant éventuellement un ou plusieurs atomes d'halogène ou un ou plusieurs atomes d'halogène), et
Q est un atome d'oxygène.

**3.** Composé selon la revendication 1,
où $R^1$ est un groupe C3 à C6 cycloalkyle ayant éventuellement un ou plusieurs atomes ou groupes choisis dans le groupe consistant en les atomes d'halogène et les groupes C1 à C3 alkyle,
un groupe C2 à C6 alkyle ayant éventuellement un ou plusieurs atomes d'halogène ou
un groupe (C3 à C6 cycloalkyl)C1 à C3 alkyle ayant éventuellement un ou plusieurs atomes d'halogène,
$R^2$, $R^4$ et $R^5$ sont identiques ou différents et sont un atome d'halogène ou un atome d'hydrogène,
$R^3$ est un groupe C1 à C6 alkyle ayant éventuellement un ou plusieurs atomes d'halogène, un groupe C2 à C6 alcényle ayant éventuellement un ou plusieurs atomes d'halogène, un groupe C2 à C6 alcynyle ayant éventuellement un ou plusieurs atomes d'halogène, un groupe hétérocyclique à 5 ou 6 chaînons (où le groupe hétérocyclique à 5 ou 6 chaînons a éventuellement un groupe C1 à C3 alkyle ayant éventuellement un ou plusieurs atomes d'halogène ou un ou plusieurs atomes d'halogène), $-OR^{12}$, $-S(O)_mR^{12}$, $-NR^{12}R^{13}$, $-NR^{12}C(O)R^{13}$, $-NR^{12}CO_2R^{13}$, $-NR^{12}S(O)_2R^{14}$, $-C(O)R^{12}$, $-CO_2R^{12}$, $-C(O)NR^{12}R^{13}$, un atome d'halogène, ou un atome d'hydrogène,
$R^6$ est un groupe méthyle, un groupe éthyle, un groupe propyle, un groupe isopropyle, un groupe butyle, un groupe isobutyle, ou un groupe hexyle,
$R^7$ et $R^{11}$ sont identiques ou différents et sont un atome de fluor ou un atome d'hydrogène,
$R^8$, $R^9$ et $R^{10}$ sont identiques ou différents et sont un groupe C1 à C6 alkyle ayant éventuellement un ou plusieurs atomes d'halogène, un groupe C2 à C6 alcényle ayant éventuellement un ou plusieurs atomes d'halogène, un groupe C2 à C6 alcynyle ayant éventuellement un ou plusieurs atomes d'halogène, $-OR^{12}$, $-S(O)_mR^{12}$, un atome d'halogène ou un atome d'hydrogène,
$R^{12}$ et $R^{13}$ sont identiques ou différents et sont un groupe C1 à C6 alkyle ayant éventuellement un ou plusieurs atomes d'halogène ou un atome d'hydrogène,
$R^{14}$ est un groupe C1 à C6 alkyle ayant éventuellement un ou plusieurs atomes d'halogène, et
Q est un atome d'oxygène.

4. Composé selon l'une quelconque des revendications 1 à 3, où $R^2$, $R^4$ et $R^5$ sont un atome d'hydrogène.

5. Composé selon l'une quelconque des revendications 1 à 4, où $R^7$ et $R^{11}$ sont un atome d'hydrogène.

6. Composé selon l'une quelconque des revendications 1 à 5, où $R^3$ est un groupe C1 à C3 alkyle ayant éventuellement un ou plusieurs atomes d'halogène, un groupe C1 à C3 alcoxy ayant éventuellement un ou plusieurs atomes d'halogène, un groupe C1 à C3 alkylsulfanyle ayant éventuellement un ou plusieurs atomes d'halogène, un groupe C1 à C3 alkylsulfinyle ayant éventuellement un ou plusieurs atomes d'halogène, un groupe C1 à C3 alkylsulfonyle ayant éventuellement un ou plusieurs atomes d'halogène, un groupe C1 à C6 alkylamino ayant éventuellement un ou plusieurs atomes d'halogène, un groupe C2 à C8 dialkylamino ayant éventuellement un ou plusieurs atomes d'halogène, un groupe C2 à C6 alkylcarbonylamino ayant éventuellement un ou plusieurs atomes d'halogène, un groupe C2 à C6 alcoxycarbonylamino ayant éventuellement un ou plusieurs atomes d'halogène, un groupe C1 à C3 alkylsulfonylamino ayant éventuellement un ou plusieurs atomes d'halogène, un groupe amino, un groupe C2 à C6 alkylcarbonyle ayant éventuellement un ou plusieurs atomes d'halogène, un groupe C2 à C6 alcoxycarbonyle ayant éventuellement un ou plusieurs atomes d'halogène, un groupe C2 à C6 alkylaminocarbonyle ayant éventuellement un ou plusieurs atomes d'halogène, un groupe C3 à C8 dialkylaminocarbonyle ayant éventuellement un ou plusieurs atomes d'halogène, un groupe aminocarbonyle, un groupe pyridyle (où le groupe pyridyle a éventuellement un ou plusieurs atomes ou substituants choisis dans le groupe consistant en les groupes C1 à C3 alkyle ayant éventuellement un atome d'halogène, les groupes C1 à C3 alcoxy ayant éventuellement un atome d'halogène, et les atomes d'halogène), un groupe pyrimidinyle (où le groupe pyrimidinyle a éventuellement un ou plusieurs atomes ou substituants choisis dans le groupe consistant en les groupes C1 à C3 alkyle ayant éventuellement un atome d'halogène, les groupes C1 à C3 alcoxy ayant éventuellement un atome d'halogène, et les atomes d'halogène), un atome d'halogène, ou un atome d'hydrogène.

7. Composé selon l'une quelconque des revendications 1 à 6, où $R^6$ est un groupe méthyle ou un groupe éthyle.

8. Composé selon l'une quelconque des revendications 1 à 7, où $R^1$ est un groupe éthyle, un groupe cyclopropyle, ou un groupe cyclopropylméthyle.

9. Composé selon l'une quelconque des revendications 1 à 8, où $R^8$, $R^9$ et $R^{10}$ sont identiques ou différents et sont un groupe C1 à C6 haloalkyle, un groupe C2 à C6 haloalcényle, un groupe C1 à C6 haloalcoxy, un groupe C1 à C6 haloalkylsulfanyle, un groupe C1 à C6 haloalkylsulfinyle, un groupe C1 à C6 haloalkylsulfonyle, un atome d'halogène, ou un atome d'hydrogène.

10. Composé selon l'une quelconque des revendications 1 à 8, où $R^8$, $R^9$ et $R^{10}$ sont identiques ou différents et sont un groupe C1 à C3 perfluoroalkyle, un groupe C1 à C3 perfluoroalcoxy, un groupe C1 à C3 perfluoroalkylsulfanyle, un groupe C1 à C3 perfluoroalkylsulfinyle, un groupe C1 à C3 perfluoroalkylsulfonyle, un atome d'halogène, ou un atome d'hydrogène.

11. Composé selon l'une quelconque des revendications 1 à 8, où $R^7$, $R^8$, $R^{10}$ et $R^{11}$ sont un atome d'hydrogène, $R^9$ est un groupe C1 à C3 perfluoroalkyle, un groupe C1 à C3 perfluoroalcoxy, un groupe C1 à C3 perfluoroalkylsulfanyle, un groupe C1 à C3 perfluoroalkylsulfinyle, un groupe C1 à C3 perfluoroalkylsulfonyle, ou un atome d'halogène.

12. Composé selon l'une quelconque des revendications 1 à 8, où $R^7$, $R^9$ et $R^{11}$ sont un atome d'hydrogène, $R^8$ et $R^{10}$ sont identiques ou différents et sont un groupe C1 à C3 perfluoroalkyle, un groupe C1 à C3 perfluoroalcoxy, un groupe C1 à C3 perfluoroalkylsulfanyle, un groupe C1 à C3 perfluoroalkylsulfinyle, un groupe C1 à C3 perfluoroalkylsulfonyle, un atome d'halogène, ou un atome d'hydrogène.

13. Composition pour la lutte contre les nuisibles comprenant le composé tel que défini dans l'une quelconque des revendications 1 à 12, et un vecteur inerte.

14. Procédé pour la lutte contre les nuisibles comprenant l'application d'une quantité efficace du composé tel que défini dans l'une quelconque des revendications 1 à 12 à des plantes, du sol ou à l'intérieur.

15. Composé tel que défini dans l'une quelconque des revendications 1 à 12 destiné à être utilisé dans la lutte contre les nuisibles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200334671 A